# EUROPEAN PATENT APPLICATION

(11) **EP 4 364 755 A1**
(43) Date of publication of application: **08.05.2024**
(21) Application number: 22833312.6
(22) Date of filing: 30.06.2022
(51) Int. Cl.: A61K 39/395, A61K 9/19, A61P 1/04, A61P 31/04, C07K 16/12, C12N 15/13

(54) **MONOCLONAL ANTIBODY-CONTAINING COMPOSITION FOR SUPPRESSING CLOSTRIDIUM DIFFICILE BACTERIA**

(30) Priority: 02.07.2021 JP 2021110423
(71) Applicant: The University of Tokyo, Bunkyo-ku, Tokyo 113-8654 (JP)
(72) Inventor: SHINKURA, Reiko, Tokyo 113-8654 (JP); MORITA, Naoki, Tokyo 113-8654 (JP); TAMANO, Ryutaro, Tokyo 113-8654 (JP)
(74) Representative: Grünecker Patent- und Rechtsanwälte PartG mbB
(86) International application number: PCT/JP2022/026399
(87) International publication number: WO 2023/277166

(57) **Abstract**

The present invention relates to a composition comprising a monoclonal antibody binding to Clostridium difficile (C. difficile) for inhibiting a growth of Clostridium difficile (C. difficile), and a use thereof. In addition, the present invention discloses the composition for treating an inflammatory bowel disease, and a use thereof.

## Description

### Technical Field

The present invention relates to a composition comprising a monoclonal antibody binding to Clostridium difficile (C. difficile) for inhibiting a growth of Clostridium difficile (C. difficile), and a use thereof.

### Background Art

Since the inside of the intestinal tract is equivalent to "the outside of the body" for a living body, and is thus constantly exposed to a significant wide variety and large number of intestinal resident bacteria, viruses, and antigens derived from, for example, food. Such intestinal resident bacteria form intestinal bacterial flora.

It has been clarified that the intestinal bacterial flora performs various functions in a living body via mucosal surfaces composed of intestinal epithelial cells and the like, and it has also been found that when intestinal bacteria are not present, the intestinal immune system does not develop normally (Non Patent Literatures 1 to 3).

When intestinal bacterial flora changes and a symbiotic relationship with a host is disrupted, the intestinal immune system is excessively stimulated and its homeostasis is disrupted, which leads to many diseases such as an inflammatory bowel disease, colorectal cancer, asthma, allergies, and obesity. In view of this, it is known that the intestinal immune system plays an important role not only in eliminating pathogens and the like, but also in maintaining homeostasis of the entire immune system (Non Patent Literatures 4 to 7).

Although many attempts have been made to control the intestinal bacterial flora using antibiotics in the related art, a problem is that the antibiotics are ineffective due to antibioticresistant bacteria. Therefore, despite the attempts in the related art, there are still many patients suffering from Clostridium difficile enteritis and other inflammatory bowel diseases associated with intestinal bacterial flora. In addition, as a relatively new approach, fecal transplantation or the like has been attempted, but its therapeutic effect is not stable. As a result, development as a therapy has already been stopped in some attempts. In addition, in the related art, use of an antibody against a toxin released from the intestinal bacterial flora as a drug has been studied and commercialized. However, since the antibody drug is only for a toxin, a therapy using the antibody drug is only a symptomatic therapy. Therefore, development of a drug for radically treating an inflammatory bowel disease associated with intestinal bacterial flora has been desired.

C. difficile associated with an inflammatory bowel disease is a bacterium that requires attention together with MRSA as a bacterium causing nosocomial infection. In fact, according to U.S. statistics, about 400,000 to 500,000 people are infected and about 15,000 to 20,000 people die every year. C. difficile infection often occurs in patients on long-term administration of antibiotics, and once infected, since C. difficile infection has a high recurrence rate of 15 to 35%, an effective treatment is particularly required for inflammatory bowel disease-associated bacteria. Currently, a treatment with a strong antibiotic such as vancomycin is performed, but an alternative method is required because toxins of bacteria remain and development of bacteria having antibiotic resistance is promoted.

### Citation List

### Non Patent Literatures

Non Patent Literature 1: Cerf-Bensussan N, and Gaboriau-Routhiau V., Nat Rev Immunol 2010;10:735
Non Patent Literature 2: Hooper LV, and Macpherson AJ., Nat Rev Immunol 2010;10:159
Non Patent Literature 3: Round JL, and Mazmanian SK, Proc Natl Acad Sci USA 2010;107:12204
Non Patent Literature 4: Vijay-Kumar M, et al., Science 2010;328:228
Non Patent Literature 5: Shulzhenko N, et al., Nat Med 2011;17:1585
Non Patent Literature 6: Bry L, et al., Science 1996;273:1380
Non Patent Literature 7: Turnbaugh PJ, et al., Nature 2006;444:1027

### Summary of Invention

### Technical Problem

An object of the present invention is to provide a monoclonal antibody used for inhibiting a growth of Clostridium difficile in a living body and treating an inflammatory bowel disease or an antigen-binding fragment thereof, a composition comprising the same, and a method for using the same.

### Solution to Problem

As a result of intensive studies, the inventors of the present invention have found that a growth of Clostridium difficile in a living body can be inhibited, and an inflammatory bowel disease can be treated, by using a monoclonal antibody binding to Clostridium difficile.

In one aspect, the present invention provides a composition for inhibiting a growth of Clostridium difficile in a living body, the composition comprising a monoclonal antibody binding to a Clostridium difficile bacterial body or an antigen-binding fragment thereof, or a composition used for treating an inflammatory bowel disease.

In one aspect, the present invention provides a composition for inhibiting a growth of Clostridium difficile in a living body, the composition comprising a monoclonal antibody binding to a Clostridium difficile bacterial body or an antigen-binding fragment thereof.

In one aspect, the present invention provides a method for inhibiting a growth of Clostridium difficile in an individual, the method including administering an effective amount of a monoclonal antibody binding to a Clostridium difficile bacterial body or an antigen-binding fragment thereof to the individual.

In one aspect, the present invention provides a method for treating an inflammatory bowel disease in an individual, the method including administering an effective amount of a monoclonal antibody binding to a Clostridium difficile bacterial body or an antigen-binding fragment thereof to the individual.

More specifically, the present invention provides the following.

### [Item 1]

A composition for inhibiting a growth of Clostridium difficile in a living body, the composition comprising a monoclonal antibody binding to a Clostridium difficile bacterial body or an antigen-binding fragment thereof.

### [Item 2]

The composition according to Item 1, in which the composition is used for treating an inflammatory bowel disease.

### [Item 3]

The composition according to Item 1 or 2, in which the monoclonal antibody binding to a Clostridium difficile bacterial body further binds to a Fusobacterium nucleatum bacterial body.

### [Item 4]

The composition according to any one of Items 1 to 3, in which the monoclonal antibody binding to a Clostridium difficile bacterial body is:
a) an antibody comprising:
   a heavy chain variable region comprising an amino acid sequence of a heavy chain CDR1, an amino acid sequence of a heavy chain CDR2, and an amino acid sequence of a heavy chain CDR3, the heavy chain variable region comprising an amino acid sequence set forth in SEQ ID NO: 7; and
   a light chain variable region comprising an amino acid sequence of a light chain CDR1, an amino acid sequence of a light chain CDR2, and an amino acid sequence of a light chain CDR3, the light chain variable region comprising an amino acid sequence set forth in SEQ ID NO: 8,
b) an antibody comprising:
   a heavy chain variable region comprising an amino acid sequence of a heavy chain CDR1, an amino acid sequence of a heavy chain CDR2, and an amino acid sequence of a heavy chain CDR3, the heavy chain variable region comprising an amino acid sequence set forth in SEQ ID NO: 17; and
   a light chain variable region comprising an amino acid sequence of a light chain CDR1, an amino acid sequence of a light chain CDR2, and an amino acid sequence of a light chain CDR3, the light chain variable region comprising an amino acid sequence set forth in SEQ ID NO: 18,
c) an antibody comprising:
   a heavy chain variable region comprising an amino acid sequence of a heavy chain CDR1, an amino acid sequence of a heavy chain CDR2, and an amino acid sequence of a heavy chain CDR3, the heavy chain variable region comprising an amino acid sequence set forth in SEQ ID NO: 27; and
   a light chain variable region comprising an amino acid sequence of a light chain CDR1, an amino acid sequence of a light chain CDR2, and an amino acid sequence of a light chain CDR3, the light chain variable region comprising an amino acid sequence set forth in SEQ ID NO: 28,
d) an antibody comprising:
   a heavy chain variable region comprising an amino acid sequence of a heavy chain CDR1, an amino acid sequence of a heavy chain CDR2, and an amino acid sequence of a heavy chain CDR3, the heavy chain variable region comprising an amino acid sequence set forth in SEQ ID NO: 37; and
   a light chain variable region comprising an amino acid sequence of a light chain CDR1, an amino acid sequence of a light chain CDR2, and an amino acid sequence of a light chain CDR3, the light chain variable region comprising an amino acid sequence set forth in SEQ ID NO: 38, or
e) an antibody comprising at least one amino acid mutation in at least one region selected from heavy chains CDR1 to 3, light chains CDR1 to 3, and a light chain FR1, and
   binding to the amino acid sequence RQEEHIELIAS in E. coli SHMT protein and the amino acid sequence VLDMMKLEKPE in C. difficile iPGM protein,
   as a reference antibody comprising a heavy chain variable region comprising an amino acid sequence set forth in SEQ ID NO: 37, and
   a light chain variable region comprising an amino acid sequence set forth in SEQ ID NO: 38.

### [Item 5]

The composition according to any one of Items 1 to 3, in which the monoclonal antibody binding to a Clostridium difficile bacterial body is an antibody comprising a heavy chain variable region comprising:
a heavy chain CDR1 comprising an amino acid sequence set forth in SEQ ID NO: 1;
a heavy chain CDR2 comprising an amino acid sequence set forth in SEQ ID NO: 2; and
a heavy chain CDR3 comprising an amino acid sequence set forth in SEQ ID NO: 3, and
a light chain variable region comprising:
   a light chain CDR1 comprising an amino acid sequence set forth in SEQ ID NO: 4;
   a light chain CDR2 comprising an amino acid sequence set forth in SEQ ID NO: 5; and
   a light chain CDR3 comprising an amino acid sequence set forth in SEQ ID NO: 6.

### [Item 6]

The composition according to any one of Items 1 to 3, in which the monoclonal antibody binding to a Clostridium difficile bacterial body is an antibody comprising a heavy chain variable region comprising:
a heavy chain CDR1 comprising an amino acid sequence set forth in SEQ ID NO: 11;
a heavy chain CDR2 comprising an amino acid sequence set forth in SEQ ID NO: 12; and
a heavy chain CDR3 comprising an amino acid sequence set forth in SEQ ID NO: 13, and
a light chain variable region comprising:
   a light chain CDR1 comprising an amino acid sequence set forth in SEQ ID NO: 14;
   a light chain CDR2 comprising an amino acid sequence set forth in SEQ ID NO: 15; and
   a light chain CDR3 comprising an amino acid sequence set forth in SEQ ID NO: 16.

### [Item 7]

The composition according to any one of Items 1 to 3, in which the monoclonal antibody binding to a Clostridium difficile bacterial body is an antibody comprising a heavy chain variable region comprising:
a heavy chain CDR1 comprising an amino acid sequence set forth in SEQ ID NO: 21;
a heavy chain CDR2 comprising an amino acid sequence set forth in SEQ ID NO: 22; and
a heavy chain CDR3 comprising an amino acid sequence set forth in SEQ ID NO: 23, and
a light chain variable region comprising:
   a light chain CDR1 comprising an amino acid sequence set forth in SEQ ID NO: 24;
   a light chain CDR2 comprising an amino acid sequence set forth in SEQ ID NO: 25; and
   a light chain CDR3 comprising an amino acid sequence set forth in SEQ ID NO: 26.

### [Item 8]

The composition according to any one of Items 1 to 3, in which the monoclonal antibody binding to a Clostridium difficile bacterial body is an antibody comprising a heavy chain variable region comprising:
a heavy chain CDR1 comprising an amino acid sequence set forth in SEQ ID NO: 31;
a heavy chain CDR2 comprising an amino acid sequence set forth in SEQ ID NO: 32; and
a heavy chain CDR3 comprising an amino acid sequence set forth in SEQ ID NO: 33, and
a light chain variable region comprising:
   a light chain CDR1 comprising an amino acid sequence set forth in SEQ ID NO: 34;
   a light chain CDR2 comprising an amino acid sequence set forth in SEQ ID NO: 35; and
   a light chain CDR3 comprising an amino acid sequence set forth in SEQ ID NO: 36.

### [Item 9]

The composition according to any one of Items 1 to 3, in which the monoclonal antibody binding to a Clostridium difficile bacterial body is, as a reference antibody comprising a heavy chain variable region comprising an amino acid sequence set forth in SEQ ID NO: 37, and
a light chain variable region comprising an amino acid sequence set forth in SEQ ID NO: 38,
an antibody comprising at least one amino acid mutation in at least one region selected from heavy chains CDR1 to 3, light chains CDR1 to 3, and a light chain FR1, and
binding to the amino acid sequence RQEEHIELIAS in E. coli SHMT protein and the amino acid sequence VLDMMKLEKPE in C. difficile iPGM protein.

### [Item 10]

The composition according to any one of Items 1 to 3, in which the monoclonal antibody binding to a Clostridium difficile bacterial body is an antibody comprising:
a heavy chain variable region comprising:
   a heavy chain CDR1 comprising an amino acid sequence of X₁YYIH;
   a heavy chain CDR2 comprising an amino acid sequence of RIDPENX₂X₃TTYAPKFQ; and
   a heavy chain CDR3 comprising an amino acid sequence of YCARSTVL; and
   a light chain variable region comprising:
      a light chain CDR1 comprising an amino acid sequence of RX₄SQSIVHTNG;
      a light chain CDR2 comprising an amino acid sequence of KLLIYKV; and
      a light chain CDR3 comprising an amino acid sequence of GVYYFQGS,
      in which a light chain FR1 contains an amino acid sequence of TPLSLPVSLGDQA or an amino acid sequence of SPASX₅SVSLGDRX₆,
      X₁, X₂, and X₃ are each independently a neutral polar amino acid or an acidic polar amino acid, X₄ is a non-polar amino acid or a neutral polar amino acid, and X₅ and X₆ are each independently a non-polar amino acid.

### [Item 11]

The composition according to any one of Items 1 to 10, in which the composition is provided as a lyophilizing agent.

### Advantageous Effects of Invention

The present invention has the effect of providing a composition for inhibiting a growth of Clostridium difficile in a living body, the composition comprising a monoclonal antibody binding to a Clostridium difficile bacterial body or an antigen-binding fragment thereof, or a composition used for treating an inflammatory bowel disease.

### Brief Description of Drawings

Fig. 1 is a view illustrating a C. difficile growth inhibitory effect by an antibody binding to a C. difficile bacterial body.
Fig. 2 is a view illustrating a C. difficile infection inhibitory effect by the antibody binding to a C. difficile bacterial body, and illustrates results obtained by using SNK0002M and W27G2 as the antibodies binding to C. difficile bacterial bodies.
Fig. 3 is a view illustrating a C. difficile infection inhibitory effect by the antibody binding to a C. difficile bacterial body, and illustrates a viable cell count of C. difficile in feces 4 days (left panel) and 10 days (right panel) after C. difficile infection. illustrates results obtained by using SNK0002M and W27G2 as the antibodies binding to C. difficile bacterial bodies.
Fig. 4 is a view illustrating a growth inhibitory effect of the antibody binding to a C. difficile bacterial body on Fusobacterium nucleatum considered to be a causative bacterium of colorectal cancer, and illustrates results obtained by using antibodies SNK0001M, SNK0003A, SNK0001MR, SNK0002MR, SNK0001AR, SNK0002AR, and RS_H000_L001 as the antibodies binding to C. difficile bacterial bodies.
Fig. 5 is a view illustrating two-dimensional electrophoresis data when an antigen molecule of C. difficile is identified, in which the same spots in three SDS-PAGE images are indicated by red arrows, and a sample obtained from the spot is subjected to mass spectrometry.
Fig. 6 is a view illustrating results of Western blot and CBB stain of W27G2 antibodies for various synthetic peptides, in which the gray letters indicate an amino acid sequence common to each bacterial species, and the underline indicates the synthetic peptide recognized by the W27G2 antibodies.
Fig. 7 is a view illustrating results of Western blot and CBB stain of W27G2 antibodies for various synthetic peptides, in which the gray letters indicate an amino acid sequence common to each bacterial species, and the underline indicates the synthetic peptide recognized by the W27G2 antibodies.
Fig. 8 is a view illustrating a part of the results of database analysis of bacteria sharing an epitope of E. coli SHMT recognized by W27G2 antibodies.
Fig. 9 is a view illustrating crystals of RS_H000_L000GR Fab - E. coli SHMT (25-45).
Fig. 10 is a view illustrating crystals of an RS_H000_L000GR Fab-C. difficile iPGM (486-509) complex.
Fig. 11 is a three-dimensional reconfiguration image showing a binding mode between W27G2 antibodies and E. coli SHMT antigen (left) and a binding mode between W27G2 antibodies and C. difficile iPGM antigen.
Fig. 12 is a three-dimensional reconfiguration image showing a binding mode between W27G2 antibodies and E. coli SHMT antigen (left) and a binding mode between W27G2 antibodies and C. difficile iPGM antigen.
Fig. 13 illustrates that amino acid sequences of heavy chain variable regions of mutants of the W27G2 antibody which is an antibody binding to a C. difficile bacterial body of the present invention are aligned.
Fig. 14 illustrates that amino acid sequences of light chain variable regions of mutants of the W27G2 antibody which is an antibody binding to a C. difficile bacterial body of the present invention are aligned.

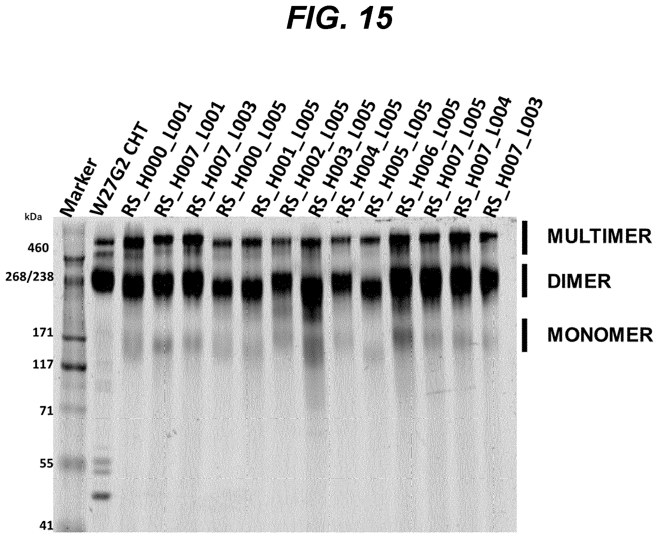

Fig. 15 is a view illustrating results of subjecting RS mutant recombinant purified antibodies to non-reduced SDS-PAGE, and performing Coomassie blue stain, in which Lane 1 shows a result of an antibody sample that is crudely purified from a W27G2 hybridoma culture solution by a hydroxyapatite column, and Lanes 2 to 14 shows results of samples of various RS mutant recombinant purified antibodies produced by CHO cells (RS mutant recombinant purified antibodies are antibodies produced in CHO cells by introducing mutations into heavy chains or light chains of the W27G2 antibody so that antigen specificity is not substantially changed, and details are omitted in Fig. 15).
Fig. 16 is a view illustrating binding of a W27G2 antibody and various RS mutant recombinant purified antibodies obtained from a hybridoma to E. coli SHMT and SHMT mutants.
Fig. 17 is a view illustrating specific binding characteristics to total proteins of a plurality of bacteria for the W27G2 antibody and various RS mutant recombinant purified antibodies, in which W27G2-CHT (obtained by crudely purifying a W27 hybridoma culture supernatant with a hydroxyapatite column) and W27G2-GF (obtained by further purifying W27CHT into a multimer fraction with a gel filtration column) are used as the W27G2 antibodies, and RS_H000_L001, RS_H000_L005, and RS_H007_L005 are used as the RS mutant recombinant purified antibodies.
Fig. 18 is a view illustrating an E. coli growth inhibitory effect by various RS variant recombinant purified antibodies.
Fig. 19 is a view illustrating that a weight loss and a decrease in survival rate of C. difficile-infected mice of each of two recombinant IgA antibodies, antibodies RS_H000_L001 (rW27), and SNK0002AR are suppressed.
Fig. 20 is a view illustrating a content of C. difficile in feces after administration of two recombinant IgA antibodies, antibodies RS_H000_L001 (rW27), SNK0002AR, and Vancomycin.
Fig. 21 is a view illustrating results of analyzing a relative abundance ratio of order levels of bacteria by 16S rRNA analysis in feces collected at 14 days after administration of two recombinant IgA antibodies, antibodies RS_H000_L001 (rW27), SNK0002AR, rW27 IgG antibodies, and Vancomycin (dead mice at the time of death).
Fig. 22 is a view illustrating results of performing β-diversity analysis using the 16S rRNA analysis results in feces collected at 14 days after administration of two recombinant IgA antibodies, antibodies RS_H000_L001 (rW27), SNK0002AR, rW27 IgG antibodies, and Vancomycin (dead mice at the time of death).

### Description of Embodiments

### (Definition)

In the present specification, in a case where a plurality of ranges of numerical values are indicated, a range including any combination of a lower limit value and an upper limit value of the plurality of ranges is also meant in the same manner.

In the present specification, the term "antibody" is used in the broadest sense, and includes, but is not limited to, a monoclonal antibody, a polyclonal antibody, and an antibody fragment that exhibit an intended antigen binding activity. A full-length antibody includes a heavy chain and a light chain mainly composed of a polypeptide. The heavy chain and the light chain each contain a site called a variable region that recognizes an antigen, and the sites are generally called a heavy chain variable region and a light chain variable region, respectively. The variable region has, in order from the amino terminus, sites referred to as CDR1 to 3, each of which is identified as a site that recognizes an antigen in more detail. Note that these CDR1 to 3 are also referred to in more detail as a heavy chain CDR1, a heavy chain CDR2, a heavy chain CDR3, a light chain CDR1, a light chain CDR2, a light chain CDR3, and the like. In addition, regions other than CDR1 to 3 of the heavy chains and the light chains are referred to as heavy chains FR1 to 4 and light chains FR1 to 4, respectively, in order from the amino terminus. The antibody may be in the form of an antibody composed of two heavy chains and two light chains, or in the form of an antibody composed of one heavy chain and one light chain (also referred to as a single-chain antibody).

The antibody may be any class such as IgG, IgE, IgM, IgD, IgA, or IgY, or may be a subclass such as IgG1, IgG2, IgG3, IgG4, IgA1, or IgA2.

The antibody may contain an amino acid sequence derived from the same species or may contain an amino acid derived from a different species. Examples of the amino acid sequence derived from the same species include amino acid sequences derived from a human, a mouse, a rat, a hamster, a rabbit, a goat, a donkey, a pig, a cow, a horse, a chicken, a monkey, a chimpanzee, a camel, and a llama.

The case of the amino acid sequence derived from a different species is not particularly limited, and examples thereof include amino acid sequences derived from two or more of a human, a mouse, a rat, a hamster, a rabbit, a goat, a sheep, a donkey, a pig, a cow, a horse, a chicken, a monkey, a chimpanzee, a camel, and a llama.

In the present specification, the "antigen-binding fragment" of the antibody refers to one or more fragments of the antibody that retain an ability to specifically bind to an antigen. It has been found that the ability of an antibody to specifically bind to an antigen can also be maintained by fragments consisting of a part thereof. As one aspect, an "antigen-binding fragment" of an antibody may be, but is not limited to, a Fab fragment consisting of a CH1 domain that is a part of a light chain variable region (VL), a heavy chain variable region (VH), a light chain constant region (CL), and a heavy chain constant region, a F(ab')2 fragment including two Fab fragments linked by a disulfide bridge at a hinge region, a Fd fragment consisting of the VH and CH1 domain, a Fv fragment consisting of the VL and VH domains of a single arm of an antibody, a dAb fragment including a single variable domain, and an isolated complementarity determining region (CDR).

The antibody of the present invention may be a CDR-grafted antibody. In one example, in a CDR-grafted antibody, some or all sequences of CDR regions of an antibody derived from one animal species are substituted with CDR sequences of another animal species. For example, CDRs of one or more mouse antibodies are substituted with CDR sequences of human antibodies.

Methods well known to those skilled in the art can be used in identifying heavy chains CDR1 to 3 in a heavy chain variable region and light chains CDR1 to 3 in a light chain variable region of an antibody. For example, "Kabat definition" (Kabat et al., Ann. NY Acad, Sci. 1971, Vol. 190, pp. 382-391 and Kabat, E.A. et al. Sequences of Proteins of Immunological Interest, 5th Ed. 1991, U.S. Department of Health and Human Services, NIH Publication, pp. 91-3242), "Chothia definition" (Chothia et al., Nature, 1989, Vol. 342, pp. 877-883), "Contact definition" (MacCallum et al., J. Mol. Biol., 1996, Vol. 262, pp. 732-745), and the like, which are well known to those skilled in the art, can be used. CDRs may be identified based on public database information to identify CDR sequences in the antibody.

In the present specification, the "identity" refers to a degree of the same amino acid sequence or base sequence of two or more comparable amino acid sequences or base sequences with respect to each other. Therefore, the higher the identity between two amino acid sequences or base sequences, the higher the identity or similarity between the sequences. A level of the identity between the amino acid sequences or base sequences is usually determined using FASTA, which is a tool for sequence analysis, and default parameters. Alternatively, it can be determined using the algorithm BLAST (for example, Karlin S, Altschul SF. Proc. Natl Acad Sci USA. 87:2264-2268 (1990), Karlin S, Altschul SF. Natl Acad Sci USA. 90:5873-7 (1993), and the like) by Karlin and Altschul. A program called BLASTN or BLASTX based on such a BLAST algorithm has been developed (for example, Altschul SF, GishW, Miller W, Myers EW, Lipman DJ. J Mol Biol. 215:403-10 (1990), and the like). Specific techniques for these analysis methods are known and can be referred to the NCBI website. For example, a case where a certain amino acid sequence A is a specific % identical to another amino acid sequence B means that the amino acid sequence A and the amino acid sequence B have the % identity.

In the present specification, the term "monoclonal" is a modifier indicating a characteristic of an antibody or the like obtained from a substantially homogeneous population of antibodies. The individual antibodies contained in such a population of antibodies are identical except for naturally occurring mutations that may be present in a small amount.

In the present specification, the term "conservative substitution technique" means a technique in which an amino acid residue is substituted with an amino acid residue having a similar side chain.

For example, substitution between amino acid residues having a basic side chain such as lysine, arginine, and histidine corresponds to a conservative substitution technique. Other examples of the conservative substitution technique include substitutions between amino acid residues having an acidic side chain such as aspartic acid and glutamic acid; substitutions between amino acid residues having an uncharged polar side chain such as glycine, asparagine, glutamine, serine, threonine, tyrosine, and cysteine; substitutions between amino acid residues having a non-polar side chain such as alanine, valine, leucine, isoleucine, proline, phenylalanine, methionine, and tryptophan; substitutions between amino acid residues having a β-branched side chain such as threonine, valine, and isoleucine; and substitutions between amino acid residues having an aromatic side chains such as tyrosine, phenylalanine, tryptophan, and histidine.

In the present specification, the term "intestinal bacteria" is not particularly limited as long as the intestinal bacteria are bacteria that are resident in the intestine in a living body. Examples of such intestinal bacteria include bacteria that form intestinal bacterial flora in a living body and are involved in maintenance of homeostasis of the intestinal immune system.

Examples of specific intestinal bacteria include, but are not particularly limited to,
bacteria belonging to the genus Prevotella,
bacteria belonging to the genus Bacteroides,
bacteria belonging to the genus Megamonas,
bacteria belonging to the genus Bifidobacterium,
bacteria belonging to the genus Faecalibacterium,
bacteria belonging to the genus Coprococcus,
bacteria belonging to the genus Ruminococcus,
bacteria belonging to the genus Blautia,
bacteria belonging to the genus Eubacterium,
bacteria belonging to the genus Roseburia,
bacteria belonging to the genus Lactobacillus,
bacteria belonging to the genus Clostridium,
bacteria belonging to the genus Escherichia,
bacteria belonging to the genus Staphylococcus,
bacteria belonging to the genus Enterococcus,
bacteria belonging to the genus Pseudomonas,
bacteria belonging to the genus Enterorhabdus, and
bacteria belonging to the genus Fusobacterium.

### (Composition)

A composition of the present invention is preferably provided as, but is not limited to, a pharmaceutical composition, an oral composition, or an enteral composition.

### (Pharmaceutical Composition)

The pharmaceutical composition according to the present invention contains a monoclonal antibody binding to a C. difficile bacterial body or an antigen-binding fragment thereof, and is used for inhibiting a growth of C. difficile in a living body.

In one aspect, the pharmaceutical composition according to the present invention is used for treating a disease associated with C. difficile.

The disease associated with C. difficile is not particularly limited, examples thereof include an inflammatory bowel disease, ulcerative colitis, Crohn's disease, allergy, asthma, obesity, autoimmune disease, and neonatal necrotizing enterocolitis, and among them, an inflammatory bowel disease is preferable.

Such a pharmaceutical composition according to the present invention only needs to contain an effective amount of the monoclonal antibody binding to a C. difficile bacterial body according to the present invention or the antigen-binding fragment thereof, and can be appropriately set, for example, so that a content ratio of the antibody according to the present invention in 100 wt% of the pharmaceutical composition is in a range of 0.001 to 99.99 wt%, in consideration of the type of a target disease, a dosage form, an administration method, a target to be administered, a degree of symptoms of an administration subject, a degree of effect exerted by administration, and the like.

The term "effective amount" used in the present specification refers to an amount by which the monoclonal antibody binding to a C. difficile bacterial body according to the present invention or the antigen-binding fragment thereof can inhibit a growth of C. difficile in a living body, or an amount by which a disease therapeutic effect can be exerted.

The pharmaceutical composition according to the present invention may contain a pharmaceutically acceptable carrier or additive together with the monoclonal antibody binding to a C. difficile bacterial body according to the present invention. The pharmaceutically acceptable carrier or additive means any carrier, diluent, excipient, suspending agent, lubricant, adjuvant, medium, delivery system, emulsifier, disintegrant, absorbent, preservative, surfactant, colorant, flavoring, or sweetener, and a known pharmaceutically acceptable carrier or additive may be employed.

The pharmaceutical composition according to the present invention has applicability in a method for treating an intestinal disease including a step of administering to an individual suffering from an intestinal disease associated with C. difficile described above. In addition, the pharmaceutical composition according to the present invention has applicability in a method for preventing an intestinal disease including a step of administering to an individual who has not developed pathology or symptoms of the intestinal disease but may have a predisposition to an intestinal disease. These individuals may be a target to be administered with the pharmaceutical composition according to the present invention.

Examples of the individual to be administered include, but are not limited to, mammals such as a human, a mouse, a rat, a guinea pig, a rabbit, a hamster, a dog, a cat, a weasel, a cow, and a pig, and birds such as a chicken.

A dose and administration method of the pharmaceutical composition vary depending on the type, gender, species, age, general condition, severity of the disease, degree of desired effect, and the like of the intestinal disease that affects the individual to be administered. Usually, the dose may be appropriately set in a range of 0.001 to 100 mg/kg/day.

In addition, the administration method is not particularly limited, but it is preferable to directly administer to the digestive tract, and examples of such an administration method include oral administration, nasal administration, transmucosal administration, and enteral administration.

Note that the enteral administration is not limited to administration via the anus, and includes, for example, administration via a tube or the like inserted into the digestive tract from the outside of the individual like a gastrostomy or the like. A position where the digestive tract is inserted is not limited to the intestine, and examples thereof include esophagus, stomach, small intestine (including duodenum, jejunum, ileum, and the like), and large intestine (including cecum, colon, rectum, and the like).

Note that in the administration of such a pharmaceutical composition according to the present invention, the above amount may be administered once a day, or may be administered in several divided doses. In addition, an administration interval may be every day, every other day, every week, every other week, every 2 or 3 weeks, every month, every other month, or every 2 or 3 months as long as the therapeutic effect on the disease is obtained.

### (Oral or Enteral Composition)

An oral or enteral composition according to the present invention contains the monoclonal antibody binding to a C. difficile bacterial body according to the present invention or the antigen-binding fragment thereof. A growth of C. difficile in a living body can be inhibited by using such an oral or enteral composition, and thus, a disease associated with C. difficile can be treated.

A blending ratio of the antibody binding to a C. difficile bacterial body in such an oral or enteral composition is not particularly limited, and may be appropriately adjusted according to the form, use, and the like of the oral or enteral composition, but may be usually about 0.001 to 99 wt% with respect to the total amount of the oral or enteral composition.

Examples of the individual to whom the oral or enteral composition according to the present invention is used include, but are not limited to, mammals such as a human, a mouse, a rat, a guinea pig, a rabbit, a hamster, a dog, a cat, a weasel, a cow, and a pig, and birds such as a chicken.

The antibody binding to a C. difficile bacterial body contained in the oral or enteral composition according to the present invention is particularly useful as an intestinal regulation composition, an intestinal environment improving composition, an intestinal environment optimization composition, or an intestinal spoilage preventing composition.

The amount of the oral or enteral composition used according to the present invention is not particularly limited as long as the effect of the oral or enteral composition as described above is exhibited, and may be set according to the type of individual who ingests the oral or enteral composition, an intended effect, a degree of the intended effect, other various conditions, and the like. Specifically, the amount of the antibody binding to a C. difficile bacterial antibody according to the present invention may be usually about 0.001 to 100 mg/kg/day, and may be taken once to several times a day.

Note that the enteral administration is not limited to administration via the anus. For example, the enteral composition of the present invention can be blended with a known component and used as an intestinal washing liquid.

The oral or enteral composition according to the present invention contains a monoclonal antibody binding to a C. difficile bacterial body according to the present invention that exerts an effect of inhibiting an abnormal growth of intestinal bacteria and/or a pathologic change of intestinal bacterial flora, and can be preferably used in the field of food and the field of feed with the expectation of exerting such an effect. Therefore, the oral or enteral composition of the present invention can be a food composition or a feed composition.

The food composition described above is a composition in which the oral or enteral composition of the present invention is preferably used exclusively in the field of food. Such a food composition can be provided as a food composition labeled as for intestinal regulation, intestinal environment improvement, intestinal environment optimization improvement, intestinal spoilage prevention, or the like.

Examples of the food composition described above include, in addition to general foods, foods for specified health uses including foods for conditional specified health uses, nutritional supplementary foods, functional foods, and foods for patients.

Specific examples of the form of the food composition described above include, but are not limited to, drinks such as soft drinks, carbonated drinks, nutritional drinks, fruit drinks, lactic acid drinks, and milk drinks; frozen desserts such as ice cream, ice sorbet, and shaved ice; confectioneries such as candies, candies, gum, chocolate, tablet candies, snacks, biscuits, jelly, jam, cream, and baked confectioneries; noodles such as buckwheat noodles, thick noodles made from wheat flour, starch noodles, Chinese-style noodles, and instant noodles; fish or livestock processed foods such as fish cakes, ham, and sausage; dairy products such as processed milk products and fermented milk products; oil and fat and oil and fat processed foods such as salad oil, tempura oil, margarine, mayonnaise, shortening, whipped cream, and dressing; seasonings such as sauces and tare sauce; and soups, stews, salads, side dishes, furikake, pickles, bread, and cereal. In addition, in the cases of foods for specified health uses, nutritional supplementary foods, functional foods, and the like, examples thereof include powders, granules, capsules, troches, tablets, and syrups.

The feed composition is a composition for which the feed composition of the present invention is exclusively used in the field of feed. Such a feed composition can be provided as a food composition labeled as for intestinal regulation, intestinal environment improvement, intestinal environment optimization improvement, intestinal spoilage prevention, or the like.

The specific form of the feed composition described above is not particularly limited, and for example, as long as the effect exhibited by the feed composition of the present invention described above is not impaired, a feed food composition may be prepared by mixing a food feed with a normal feed, and as necessary, by mixing a component that can be blended with a normal feed, or a food feed composition itself may be used as a feed.

### (Method for Inhibiting Growth of C. difficile In Living Body)

A method for inhibiting a growth of C. difficile in a living body according to the present invention is a method including a step of administering, to a living body, an effective amount of the antibody binding to a C. difficile bacterial body according to the present invention or the antigen-binding fragment thereof, or the pharmaceutical composition, the oral composition, or the enteral composition comprising the antibody or the antigen-binding fragment thereof.

The method for inhibiting a growth of C. difficile in a living body according to the present invention may be performed to treat a disease associated with C. difficile. The disease associated with C. difficile may be the disease described in [Pharmaceutical Composition According To Present Invention] described above. In addition, the specific administration method and dose can also be as described in [Pharmaceutical Composition According To Present Invention] described above.

### (Antibody Binding to C. difficile Bacterial Body or Antigen-Binding Fragment Thereof)

The antibody binding to a C. difficile bacterial body of the present invention binds to C. difficile. Typically, the antibody of the present invention binds to undestroyed C. difficile present in a culture supernatant.

As the antibody binding to a C. difficile bacterial body of the present invention, an antibody produced from an antibody-producing cell derived from a B cell such as a hybridoma may be used,
or an antibody produced by introducing a nucleic acid encoding the antibody into a cell other than the immune system using a genetic recombination technique may be used as a recombinant antibody.

In one aspect, the antibody binding to a C. difficile bacterial body of the present invention includes a heavy chain variable region comprising an amino acid sequence of a heavy chain CDR1, an amino acid sequence of a heavy chain CDR2, and an amino acid sequence of a heavy chain CDR3, the heavy chain variable region comprising an amino acid sequence set forth in SEQ ID NO: 7, and
a light chain variable region comprising an amino acid sequence of a light chain CDR1, an amino acid sequence of a light chain CDR2, and an amino acid sequence of a light chain CDR3, the light chain variable region comprising an amino acid sequence set forth in SEQ ID NO: 8.

| Sequence name (SEQ ID NO:) | Amino acid sequence |
|---|---|
| SNK0001_HV (SEQ ID NO: 7) | MEWIWIFLFILSGTAGVQSQVQLQQSGAELARPG ASVKLSCKASGYTFTSYGISWVKQRTGQGLEWIG EIYPRSGNTYYNEKFKGKATLTADKSSSTAYMEL RSLTSEDSAVYFCARLASSYYGSSYDWYFDVWGT GTTVTVSS |
| SNK0001_LV (SEQ ID NO: 8) | MRTPAQFLGILLLWFPGIKCDIKMTQSPSSMYAS LGERVTITCKASQDINSYLSWFQQKPGKSPKTLI YRANRLVDGVPSRFSGSGSGQDYSLTISSLEYED MGIYYCLQYDEFPLTFGAGTKLELK |

In one aspect, the antibody binding to a C. difficile bacterial body of the present invention includes:
a heavy chain variable region comprising:
a heavy chain CDR1 comprising an amino acid sequence set forth in SEQ ID NO: 1;
a heavy chain CDR2 comprising an amino acid sequence set forth in SEQ ID NO: 2; and
a heavy chain CDR3 comprising an amino acid sequence set forth in SEQ ID NO: 3; and
a light chain variable region comprising:
   a light chain CDR1 comprising an amino acid sequence set forth in SEQ ID NO: 4;
   a light chain CDR2 comprising an amino acid sequence set forth in SEQ ID NO: 5; and
   a light chain CDR3 comprising an amino acid sequence set forth in SEQ ID NO: 6.

| Sequence name (SEQ ID NO:) | Amino acid sequence |
|---|---|
| SNK0001_HCDR1 (SEQ ID NO: 1) | SYGIS |
| SNK0001_HCDR2 (SEQ ID NO: 2) | EIYPRSGNTYYNEKFK |
| SNK0001_HCDR3 (SEQ ID NO: 3) | FCARLASS |
| SNK0001_LCDR1 (SEQ ID NO: 4) | KASQDINSYLS |
| SNK0001_LCDR2 (SEQ ID NO: 5) | RANRLVD |
| SNK0001_LCDR3 (SEQ ID NO: 6) | LQYDEFPLT |

In one aspect, the antibody binding to a C. difficile bacterial body of the present invention includes:
a heavy chain variable region comprising an amino acid sequence set forth in SEQ ID NO: 7 or a sequence having at least 90%, at least 95%, at least 98%, or at least 99% sequence identity therewith; and
a light chain variable region comprising an amino acid sequence set forth in SEQ ID NO: 8 or a sequence having at least 90%, at least 95%, at least 98%, or at least 99% sequence identity therewith.

In one aspect, the antibody binding to a C. difficile bacterial body of the present invention is IgM antibody SNK0001M produced by a hybridoma obtained from B cells derived from spleen. A recombinant purified antibody SNK0001MR produced by determining a nucleic acid sequence encoding the antibody SNK0001M and applying a recombination technique, and a recombinant purified antibody SNK0001AR as an IgA antibody can also be used in the same manner.

The antibody SNK0001AR has the following amino acid sequence configuration.

| Amino acid sequence of SNK0001AR | | |
|---|---|---|
| Heavy chain | Full-length sequence | MEWIWIFLFILSGTAGVQSQVQLQQSGAELARPGASVKLSCKASGYTFT SYGISWVKQRTGQGLEWIGEIYPRSGNTYYNEKFKGKATLTADKSSSTA YMELRSLTSEDSAVYFCARLASSYYGSSYDWYFDVWGTGTTVTVSSESA RNPTIYPLTLPRALSSDPVIIGCLIHDYFPSGTMNVTWGKSGKDITTVN FPPALASGGGYTMSSQLTLPAVECPEGESVKCSVQHDSNAVQELDVKCS GPPPPCPPCPPSCHPSLSLQRPALEDLLLGSDASLTCTLNGLRNPEGAV FTWEPSTGKDAVQKKAVQNSCGCYSVSSVLPGCAERWNSGASFKCTVTH PESDTLTGTIAKITVNTFPPQVHLLPPPSEELALNELVSLTCLVRAFNP KEVLVRWLHGNEELSPESYLVFEPLKEPGEGATTYLVTSVLRVSAELWK QGDQYSCMVGHEALPMNFTQKTIDRLSGKPTNVSVSVIMSEGDGICY |
| | Variable region | MEWIWIFLFILSGTAGVQSQVQLQQSGAELARPGASVKLSCKASGYTFT SYGISWVKQRTGQGLEWIGEIYPRSGNTYYNEKFKGKATLTADKSSSTA YMELRSLTSEDSAVYFCARLASSYYGSSYDWYFDVWGTGTTVTVSS |
| | CDR1 | SYGIS |
| | CDR2 | EIYPRSGNTYYNEKFK |
| | CDR3 | FCARLASS |
| Light chain | Full-length sequence | MRTPAQFLGILLLWFPGIKCDIKMTQSPSSMYASLGERVTITCKASQDI NSYLSWFQQKPGKSPKTLIYRANRLVDGVPSRFSGSGSGQDYSLTISSL EYEDMGIYYCLQYDEFPLTFGAGTKLELKRADAAPTVSIFPPSSEQLTS GGASVVCFLNNFYPKDINVKWKIDGSERQNGVLNSWTDQDSKDSTYSMS STLTLTKDEYERHNSYTCEATHKTSTSPIVKSFNRNEC |
| | Variable region | MRTPAQFLGILLLWFPGIKCDIKMTQSPSSMYASLGERVTITCKASQDI NSYLSWFQQKPGKSPKTLIYRANRLVDGVPSRFSGSGSGQDYSLTISSL EYEDMGIYYCLQYDEFPLTFGAGTKLELK |
| | CDR1 | KASQDINSYLS |
| | CDR2 | RANRLVD |
| | CDR3 | LQYDEFPLT |

In one aspect, the antibody binding to a C. difficile bacterial body of the present invention includes a heavy chain variable region comprising an amino acid sequence of a heavy chain CDR1, an amino acid sequence of a heavy chain CDR2, and an amino acid sequence of a heavy chain CDR3, the heavy chain variable region comprising an amino acid sequence set forth in SEQ ID NO: 17, and
a light chain variable region comprising an amino acid sequence of a light chain CDR1, an amino acid sequence of a light chain CDR2, and an amino acid sequence of a light chain CDR3, the light chain variable region comprising an amino acid sequence set forth in SEQ ID NO: 18.

| Sequence name (SEQ ID NO:) | Amino acid sequence |
|---|---|
| SNK0002_HV (SEQ ID NO: 17) | MGRLTSSFLLLIVPAYVLSQVTLKESGPGILQPSQ TLSLTCSFSGFSLSTFGMGVGWIRQPSGKGLEWLA HIWWDDDKYYNPALKSRLTISKDTSKNQVFLKIAN VDTADTATYYCARIAGFDYWGQGTTLTVSS |
| SNK0002_LV (SEQ ID NO: 18) | MHFQVQIFSFLLISASVIMSRGQIVLTQSPAIMSA SPGEKVTITCSASSSVSYMHWFQQKPGTSPKLWIY STSNLASGVPARFSGSGSGTSYSLTISRMEAEDAA TYYCQQRSSYPYTFGGGTKLEIK |

In one aspect, the antibody binding to a C. difficile bacterial body of the present invention includes:
a heavy chain variable region comprising:
a heavy chain CDR1 comprising an amino acid sequence set forth in SEQ ID NO: 11;
a heavy chain CDR2 comprising an amino acid sequence set forth in SEQ ID NO: 12; and
a heavy chain CDR3 comprising an amino acid sequence set forth in SEQ ID NO: 13; and
a light chain variable region comprising:
a light chain CDR1 comprising an amino acid sequence set forth in SEQ ID NO: 14;
a light chain CDR2 comprising an amino acid sequence set forth in SEQ ID NO: 15; and
a light chain CDR3 comprising an amino acid sequence set forth in SEQ ID NO: 16.

| Sequence name (SEQ ID NO:) | Amino acid sequence |
|---|---|
| SNK0002_HCDR1 (SEQ ID NO: 11) | TFGMG |
| SNK0002_HCDR2 (SEQ ID NO: 12) | LAHIWWDDDKYYNPAL |
| SNK0002_HCDR3 (SEQ ID NO: 13) | YYCARIAG |
| SNK0002_LCDR1 (SEQ ID NO: 14) | SASSSVSYMHW |
| SNK0002_LCDR2 (SEQ ID NO: 15) | STSNLASG |
| SNK0002_LCDR3 (SEQ ID NO: 16) | QRSSYPYTF |

In one aspect, the antibody binding to a C. difficile bacterial body of the present invention includes:
a heavy chain variable region comprising an amino acid sequence set forth in SEQ ID NO: 17 or a sequence having at least 90%, at least 95%, at least 98%, or at least 99% sequence identity therewith; and
a light chain variable region comprising an amino acid sequence set forth in SEQ ID NO: 18 or a sequence having at least 90%, at least 95%, at least 98%, or at least 99% sequence identity therewith.

In one aspect, the antibody binding to a C. difficile bacterial body of the present invention is IgM antibody SNK0002M produced by a hybridoma obtained from B cells derived from spleen. A recombinant purified antibody SNK0002AR produced by determining a nucleic acid sequence encoding the antibody SNK0002M and applying a recombination technique, and a recombinant purified antibody SNK0002MR as an IgA antibody can also be used in the same manner.

The antibody SNK0002AR has the following amino acid sequence configuration.

| Amino acid sequence of SNK0002AR | | |
|---|---|---|
| Heavy chain | Full-length sequence | MGRLTSSFLLLIVPAYVLSQVTLKESGPGILQPSQTLSLTCSFSGFSLSTFGMGV GWIRQPSGKGLEWLAHIWWDDDKYYNPALKSRLTISKDTSKNQVFLKIANVDTAD TATYYCARIAGFDYWGQGTTLTVSSESARNPTIYPLTLPRALSSDPVIIGCLIHD YFPSGTMNVTWGKSGKDITTVNFPPALASGGGYTMSSQLTLPAVECPEGESVKCS VQHDSNAVQELDVKCSGPPPPCPPCPPSCHPSLSLQRPALEDLLLGSDASLTCTL NGLRNPEGAVFTWEPSTGKDAVQKKAVQNSCGCYSVSSVLPGCAERWNSGASFKC TVTHPESDTLTGTIAKITVNTFPPQVHLLPPPSEELALNELVSLTCLVRAFNPKE VLVRWLHGNEELSPESYLVFEPLKEPGEGATTYLVTSVLRVSAELWKQGDQYSCM VGHEALPMNFTQKTIDRLSGKPTNVSVSVIMSEGDGICY |
| | Variable region | MGRLTSSFLLLIVPAYVLSQVTLKESGPGILQPSQTLSLTCSFSGFSLSTFGMGV GWIRQPSGKGLEWLAHIWWDDDKYYNPALKSRLTISKDTSKNQVFLKIANVDTAD TATYYCARIAGFDYWGQGTTLTVSS |
| | CDR1 | TFGMG |
| | CDR2 | LAHIWWDDDKYYNPAL |
| | CDR3 | YYCARIAG |
| Light chain | Full-length sequence | MHFQVQIFSFLLISASVIMSRGQIVLTQSPAIMSASPGEKVTITCSASSSVSYMH WFQQKPGTSPKLWIYSTSNLASGVPARFSGSGSGTSYSLTISRMEAEDAATYYCQ QRSSYPYTFGGGTKLEIKRADAAPTVSIFPPSSEQLTSGGASVVCFLNNFYPKDI NVKWKIDGSERQNGVLNSWTDQDSKDSTYSMSSTLTLTKDEYERHNSYTCEATHK TSTSPIVKSFNRNEC |
| | Variable region | MHFQVQIFSFLLISASVIMSRGQIVLTQSPAIMSASPGEKVTITCSASSSVSYMH WFQQKPGTSPKLWIYSTSNLASGVPARFSGSGSGTSYSLTISRMEAEDAATYYCQ QRSSYPYTFGGGTKLEIK |
| | CDR1 | SASSSVSYMHW |
| | CDR2 | STSNLASG |
| | CDR3 | QRSSYPYTF |

In one aspect, the antibody binding to a C. difficile bacterial body of the present invention includes a heavy chain variable region comprising an amino acid sequence of a heavy chain CDR1, an amino acid sequence of a heavy chain CDR2, and an amino acid sequence of a heavy chain CDR3, the heavy chain variable region comprising an amino acid sequence set forth in SEQ ID NO: 27, and
a light chain variable region comprising an amino acid sequence of a light chain CDR1, an amino acid sequence of a light chain CDR2, and an amino acid sequence of a light chain CDR3, the light chain variable region comprising an amino acid sequence set forth in SEQ ID NO: 28.

| Sequence name (SEQ ID NO:) | Amino acid sequence |
|---|---|
| SNK0003_HV (SEQ ID NO: 27) | MGFSRIFLFLLSVTTGVHSQAYLQQSGAELVRPGAS VRMSCKASDYTFTSYNIHWVKQTPRQGLEWIGAIYS GNGATSHNQKFKGRATLTVDKSSSTAYMQLSSLTSE DSAVYFCTRVGLRSPFDFWGQGTTLTVSS |
| SNK0003_LV (SEQ ID NO: 28) | MESQTQVFVYMLLWLSGVDGDIVMTQSLKFMST SGGDRVSVTCKASQSVGTSVAWYQQKPGQSPKP LIYSASYRYSGVPDRFTGSGSGTDFSLTISNVQ SEDLAEYFCQQYNNYPYTFGGGTKLEIK |

In one aspect, the antibody binding to a C. difficile bacterial body of the present invention includes:
a heavy chain variable region comprising:
a heavy chain CDR1 comprising an amino acid sequence set forth in SEQ ID NO: 21;
a heavy chain CDR2 comprising an amino acid sequence set forth in SEQ ID NO: 22; and
a heavy chain CDR3 comprising an amino acid sequence set forth in SEQ ID NO: 23; and
a light chain variable region comprising:
   a light chain CDR1 comprising an amino acid sequence set forth in SEQ ID NO: 24;
   a light chain CDR2 comprising an amino acid sequence set forth in SEQ ID NO: 25; and
   a light chain CDR3 comprising an amino acid sequence set forth in SEQ ID NO: 26.

| Sequence name (SEQ ID NO:) | Amino acid sequence |
|---|---|
| SNK0003_HCDR1 (SEQ ID NO: 21) | SYNIH |
| SNK0003_HCDR2 (SEQ ID NO: 22) | AIYSGNGATSHNQKFK |
| SNK0003_HCDR3 (SEQ ID NO: 23) | FCTRVGLR |
| SNK0003_LCDR1 (SEQ ID NO: 24) | KASQSVGTSVA |
| SNK0003_LCDR2 (SEQ ID NO: 25) | SASYRYS |
| SNK0003_LCDR3 (SEQ ID NO: 26) | QQYNNYPYT |

In one aspect, the antibody binding to a C. difficile bacterial body of the present invention includes:
a heavy chain variable region comprising an amino acid sequence set forth in SEQ ID NO: 27 or a sequence having at least 90%, at least 95%, at least 98%, or at least 99% sequence identity therewith; and
a light chain variable region comprising an amino acid sequence set forth in SEQ ID NO: 28 or a sequence having at least 90%, at least 95%, at least 98%, or at least 99% sequence identity therewith.

In one aspect, the antibody binding to a C. difficile bacterial body of the present invention is IgA antibody SNK0003A produced by a hybridoma obtained from B cells derived from intestinal mucosa lamina propria. A recombinant purified antibody SNK0003MR produced by determining a nucleic acid sequence encoding the antibody SNK0003M and applying a recombination technique, and a recombinant purified antibody SNK0003AR as an IgA antibody can also be used in the same manner.

The antibody SNK0003A has the following amino acid sequence configuration.

| Amino acid sequence of SNK0003A | | |
|---|---|---|
| Heavy chain | Full-length sequence | MGFSRIFLFLLSVTTGVHSQAYLQQSGAELVRPGASVRMSCKASDYTFTSYNIHW VKQTPRQGLEWIGAIYSGNGATSHNQKFKGRATLTVDKSSSTAYMQLSSLTSEDS AVYFCTRVGLRSPFDFWGQGTTLTVSSESARNPTIYPLTLPRALSSDPVIIGCLI HDYFPSGTMNVTWGKSGKDITTVNFPPALASGGGYTMSSQLTLPAVECPEGESVK CSVQHDSNAVQELDVKCSGPPPPCPPCPPSCHPSLSLQRPALEDLLLGSDASLTC TLNGLRNPEGAVFTWEPSTGKDAVQKKAVQNSCGCYSVSSVLPGCAERWNSGASF KCTVTHPESDTLTGTIAKITVNTFPPQVHLLPPPSEELALNELVSLTCLVRAFNP KEVLVRWLHGNEELSPESYLVFEPLKEPGEGATTYLVTSVLRVSAELWKQGDQYS CMVGHEALPMNFTQKTIDRLSGKPTNVSVSVIMSEGDGICY |
| | Variable region | MGFSRIFLFLLSVTTGVHSQAYLQQSGAELVRPGASVRMSCKASDYTFTSYNIHW VKQTPRQGLEWIGAIYSGNGATSHNQKFKGRATLTVDKSSSTAYMQLSSLTSEDS AVYFCTRVGLRSPFDFWGQGTTLTVSS |
| | CDR1 | SYNIH |
| | CDR2 | AIYSGNGATSHNQKFK |
| | CDR3 | FCTRVGLR |
| Light chain | Full-length sequence | MESQTQVFVYMLLWLSGVDGDIVMTQSLKFMSTSGGDRVSVTCKASQSVGTSVAW YQQKPGQSPKPLIYSASYRYSGVPDRFTGSGSGTDFSLTISNVQSEDLAEYFCQQ YNNYPYTFGGGTKLEIKRADAAPTVSIFPPSSEQLTSGGASVVCFLNNFYPKDIN VKWKIDGSERQNGVLNSWTDQDSKDSTYSMSSTLTLTKDEYERHNSYTCEATHKT STSPIVKSFNRNEC |
| | Variable region | MESQTQVFVYMLLWLSGVDGDIVMTQSLKFMSTSGGDRVSVTCKASQSVGTSVAW YQQKPGQSPKPLIYSASYRYSGVPDRFTGSGSGTDFSLTISNVQSEDLAEYFCQQ YNNYPYTFGGGTKLEIK |
| | CDR1 | KASQSVGTSVA |
| | CDR2 | SASYRYS |
| | CDR3 | QQYNNYPYT |

In one aspect, the monoclonal antibody binding to a C. difficile bacterial body of the present invention is an antibody comprising a heavy chain variable region comprising:
a heavy chain CDR1 comprising an amino acid sequence set forth in SEQ ID NO: 31;
a heavy chain CDR2 comprising an amino acid sequence set forth in SEQ ID NO: 32; and
a heavy chain CDR3 comprising an amino acid sequence set forth in SEQ ID NO: 33, and
a light chain variable region comprising:
   a light chain CDR1 comprising an amino acid sequence set forth in SEQ ID NO: 34;
   a light chain CDR2 comprising an amino acid sequence set forth in SEQ ID NO: 35; and
   a light chain CDR3 comprising an amino acid sequence set forth in SEQ ID NO: 36.

In one aspect, the antibody binding to a C. difficile bacterial body of the present invention includes:
a heavy chain variable region comprising an amino acid sequence set forth in SEQ ID NO: 37 or a sequence having at least 90%, at least 95%, at least 98%, or at least 99% sequence identity therewith; and
a light chain variable region comprising an amino acid sequence set forth in SEQ ID NO: 38 or a sequence having at least 90%, at least 95%, at least 98%, or at least 99% sequence identity therewith.

In one aspect, the antibody binding to a C. difficile bacterial body of the present invention is, as a reference antibody comprising a heavy chain variable region comprising an amino acid sequence set forth in SEQ ID NO: 37, and
a light chain variable region comprising an amino acid sequence set forth in SEQ ID NO: 38,
an antibody comprising at least one amino acid mutation in at least one region selected from heavy chains CDR1 to 3, light chains CDR1 to 3, and a light chain FR1, and
binding to the amino acid sequence RQEEHIELIAS (SEQ ID NO: 72) in E. coli SHMT protein and the amino acid sequence VLDMMKLEKPE (SEQ ID NO: 73) in C. difficile iPGM protein.

| Sequence name (SEQ ID NO:) | Amino acid sequence |
|---|---|
| W27G2_HV (SEQ ID NO: 37) | MKCSWIIFFLMAVVTGVNSEVQLQQSGSEL VKSGASVKLSCTVSGFNFTDYYIHWVRQRT EQGLEWIGRIDPENDETTYAPKFQGKATMT ADTSSNTAYLQLTSLTSEDTAVYYCARSTV LDYWGHGTTLTVSS |
| W27G2_LV (SEQ ID NO: 38) | MKLPVRLLVLMFWIPGFSSDVLMTQTPLSL PVSLGDQASISCRASQSIVHTNGNTYLEWY LQKPGQSPKLLIYKVSNRFSGVPDRFSGSG SGTDFILKISRVEAEDLGVYYCFQGSHVPP TFGGGTKLEVK |

The at least one amino acid mutation of the antibody can be identified by applying a technique available to those skilled in the art by using results of three-dimensional structure analysis of conjugates of an antibody comprising an amino acid sequence of the reference antibody and E. coli SHMT protein, and an antibody comprising an amino acid sequence of the reference antibody and C. difficile iPGM protein. The number of amino acid mutations to the reference antibody may be 1, 2, 3, 4, 5, 6, 7, 8, 9, 10, 11, 12, 13, 14, 15, 16, 17, 18, 19, 20, or more.

Such a reference antibody is not particularly limited as long as it is an antibody comprising W27G2_HV (SEQ ID NO: 37) as a heavy chain variable region and W27G2_LV (SEQ ID NO: 38) as a light chain variable region. For example, an IgG antibody (W27RS_H000_L000GR) can be used. The antibody W27RS_H000_L000GR includes the same heavy chain variable region and light chain variable region as the antibody W27G2 produced by the hybridoma obtained from B cells derived from intestinal mucosa lamina propria.

| Amino acid sequence of W27RS_H000_L000GR | | |
|---|---|---|
| Heavy chain | Variable region | MKCSWIIFFLMAVVTGVNSEVQLQQSGSELVKSGASVKLSCTVSGFNFTDYYIHW VRQRTEQGLEWIGRIDPENDETTYAPKFQGKATMTADTSSNTAYLQLTSLTSEDT AVYYCARSTVLDYWGHGTTLTVSS |
| | CDR1 | DYYIH |
| | CDR2 | RIDPENDETTYAPKFQ |
| | CDR3 | YCARSTVL |
| Light chain | Variable region | MKLPVRLLVLMFWIPGFSSDVLMTQTPLSLPVSLGDQASISCRASQSIVHTNGNTYLE WYLQKPGQSPKLLIYKVSNRFSGVPDRFSGSGSGTDFILKISRVEAEDLGVYYCFQGS HVPPTFGGGTKLEVK |
| | CDR1 | RASQSIVHTNG |
| | CDR2 | KLLIYKV |
| | CDR3 | GVYYCFQGS |

It can be confirmed by a method well known to those skilled in the art, such as ELISA or Western blot, that an antibody comprising an identified mutation actually binds to the amino acid sequence RQEEHIELIAS in E. coli SHMT protein and the amino acid sequence VLDMMKLEKPE in C. difficile iPGM protein.

In one aspect, the antibody binding to a C. difficile bacterial body of the present invention is an antibody comprising:
a heavy chain variable region comprising:
a heavy chain CDR1 comprising an amino acid sequence of X₁YYIH;
a heavy chain CDR2 comprising an amino acid sequence of RIDPENX₂X₃TTYAPKFQ; and
a heavy chain CDR3 comprising an amino acid sequence of YCARSTVL; and
a light chain variable region comprising:
   a light chain CDR1 comprising an amino acid sequence of RX₄SQSIVHTNG;
   a light chain CDR2 comprising an amino acid sequence of KLLIYKV; and
   a light chain CDR3 comprising an amino acid sequence of GVYYFQGS,
   in which a light chain FR1 contains an amino acid sequence of TPLSLPVSLGDQA or an amino acid sequence of SPASX₅SVSLGDRX₆,
   X₁, X₂, and X₃ are each independently a neutral polar amino acid or an acidic polar amino acid, X₄ is a non-polar amino acid or a neutral polar amino acid, and X₅ and X₆ are each independently a non-polar amino acid.

The antibody is designed to have a binding mode similar to that of the W27G2 antibody with respect to the amino acid sequence RQEEHIELIAS in E. coli SHMT protein and the amino acid sequence VLDMMKLEKPE in C. difficile iPGM protein by three-dimensional structure analysis, and actually exhibits a binding mode similar to that of the W27G2 antibody and a bacterial growth inhibitory effect on these bacteria.

In one aspect, the antibody binding to a C. difficile bacterial body of the present invention is an antibody comprising:
a heavy chain variable region comprising:
a heavy chain CDR1 comprising an amino acid sequence of X₁YYIH;
a heavy chain CDR2 comprising an amino acid sequence of RIDPENX₂X₃TTYAPKFQ; and
a heavy chain CDR3 comprising an amino acid sequence of YCARSTVL; and
a light chain variable region comprising:
   a light chain CDR1 comprising an amino acid sequence of RX₄SQSIVHTNG;
   a light chain CDR2 comprising an amino acid sequence of KLLIYKV; and
   a light chain CDR3 comprising an amino acid sequence of GVYYFQGS,
   in which a light chain FR1 contains an amino acid sequence of TPLSLPVSLGDQA or an amino acid sequence of SPASX₅SVSLGDRX₆,
   X₁ and X₂ are each independently asparagine or aspartic acid, X₃ is glutamine or glutamic acid, X₄ is alanine or serine, X₅ is leucine or methionine, and X₆ is alanine or valine.

In one aspect, the heavy chain of the antibody binding to a C. difficile bacterial body of the present invention includes:
a heavy chain CDR1 comprising an amino acid sequence set forth in SEQ ID NO: 31 or 41;
a heavy chain CDR2 comprising an amino acid sequence set forth in any one of SEQ ID NOs: 32 and 42 to 44; and
a heavy chain CDR3 comprising an amino acid sequence set forth in SEQ ID NO: 33.

| Sequence name (SEQ ID NO:) | Amino acid sequence |
|---|---|
| W27G2_HCDR1 (SEQ ID NO: 31) | DYYIH |
| W27G2_HCDR2 (SEQ ID NO: 32) | RIDPENDETTYAPKFQ |
| W27G2_HCDR3 (SEQ ID NO: 33) | YCARSTVL |
| RS_HCDR1_D50N (SEQ ID NO: 41) | NYYIH |
| RS_HCDR2_D75N (SEQ ID NO: 42) | RIDPENNETTYAPKFQ |
| RS_HCDR2_E76Q (SEQ ID NO: 43) | RIDPENDQTTYAPKFQ |
| RS_HCDR2_D75N_E76Q (SEQ ID NO: 44) | RIDPENNQTTYAPKFQ |

In one aspect, the heavy chain of the antibody binding to a C. difficile bacterial body of the present invention includes:
a heavy chain CDR1 comprising an amino acid sequence set forth in SEQ ID NO: 31;
a heavy chain CDR2 comprising an amino acid sequence set forth in SEQ ID NO: 32; and
a heavy chain CDR3 comprising an amino acid sequence set forth in SEQ ID NO: 33.

In one aspect, the heavy chain of the antibody binding to a C. difficile bacterial body of the present invention includes:
a heavy chain CDR1 comprising an amino acid sequence set forth in SEQ ID NO: 31;
a heavy chain CDR2 comprising an amino acid sequence set forth in SEQ ID NO: 42; and
a heavy chain CDR3 comprising an amino acid sequence set forth in SEQ ID NO: 33.

In one aspect, the heavy chain of the antibody binding to a C. difficile bacterial body of the present invention includes:
a heavy chain CDR1 comprising an amino acid sequence set forth in SEQ ID NO: 31;
a heavy chain CDR2 comprising an amino acid sequence set forth in SEQ ID NO: 43; and
a heavy chain CDR3 comprising an amino acid sequence set forth in SEQ ID NO: 33.

In one aspect, the heavy chain of the antibody binding to a C. difficile bacterial body of the present invention includes:
a heavy chain CDR1 comprising an amino acid sequence set forth in SEQ ID NO: 31;
a heavy chain CDR2 comprising an amino acid sequence set forth in SEQ ID NO: 44; and
a heavy chain CDR3 comprising an amino acid sequence set forth in SEQ ID NO: 33.

In one aspect, the heavy chain of the antibody binding to a C. difficile bacterial body of the present invention includes:
a heavy chain CDR1 comprising an amino acid sequence set forth in SEQ ID NO: 41;
a heavy chain CDR2 comprising an amino acid sequence set forth in SEQ ID NO: 32; and
a heavy chain CDR3 comprising an amino acid sequence set forth in SEQ ID NO: 33.

In one aspect, the heavy chain of the antibody binding to a C. difficile bacterial body of the present invention includes:
a heavy chain CDR1 comprising an amino acid sequence set forth in SEQ ID NO: 41;
a heavy chain CDR2 comprising an amino acid sequence set forth in SEQ ID NO: 42; and
a heavy chain CDR3 comprising an amino acid sequence set forth in SEQ ID NO: 33.

In one aspect, the heavy chain of the antibody binding to a C. difficile bacterial body of the present invention includes:
a heavy chain CDR1 comprising an amino acid sequence set forth in SEQ ID NO: 41;
a heavy chain CDR2 comprising an amino acid sequence set forth in SEQ ID NO: 43; and
a heavy chain CDR3 comprising an amino acid sequence set forth in SEQ ID NO: 33.

In one aspect, the heavy chain of the antibody binding to a C. difficile bacterial body of the present invention includes:
a heavy chain CDR1 comprising an amino acid sequence set forth in SEQ ID NO: 41;
a heavy chain CDR2 comprising an amino acid sequence set forth in SEQ ID NO: 44; and
a heavy chain CDR3 comprising an amino acid sequence set forth in SEQ ID NO: 33.

In one aspect, the light chain of the antibody binding to a C. difficile bacterial body of the present invention includes:
a light chain CDR1 comprising an amino acid sequence set forth in SEQ ID NO: 34 or 52;
a light chain CDR2 comprising an amino acid sequence set forth in SEQ ID NO: 35; and
a light chain CDR3 comprising an amino acid sequence set forth in SEQ ID NO: 36.

| Sequence name (SEQ ID NO:) | Amino acid sequence |
|---|---|
| W27G2_LCDR1 (SEQ ID NO: 34) | RASQSIVHTNG |
| W27G2_LCDR2 (SEQ ID NO: 35) | KLLIYKV |
| W27G2_LCDR3 (SEQ ID NO: 36) | GVYYCFQGS |
| RS_LCDR1_A44S (SEQ ID NO: 52) | RSSQSIVHTNG |

In one aspect, the light chain of the antibody binding to a C. difficile bacterial body of the present invention includes:
a light chain CDR1 comprising an amino acid sequence set forth in SEQ ID NO: 34;
a light chain CDR2 comprising an amino acid sequence set forth in SEQ ID NO: 35; and
a light chain CDR3 comprising an amino acid sequence set forth in SEQ ID NO: 36.

In one aspect, the light chain of the antibody binding to a C. difficile bacterial body of the present invention includes:
a light chain CDR1 comprising an amino acid sequence set forth in SEQ ID NO: 52;
a light chain CDR2 comprising an amino acid sequence set forth in SEQ ID NO: 35; and
a light chain CDR3 comprising an amino acid sequence set forth in SEQ ID NO: 36.

In one aspect, the antibody binding to a C. difficile bacterial body of the present invention or the antigen-binding fragment thereof contains a sequence that binds to Protein L in the light chain variable region. The antibody or the antigen-binding fragment thereof comprising the sequence that binds to Protein L can be purified using a Protein L column.

In one aspect, the antibody binding to a C. difficile bacterial body and comprising a sequence that binds to Protein L of the present invention or the antigen-binding fragment thereof includes a light chain variable region comprising the SPASX₅SVSLGDRX₆ amino acid sequence, in which X₅ and X₆ are each independently a non-polar amino acid.

In one aspect, the antibody binding to a C. difficile bacterial body and comprising a sequence that binds to Protein L of the present invention or the antigen-binding fragment thereof includes a light chain variable region comprising an amino acid sequence of SPASX₅SVSLGDRX₆, in which X₅ is leucine or methionine and X₆ is alanine or valine.

In one aspect, the antibody binding to a C. difficile bacterial body and comprising a sequence that binds to Protein L of the present invention or the antigen-binding fragment thereof includes a light chain variable region comprising an amino acid sequence set forth in one selected from the group consisting of SEQ ID NOs: 53 to 55.

| Sequence name (SEQ ID NO:) | Amino acid sequence |
|---|---|
| RS_LFR1_L001 (SEQ ID NO: 53) | SPASLSVSLGDRA |
| RS_LFR1_L002 (SEQ ID NO: 54) | SPASLSVSLGDRV |
| RS_LFR1_L003 (SEQ ID NO: 55) | SPASMSVSLGDRA |
| RS_LFR1_L000 (SEQ ID NO: 56) | TPLSLPVSLGDQA |

In one aspect, the antibody binding to a C. difficile bacterial body and comprising a sequence that binds to Protein L of the present invention or the antigen-binding fragment thereof includes a light chain variable region comprising an amino acid sequence set forth in SEQ ID NO: 54.

The sequence is obtained by introducing a mutation into the sequence TPLSLPVSLGDQA (SEQ ID NO: 56) of the light chain FR1 region of the W27G2 antibody so that Protein L binds to the antibody molecule.

In one aspect, the antibody binding to a C. difficile bacterial body and comprising a sequence that binds to Protein L of the present invention or the antigen-binding fragment thereof includes a light chain variable region comprising an amino acid sequence set forth in SEQ ID NO: 55.

This sequence is obtained by further introducing a mutation into the light chain FR1 region of the light chain variable region comprising an amino acid sequence set forth in SEQ ID NO: 54 so that Protein L binds to the antibody molecule more firmly.

In one aspect, the antibody binding to a C. difficile bacterial body and comprising a sequence that binds to Protein L of the present invention or the antigen-binding fragment thereof includes a light chain variable region comprising an amino acid sequence set forth in SEQ ID NO: 58.

This sequence is obtained by further introducing a mutation into the light chain FR1 region of the light chain variable region comprising an amino acid sequence set forth in SEQ ID NO: 54 so that Protein L binds to the antibody molecule more firmly.

The antibody binding to a C. difficile bacterial body of the present invention may include any combination of the heavy chain and the light chain described above, or may not contain a sequence that binds to Protein L.

In one aspect, the antibody binding to a C. difficile bacterial body of the present invention or the antigen-binding fragment thereof is an antibody comprising:
a heavy chain variable region comprising:
a heavy chain CDR1 comprising an amino acid sequence set forth in SEQ ID NO: 31 or 41;
a heavy chain CDR2 comprising an amino acid sequence set forth in any one of SEQ ID NOs: 32 and 42 to 44; and
a heavy chain CDR3 comprising an amino acid sequence set forth in SEQ ID NO: 33; and
a light chain variable region comprising:
   a light chain CDR1 comprising an amino acid sequence set forth in SEQ ID NO: 34 or 52;
   a light chain CDR2 comprising an amino acid sequence set forth in SEQ ID NO: 35;
   a light chain CDR3 comprising an amino acid sequence set forth in SEQ ID NO: 36; and
   a light chain FR1 comprising an amino acid sequence set forth in any one of SEQ ID NOs: 53 to 56.

For example, the antibody binding to a C. difficile bacterial body of the present invention includes a combination of any of the following heavy chain variable regions and any of the following light chain variable regions.

| Heavy chain variable region | Amino acid sequence |
|---|---|
| W27G2_HV(SEQ ID NO: 37) | MKCSWIIFFLMAVVTGVNSEVQLQQSGSELVKSGASVKLS CTVSGFNFTDYYIHWVRQRTEQGLEWIGRIDPENDETTYA PKFQGKATMTADTSSNTAYLQLTSLTSEDTAVYYCARSTV LDYWGHGTTLTVSS |
| RS_HV001 (SEQ ID NO: 45) | MKCSWIIFFLMAVVTGVNSEVQLQQSGSELVKSGASVKLS CTVSGFNFTNYYIHWVRQRTEQGLEWIGRIDPENDETTYA PKFQGKATMTADTSSNTAYLQLTSLTSEDTAVYYCARSTV LDYWGHGTTLTVSS |
| RS_HV002 (SEQ ID NO: 46) | MKCSWIIFFLMAVVTGVNSEVQLQQSGSELVKSGASVKLS CTVSGFNFTDYYIHWVRQRTEQGLEWIGRIDPENNETTYA PKFQGKATMTADTSSNTAYLQLTSLTSEDTAVYYCARSTV LDYWGHGTTLTVSS |
| RS_HV003 (SEQ ID NO: 47) | MKCSWIIFFLMAVVTGVNSEVQLQQSGSELVKSGASVKLS CTVSGFNFTDYYIHWVRQRTEQGLEWIGRIDPENDQTTYA PKFQGKATMTADTSSNTAYLQLTSLTSEDTAVYYCARSTV LDYWGHGTTLTVSS |
| RS_HV004 (SEQ ID NO: 48) | MKCSWIIFFLMAVVTGVNSEVQLQQSGSELVKSGASVKLS CTVSGFNFTNYYIHWVRQRTEQGLEWIGRIDPENNETTYA PKFQGKATMTADTSSNTAYLQLTSLTSEDTAVYYCARSTV LDYWGHGTTLTVSS |
| RS_HV005 (SEQ ID NO: 49) | MKCSWIIFFLMAVVTGVNSEVQLQQSGSELVKSGASVKLS CTVSGFNFTNYYIHWVRQRTEQGLEWIGRIDPENDQTTYA PKFQGKATMTADTSSNTAYLQLTSLTSEDTAVYYCARSTV LDYWGHGTTLTVSS |
| RS_HV006 (SEQ ID NO: 50) | MKCSWIIFFLMAVVTGVNSEVQLQQSGSELVKSGASVKLS CTVSGFNFTDYYIHWVRQRTEQGLEWIGRIDPENNQTTYA PKFQGKATMTADTSSNTAYLQLTSLTSEDTAVYYCARSTV LDYWGHGTTLTVSS |
| RS_HV007 (SEQ ID NO: 51) | MKCSWIIFFLMAVVTGVNSEVQLQQSGSELVKSGASVKLS CTVSGFNFTNYYIHWVRQRTEQGLEWIGRIDPENNQTTYA PKFQGKATMTADTSSNTAYLQLTSLTSEDTAVYYCARSTV LDYWGHGTTLTVSS |

| Light chain variable region | Amino acid sequence |
|---|---|
| W27G2_LV (SEQ ID NO: 38) | MKLPVRLLVLMFWIPGFSSDVLMTQTPASLPVSLGDQASISC RASQSIVHTNGNTYLEWYLQKPGQSPKLLIYKVSNRFSGVPD RFSGSGSGTDFILKISRVEAEDLGVYYCFQGSHVPPTFGGGT KLEVK |
| RS_LV001 (SEQ ID NO: 57) | MKLPVRLLVLMFWIPGFSSDVLMTQSPASLSVSLGDRATISC RASQSIVHTNGNTYLEWYLQKPGQSPKLLIYKVSNRFSGVPD RFSGSGSGTDFILKISRVEAEDLGVYYCFQGSHVPPTFGGGT KLEVK |
| RS_LV002 (SEQ ID NO: 58) | MKLPVRLLVLMFWIPGFSSDVLMTQSPASLSVSLGDRVTISC RASQSIVHTNGNTYLEWYLQKPGQSPKLLIYKVSNRFSGVPD RFSGSGSGTDFILKISRVEAEDLGVYYCFQGSHVPPTFGGGT KLEVK |
| RS_LV003 (SEQ ID NO: 59) | MKLPVRLLVLMFWIPGFSSDVLMTQSPASLSVSLGDRVTISC RSSQSIVHTNGNTYLEWYLQKPGQSPKLLIYKVSNRFSGVPD RFSGSGSGTDFILKISRVEAEDLGVYYCFQGSHVPPTFGGGT KLEVK |
| RS_LV004 (SEQ ID NO: 60) | MKLPVRLLVLMFWIPGFSSDVLMTQSPASMSVSLGDRATISC RSSQSIVHTNGNTYLEWYLQKPGQSPKLLIYKVSNRFSGVPD RFSGSGSGTDFILKISRVEAEDLGVYYCFQGSHVPPTFGGGT KLEVK |
| RS_LV005 (SEQ ID NO: 61) | MKLPVRLLVLMFWIPGFSSDVLMTQSPASLSVSLGDRATISC RSSQSIVHTNGNTYLEWYLQKPGQSPKLLIYKVSNRFSGVPD RFSGSGSGTDFILKISRVEAEDLGVYYCFQGSHVPPTFGGGT KLEVK |

Preferably, the antibody binding to a C. difficile bacterial body of the present invention includes a combination of the following heavy chain variable region and light chain variable region.

| Heavy chain variable region | Light chain variable region |
|---|---|
| W27G2_HV (SEQ ID NO: 37) | RS_LV001(SEQ ID NO: 57) |
| W27G2_HV (SEQ ID NO: 37) | RS_LV004(SEQ ID NO: 60) |
| W27G2_HV (SEQ ID NO: 37) | RS_LV005 (SEQ ID NO: 61) |
| RS_HV001 (SEQ ID NO: 45) | RS_LV005 (SEQ ID NO: 61) |
| RS_HV002 (SEQ ID NO: 46) | RS_LV005 (SEQ ID NO: 61) |
| RS_HV003 (SEQ ID NO: 47) | RS_LV005 (SEQ ID NO: 61) |
| RS_HV004 (SEQ ID NO: 48) | RS_LV005 (SEQ ID NO: 61) |
| RS_HV005 (SEQ ID NO: 49) | RS_LV005 (SEQ ID NO: 61) |
| RS_HV006 (SEQ ID NO: 50) | RS_LV005 (SEQ ID NO: 61) |
| RS_HV007 (SEQ ID NO: 51) | RS_LV001 (SEQ ID NO: 57) |
| RS_HV007(SEQ ID NO: 51) | RS_LV002 (SEQ ID NO: 58) |
| RS_HV007 (SEQ ID NO: 51) | RS_LV003 (SEQ ID NO: 59) |
| RS_HV007 (SEQ ID NO: 51) | RS_LV004 (SEQ ID NO: 60) |
| RS_HV007 (SEQ ID NO: 51) | RS_LV005 (SEQ ID NO: 61) |

Preferably, the antibody binding to a C. difficile bacterial body of the present invention includes the following.

| Antibody name | Heavy chain variable region | Light chain variable region | Explanation |
|---|---|---|---|
| SNK0001M | SNK0001_HV | SNK0001_LV | Hybridoma-producing IgM |
| SNK0001MR | SNK0001_HV | SNK0001_LV | CHO cell-producing recombinant IgM |
| SNK0001MA | SNK0001_HV | SNK0001_LV | CHO cell-producing recombinant IgA |
| SNK0002M | SNK0002_HV | SNK0002_LV | Hybridoma-producing IgM |
| SNK0002MR | SNK0002_HV | SNK0002_LV | CHO cell-producing recombinant IgM |
| SNK0002MA | SNK0002_HV | SNK0002_LV | CHO cell-producing recombinant IgA |
| SNK0003A | SNK0003_HV | SNK0003_LV | Hybridoma-producing IgA |
| SNK0003AR | SNK0003_HV | SNK0003_LV | CHO cell-producing recombinant IgA |
| W27G2 | W27G2_HV | W27G2_LV | Hybridoma-producing IgA |
| RS_H000_L001 | W27G2_HV | RS_LV001 | CHO cell-producing recombinant IgA |
| | | | Protein L binding type |
| RS_H000_L005 | W27G2_HV | RS_LV005 | CHO cell-producing recombinant IgA |
| | | | Protein L binding type |
| | | | Mutation to light chain CDR1 of W27G2 |
| RS_H001_L005 | RS_HV001 | RS_LV005 | CHO cell-producing recombinant IgA |
| | | | Protein L binding type |
| | | | Mutation to heavy chain CDR1 of W27G2 |
| | | | Mutation to light chain CDR1 of W27G2 |
| RS_H002_L005 | RS_HV002 | RS_LV005 | CHO cell-producing recombinant IgA |
| | | | Protein L binding type |
| | | | Mutation to heavy chain CDR2 of W27G2 |
| | | | Mutation to light chain CDR1 of W27G2 |
| RS_H003_L005 | RS_HV003 | RS_LV005 | CHO cell-producing recombinant IgA |
| | | | Protein L binding type |
| | | | Mutation to heavy chain CDR2 of W27G2 |
| | | | Mutation to light chain CDR1 of W27G2 |
| RS_H004_L005 | RS_HV004 | RS_LV005 | CHO cell-producing recombinant IgA |
| | | | Protein L binding type |
| | | | Mutation to heavy chains CDR1 and CDR2 of W27G2 |
| | | | Mutation to light chain CDR1 of W27G2 |
| RS_H005_L005 | RS_HV005 | RS_LV005 | CHO cell-producing recombinant IgA |
| | | | Protein L binding type |
| | | | Mutation to heavy chains CDR1 and CDR2 of W27G2 |
| | | | Mutation to light chain CDR1 of W27G2 |
| RS_H006_L005 | RS_HV006 | RS_LV005 | CHO cell-producing recombinant IgA |
| | | | Protein L binding type |
| | | | Mutation to heavy chain CDR2 of W27G2 |
| | | | Mutation to light chain CDR1 of W27G2 |
| RS_H007_L001 | RS_HV007 | RS_LV001 | CHO cell-producing recombinant IgA |
| | | | Protein L binding type |
| | | | Mutation to heavy chains CDR1 and CDR2 of W27G2 |
| RS_H007_L002 | RS_HV007 | RS_LV002 | CHO cell-producing recombinant IgA |
| | | | Protein L binding type |
| | | | Mutation to heavy chains CDR1 and CDR2 of W27G2 |
| RS_H007_L003 | RS_HV007 | RS_LV003 | CHO cell-producing recombinant IgA |
| | | | Protein L binding type |
| | | | Mutation to heavy chains CDR1 and CDR2 of W27G2 |
| | | | Mutation to light chain CDR1 of W27G2 |
| RS_H007_L004 | RS_HV007 | RS_LV004 | CHO cell-producing recombinant IgA |
| | | | Protein L binding type |
| | | | Mutation to heavy chains CDR1 and CDR2 of W27G2 |
| | | | Mutation to light chain CDR1 of W27G2 |
| RS_H007_L005 | RS_HV007 | RS_LV005 | CHO cell-producing recombinant IgA |
| | | | Protein L binding type |
| | | | Mutation to heavy chains CDR1 and CDR2 of W27G2 |
| | | | Mutation to light chain CDR1 of W27G2 |

An antibody produced in CHO cells by introducing mutations into heavy chains or light chains of the W27G2 antibody so that antigen specificity is not substantially changed is referred to as an RS mutant recombinant purified antibody. These RS mutant recombinant purified antibodies further contain mutations for binding to Protein L.

The antibody binding to a C. difficile bacterial body of the present invention can have multispecificity that binds to a C. difficile bacterial body as well as to other disease-associated bacteria. For example, the antibody binding to a C. difficile bacterial body of the present invention further binds to Fusobacterium nucleatum, which is considered to be a causative bacterium of colorectal cancer, and preferably inhibits its growth.

The antibody binding to a C. difficile bacterial body of the present invention can contain a mutation in a constituent amino acid sequence, for example, a heavy chain variable region, light chain variable region, heavy chains CDR1 to 3, or light chains CDR1 to 3 thereof, as long as its antigen-binding characteristics are not lost. The mutation may be a substitution, a deletion, an insertion, or the like, and is not limited thereto. For example, in the case of the substitution, a conservative substitution technique can be adopted. Furthermore, the binding mode of the antibody and the antigen is analyzed in detail using three-dimensional structural analysis or the like, such that various mutations can be introduced into the antibody binding to a C. difficile bacterial body of the present invention as long as the antigen-binding characteristics are not lost.

### (Nucleic Acid)

A nucleic acid encoding the antibody of the present invention or the antigen-binding fragment thereof may be a ribonucleotide or a deoxynucleotide. In addition, the form of the nucleic acid is not particularly limited, and may be a single-stranded form or a double-stranded form. A codon used in the nucleic acid sequence is not particularly limited, and various codons can be appropriately selected and used according to the purpose. For example, it can be appropriately selected in consideration of codon frequency and the like according to the type of host cell to be adopted at the time of production. In one aspect, the nucleic acid encoding the antibody of the present invention or the antigen-binding fragment thereof is used for expressing and producing the antibody according to the present invention or the antigen-binding fragment thereof.

In a case where a base nucleic acid encoding the antibody of the present invention or the antigen-binding fragment thereof encodes an antigen-binding fragment, for example, two domains of Fv fragments and VL and VH may be encoded by separate nucleic acid molecules, or a single protein chain (single-stranded Fv (scFv)) in which a VL and VH region pair form a monovalent molecule may be encoded by a single nucleic acid using a recombinant technique.

Sequence information on the base nucleic acid encoding the antibody of the present invention or the antigen-binding fragment thereof is exemplified below.
>SEQ ID NO: 1:SNK0001_HCDR1
   SYGIS
>SEQ ID NO: 2: SNK0001_HCDR2
   EIYPRSGNTYYNEKFK
>SEQ ID NO: 3: SNK0001_HCDR3
   FCARLASS
>SEQ ID NO: 4: SNK0001_LCDR1
   KASQDINSYLS
>SEQ ID NO: 5: SNK0001_LCDR2
   RANRLVD
>SEQ ID NO: 6: SNK0001_LCDR3
   LQYDEFPLT
>SEQ ID NO: 7: SNK0001_HV 
MEWIWIFLFILSGTAGVQSQVQLQQSGAELARPGASVKLSCKASGYTFTSYGISWVKQRTGQGLEW

IGEIYPRSGNTYYNEKFKGKATLTADKSSSTAYMELRSLTSEDSAVYFCARLASSYYGSSYDWYFD

VWGTGTTVTVSS
>SEQ ID NO: 8: SNK0001_LV 
MRTPAQFLGILLLWFPGIKCDIKMTQSPSSMYASLGERVTITCKASQDINSYLSWFQQKPGKSPKT
LIYRANRLVDGVPSRFSGSGSGQDYSLTISSLEYEDMGIYYCLQYDEFPLTFGAGTKLELK
 (SNK0001AR antibody heavy chain amino acid sequence)
>SEQ ID NO: 9: SNK0001AR_H 
MEWIWIFLFILSGTAGVQSQVQLQQSGAELARPGASVKLSCKASGYTFTSYGISWVKQRTGQGLEW
IGEIYPRSGNTYYNEKFKGKATLTADKSSSTAYMELRSLTSEDSAVYFCARLASSYYGSSYDWYFD
VWGTGTTVTVSSESARNPTIYPLTLPRALSSDPVIIGCLIHDYFPSGTMNVTWGKSGKDITTVNFP
PALASGGGYTMSSQLTLPAVECPEGESVKCSVQHDSNAVQELDVKCSGPPPPCPPCPPSCHPSLSL
QRPALEDLLLGSDASLTCTLNGLRNPEGAVFTWEPSTGKDAVQKKAVQNSCGCYSVSSVLPGCAER
WNSGASFKCTVTHPESDTLTGTIAKITVNTFPPQVHLLPPPSEELALNELVSLTCLVRAFNPKEVL
VRWLHGNEELSPESYLVFEPLKEPGEGATTYLVTSVLRVSAELWKQGDQYSCMVGHEALPMNFTQK
TIDRLSGKPTNVSVSVIMSEGDGICY
 (SNK0001AR antibody light chain amino acid sequence)
>SEQ ID NO: 10: SNK0001AR_L 
MRTPAQFLGILLLWFPGIKCDIKMTQSPSSMYASLGERVTITCKASQDINSYLSWFQQKPGKSPKT

LIYRANRLVDGVPSRFSGSGSGQDYSLTISSLEYEDMGIYYCLQYDEFPLTFGAGTKLELKRADAA

PTVSIFPPSSEQLTSGGASVVCFLNNFYPKDINVKWKIDGSERQNGVLNSWTDQDSKDSTYSMSST

LTLTKDEYERHNSYTCEATHKTSTSPIVKSFNRNEC
>SEQ ID NO: 11: SNK0002_HCDR1
   TFGMG
>SEQ ID NO: 12: SNK0002_HCDR2
   LAHIWWDDDKYYNPAL
>SEQ ID NO: 13: SNK0002_HCDR3
   YYCARIAG
>SEQ ID NO: 14: SNK0002_LCDR1
   SASSSVSYMHW
>SEQ ID NO: 15: SNK0002_LCDR2
   STSNLASG
>SEQ ID NO: 16: SNK0002_LCDR3
   QRSSYPYTF
>SEQ ID NO: 17: SNK0002_HV 
MGRLTSSFLLLIVPAYVLSQVTLKESGPGILQPSQTLSLTCSFSGFSLSTFGMGVGWIRQPSGKGL

EWLAHIWWDDDKYYNPALKSRLTISKDTSKNQVFLKIANVDTADTATYYCARIAGFDYWGQGTTLT

VSS
>SEQ ID NO: 18: SNK0002_LV 
MHFQVQIFSFLLISASVIMSRGQIVLTQSPAIMSASPGEKVTITCSASSSVSYMHWFQQKPGTSPK
LWIYSTSNLASGVPARFSGSGSGTSYSLTISRMEAEDAATYYCQQRSSYPYTFGGGTKLEIK
 (SNK0002AR antibody heavy chain amino acid sequence)
>SEQ ID NO: 19: SNK0002AR_H 
MGRLTSSFLLLIVPAYVLSQVTLKESGPGILQPSQTLSLTCSFSGFSLSTFGMGVGWIRQPSGKGL

EWLAHIWWDDDKYYNPALKSRLTISKDTSKNQVFLKIANVDTADTATYYCARIAGFDYWGQGTTLT

VSSESARNPTIYPLTLPRALSSDPVIIGCLIHDYFPSGTMNVTWGKSGKDITTVNFPPALASGGGY

TMSSQLTLPAVECPEGESVKCSVQHDSNAVQELDVKCSGPPPPCPPCPPSCHPSLSLQRPALEDLL

LGSDASLTCTLNGLRNPEGAVFTWEPSTGKDAVQKKAVQNSCGCYSVSSVLPGCAERWNSGASFKC

TVTHPESDTLTGTIAKITVNTFPPQVHLLPPPSEELALNELVSLTCLVRAFNPKEVLVRWLHGNEE

LSPESYLVFEPLKEPGEGATTYLVTSVLRVSAELWKQGDQYSCMVGHEALPMNFTQKTIDRLSGKP

TNVSVSVIMSEGDGICY
 (SNK0002AR antibody light chain amino acid sequence)
>SEQ ID NO: 20: SNK0002AR_L 
MHFQVQIFSFLLISASVIMSRGQIVLTQSPAIMSASPGEKVTITCSASSSVSYMHWFQQKPGTSPK

LWIYSTSNLASGVPARFSGSGSGTSYSLTISRMEAEDAATYYCQQRSSYPYTFGGGTKLEIKRADA

APTVSIFPPSSEQLTSGGASVVCFLNNFYPKDINVKWKIDGSERQNGVLNSWTDQDSKDSTYSMSS

TLTLTKDEYERHNSYTCEATHKTSTSPIVKSFNRNEC
>SEQ ID NO: 21: SNK0003_HCDR1
   SYNIH
>SEQ ID NO: 22: SNK0003_HCDR2
   AIYSGNGATSHNQKFK
>SEQ ID NO: 23: SNK0003_HCDR3
   FCTRVGLR
>SEQ ID NO: 24: SNK0003_LCDR1
   KASQSVGTSVA
>SEQ ID NO: 25: SNK0003_LCDR2
   SASYRYS
>SEQ ID NO: 26: SNK0003_LCDR3
   QQYNNYPYT
>SEQ ID NO: 27: SNK0003_HV 
MGFSRIFLFLLSVTTGVHSQAYLQQSGAELVRPGASVRMSCKASDYTFTSYNIHWVKQTPRQGLEW

IGAIYSGNGATSHNQKFKGRATLTVDKSSSTAYMQLSSLTSEDSAVYFCTRVGLRSPFDFWGQGTT

LTVSS
>SEQ ID NO: 28: SNK0003_LV 
MESQTQVFVYMLLWLSGVDGDIVMTQSLKFMSTSGGDRVSVTCKASQSVGTSVAWYQQKPGQSPKP

LIYSASYRYSGVPDRFTGSGSGTDFSLTISNVQSEDLAEYFCQQYNNYPYTFGGGTKLEIK
 (SNK0003A antibody heavy chain amino acid sequence)
>SEQ ID NO: 29:SNK0003A_H 
MGFSRIFLFLLSVTTGVHSQAYLQQSGAELVRPGASVRMSCKASDYTFTSYNIHWVKQTPRQGLEW
IGAIYSGNGATSHNQKFKGRATLTVDKSSSTAYMQLSSLTSEDSAVYFCTRVGLRSPFDFWGQGTT
LTVSSESARNPTIYPLTLPRALSSDPVIIGCLIHDYFPSGTMNVTWGKSGKDITTVNFPPALASGG
GYTMSSQLTLPAVECPEGESVKCSVQHDSNAVQELDVKCSGPPPPCPPCPPSCHPSLSLQRPALED
LLLGSDASLTCTLNGLRNPEGAVFTWEPSTGKDAVQKKAVQNSCGCYSVSSVLPGCAERWNSGASF
KCTVTHPESDTLTGTIAKITVNTFPPQVHLLPPPSEELALNELVSLTCLVRAFNPKEVLVRWLHGN
EELSPESYLVFEPLKEPGEGATTYLVTSVLRVSAELWKQGDQYSCMVGHEALPMNFTQKTIDRLSG
KPTNVSVSVIMSEGDGICY
 (SNK0003A antibody light chain amino acid sequence)
>SEQ ID NO: 30: SNK0003A_L 
MESQTQVFVYMLLWLSGVDGDIVMTQSLKFMSTSGGDRVSVTCKASQSVGTSVAWYQQKPGQSPKP

LIYSASYRYSGVPDRFTGSGSGTDFSLTISNVQSEDLAEYFCQQYNNYPYTFGGGTKLEIKRADAA

PTVSIFPPSSEQLTSGGASVVCFLNNFYPKDINVKWKIDGSERQNGVLNSWTDQDSKDSTYSMSST

LTLTKDEYERHNSYTCEATHKTSTSPIVKSFNRNEC
>SEQ ID NO: 31: W27G2_HCDR1
   DYYIH
>SEQ ID NO: 32: W27G2_HCDR2
   RIDPENDETTYAPKFQ
>SEQ ID NO: 33: W27G2_HCDR3
   YCARSTVL
>SEQ ID NO: 34: W27G2_LCDR1
   RASQSIVHTNG
>SEQ ID NO: 35: W27G2_LCDR2
   KLLIYKV
>SEQ ID NO: 36: W27G2_LCDR3
   GVYYCFQGS
>SEQ ID NO: 37:W27G2_HV 
MKCSWIIFFLMAVVTGVNSEVQLQQSGSELVKSGASVKLSCTVSGFNFTDYYIHWVRQRTEQGLEW

IGRIDPENDETTYAPKFQGKATMTADTSSNTAYLQLTSLTSEDTAVYYCARSTVLDYWGHGTTLTV

SS
>SEQ ID NO: 38:W27G2_LV 
MKLPVRLLVLMFWIPGFSSDVLMTQTPLSLPVSLGDQASISCRASQSIVHTNGNTYLEWYLQKPGQ

SPKLLIYKVSNRFSGVPDRFSGSGSGTDFILKISRVEAEDLGVYYCFQGSHVPPTFGGGTKLEVK
   (Mouse W27G2 hybridoma-producing antibody heavy chain amino >SEQ ID NO: 39:W27G2_H 
MKCSWIIFFLMAVVTGVNSEVQLQQSGSELVKSGASVKLSCTVSGFNFTDYYIHWVRQRTEQGLEW
IGRIDPENDETTYAPKFQGKATMTADTSSNTAYLQLTSLTSEDTAVYYCARSTVLDYWGHGTTLTV
SSESARNPTIYPLTLPRALSSDPVIIGCLIHDYFPSGTMNVTWGKSGKDITTVNFPPALASGGGYT
MSSQLTLPAVECPEGESVKCSVQHDSNAVQELDVKCSGPPPPCPPCPPSCHPSLSLQRPALEDLLL
GSDASLTCTLNGLRNPEGAVFTWEPSTGKDAVQKKAVQNSCGCYSVSSVLPGCAERWNSGASFKCT
VTHPESDTLTGTIAKITVNTFPPQVHLLPPPSEELALNELVSLTCLVRAFNPKEVLVRWLHGNEEL
SPESYLVFEPLKEPGEGATTYLVTSVLRVSAELWKQGDQYSCMVGHEALPMNFTQKTIDRLSGKPT
NVSVSVIMSEGDGICY
   (Mouse W27G2 hybridoma-producing antibody light chain amino >SEQ ID NO: 40:W27G2_L 
MKLPVRLLVLMFWIPGFSSDVLMTQTPLSLPVSLGDQASISCRASQSIVHTNGNTYLEWYLQKPGQ

SPKLLIYKVSNRFSGVPDRFSGSGSGTDFILKISRVEAEDLGVYYCFQGSHVPPTFGGGTKLEVKR
 
ADAAPTVSIFPPSSEQLTSGGASVVCFLNNFYPKDINVKWKIDGSERQNGVLNSWTDQDSKDSTYS

MSSTLTLTKDEYERHNSYTCEATHKTSTSPIVKSFNRNEC
>SEQ ID NO: 41:RS_HCDR1_D50N
   NYYIH
>SEQ ID NO: 42:RS_HCDR2_D75N
   RIDPENNETTYAPKFQ
>SEQ ID NO: 43:RS_HCDR2_E76Q
   RIDPENDQTTYAPKFQ
>SEQ ID NO: 44:RS_HCDR2_D75N_E76Q
   RIDPENNQTTYAPKFQ
>SEQ ID NO: 45:RS_HV001 
MKCSWIIFFLMAVVTGVNSEVQLQQSGSELVKSGASVKLSCTVSGFNFTNYYIHWVRQRTEQGLEW

IGRIDPENDETTYAPKFQGKATMTADTSSNTAYLQLTSLTSEDTAVYYCARSTVLDYWGHGTTLTV

SS
>SEQ ID NO: 46:RS_HV002 
MKCSWIIFFLMAVVTGVNSEVQLQQSGSELVKSGASVKLSCTVSGFNFTDYYIHWVRQRTEQGLEW

IGRIDPENNETTYAPKFQGKATMTADTSSNTAYLQLTSLTSEDTAVYYCARSTVLDYWGHGTTLTV

SS
>SEQ ID NO: 47:RS_HV003 
MKCSWIIFFLMAVVTGVNSEVQLQQSGSELVKSGASVKLSCTVSGFNFTDYYIHWVRQRTEQGLEW

IGRIDPENDQTTYAPKFQGKATMTADTSSNTAYLQLTSLTSEDTAVYYCARSTVLDYWGHGTTLTV

SS
>SEQ ID NO: 48:RS_HV004 
MKCSWIIFFLMAVVTGVNSEVQLQQSGSELVKSGASVKLSCTVSGFNFTNYYIHWVRQRTEQGLEW
IGRIDPENNETTYAPKFQGKATMTADTSSNTAYLQLTSLTSEDTAVYYCARSTVLDYWGHGTTLTV
SS
>SEQ ID NO: 49:RS_HV005 
MKCSWIIFFLMAVVTGVNSEVQLQQSGSELVKSGASVKLSCTVSGFNFTNYYIHWVRQRTEQGLEW

IGRIDPENDQTTYAPKFQGKATMTADTSSNTAYLQLTSLTSEDTAVYYCARSTVLDYWGHGTTLTV

SS
>SEQ ID NO: 50:RS_HV006 
MKCSWIIFFLMAVVTGVNSEVQLQQSGSELVKSGASVKLSCTVSGFNFTDYYIHWVRQRTEQGLEW

IGRIDPENNQTTYAPKFQGKATMTADTSSNTAYLQLTSLTSEDTAVYYCARSTVLDYWGHGTTLTV

SS
>SEQ ID NO: 51:RS_HV007 
MKCSWIIFFLMAVVTGVNSEVQLQQSGSELVKSGASVKLSCTVSGFNFTNYYIHWVRQRTEQGLEW
IGRIDPENNQTTYAPKFQGKATMTADTSSNTAYLQLTSLTSEDTAVYYCARSTVLDYWGHGTTLTV
SS
>SEQ ID NO: 52: RS_LCDR1_A44S
   RSSQSIVHTNG
>SEQ ID NO: 53: RS_LFR1_L001
   SPASLSVSLGDRA
>SEQ ID NO: 54: RS_LFR1_L002
   SPASLSVSLGDRV
>SEQ ID NO: 55: RS_LFR1_L003
   SPASMSVSLGDRA
>SEQ ID NO: 56: RS_LFR1_L000
   TPLSLPVSLGDQA
>SEQ ID NO: 57:RS_LV001 
MKLPVRLLVLMFWIPGFSSDVLMTQSPASLSVSLGDRATISCRASQSIVHTNGNTYLEWYLQKPGQ

SPKLLIYKVSNRFSGVPDRFSGSGSGTDFILKISRVEAEDLGVYYCFQGSHVPPTFGGGTKLEVK
>SEQ ID NO: 58:RS_LV002 
MKLPVRLLVLMFWIPGFSSDVLMTQSPASLSVSLGDRVTISCRASQSIVHTNGNTYLEWYLQKPGQ
SPKLLIYKVSNRFSGVPDRFSGSGSGTDFILKISRVEAEDLGVYYCFQGSHVPPTFGGGTKLEVK
>SEQ ID NO: 59:RS_LV003 
MKLPVRLLVLMFWIPGFSSDVLMTQSPASLSVSLGDRVTISCRSSQSIVHTNGNTYLEWYLQKPGQ

SPKLLIYKVSNRFSGVPDRFSGSGSGTDFILKISRVEAEDLGVYYCFQGSHVPPTFGGGTKLEVK
>SEQ ID NO: 60:RS_LV004 
MKLPVRLLVLMFWIPGFSSDVLMTQSPASMSVSLGDRATISCRSSQSIVHTNGNTYLEWYLQKPGQ

SPKLLIYKVSNRFSGVPDRFSGSGSGTDFILKISRVEAEDLGVYYCFQGSHVPPTFGGGTKLEVK
>SEQ ID NO: 61:RS_LV005 
MKLPVRLLVLMFWIPGFSSDVLMTQSPASLSVSLGDRATISCRSSQSIVHTNGNTYLEWYLQKPGQ

SPKLLIYKVSNRFSGVPDRFSGSGSGTDFILKISRVEAEDLGVYYCFQGSHVPPTFGGGTKLEVK
>SEQ ID NO: 62: RS_HCDR1_X
   XYYIH
>SEQ ID NO: 63: RS_HCDR2_X
   RIDPENXXTTYAPKFQ
>SEQ ID NO: 64: RS_LCDR1_X
   RXSQSIVHTNG
   (SNK0001AR antibody heavy chain cDNA sequence)
>SEQ ID NO: 65: SNK0001AR_H_CDNA 
ATGGAATGGATCTGGATCTTTCTCTTCATCCTGTCAGGAACTGCAGGTGTCCAATCCCAGGTTCAG
CTGCAGCAGTCTGGAGCTGAGCTGGCGAGGCCTGGGGCTTCAGTGAAGCTGTCCTGCAAGGCTTCT
GGCTACACCTTCACAAGCTATGGTATAAGCTGGGTGAAGCAGAGAACTGGACAGGGCCTTGAGTGG
ATTGGAGAGATTTATCCTAGAAGTGGTAATACTTACTACAATGAGAAGTTCAAGGGCAAGGCCACA
CTGACTGCAGACAAATCCTCCAGCACAGCGTACATGGAGCTCCGCAGCCTGACATCTGAGGACTCT
GCGGTCTATTTCTGTGCAAGATTGGCATCGAGCTACTACGGTAGTAGCTACGACTGGTACTTCGAT
GTCTGGGGCACAGGGACCACGGTCACCGTCTCCTCAGAGTCTGCGAGAAATCCCACCATCTACCCA
CTGACACTCCCACGAGCTCTGTCAAGTGACCCAGTGATAATCGGCTGCCTGATTCACGATTACTTC
CCTTCCGGCACGATGAATGTGACCTGGGGAAAGAGTGGGAAGGATATAACCACCGTAAACTTCCCA
CCTGCCCTGGCCTCTGGGGGAGGGTACACCATGAGCAGCCAGTTGACCCTGCCAGCTGTCGAGTGC
CCAGAAGGAGAATCCGTGAAATGTTCCGTGCAACATGACTCTAACGCCGTCCAAGAATTGGATGTG
AAGTGCTCTGGTCCTCCTCCTCCTTGTCCTCCTTGTCCTCCTTCCTGCCATCCCAGCCTGTCACTG
CAGCGGCCAGCTCTTGAGGACCTGCTCCTGGGTTCAGATGCCAGCCTCACATGTACTCTGAATGGC
CTGAGAAATCCTGAGGGAGCTGTCTTCACCTGGGAGCCCTCCACTGGGAAGGATGCAGTGCAGAAG
AAAGCTGTGCAGAATTCCTGCGGCTGCTACAGTGTGTCCAGCGTCCTGCCTGGCTGTGCTGAGCGC
TGGAACAGTGGCGCATCATTCAAGTGCACAGTTACCCATCCTGAGTCTGACACCTTAACTGGCACA
ATTGCCAAAATCACAGTGAACACCTTCCCACCCCAGGTCCACCTGCTACCGCCGCCGTCGGAGGAG
CTGGCCCTGAATGAGCTCGTGTCCCTGACATGCCTGGTGCGAGCTTTCAACCCTAAAGAAGTGCTG
GTGCGATGGCTGCATGGAAATGAGGAGCTGTCCCCAGAAAGCTACCTAGTGTTTGAGCCCCTAAAG
GAGCCAGGCGAGGGAGCCACCACCTACCTGGTGACAAGCGTGTTGCGTGTATCAGCTGAACTCTGG
AAACAGGGTGACCAGTACTCCTGCATGGTGGGCCACGAGGCCTTGCCCATGAACTTCACCCAGAAG
ACCATCGACCGTCTGTCGGGTAAACCCACCAATGTCAGCGTGTCTGTGATCATGTCAGAGGGAGAT
GGCATCTGCTACTGA
 (SNK0001AR antibody light chain cDNA sequence)
>SEQ ID NO: 66:SNK0001AR_L_CDNA 
ATGAGGACCCCTGCTCAGTTTCTTGGAATCTTGTTGCTCTGGTTTCCAGGTATCAAATGTGACATC

AAGATGACCCAGTCTCCATCTTCCATGTATGCATCTCTAGGAGAGAGAGTCACTATCACTTGCAAG

GCGAGTCAGGACATTAATAGCTATTTAAGCTGGTTCCAGCAGAAACCAGGGAAATCTCCTAAGACC

CTGATCTATCGTGCAAACAGATTGGTAGATGGGGTCCCATCAAGGTTCAGTGGCAGTGGATCTGGG

CAAGATTATTCTCTCACCATCAGCAGCCTGGAGTATGAAGATATGGGAATTTATTATTGTCTACAG
 
TATGATGAGTTTCCGCTCACGTTCGGTGCTGGGACCAAGCTGGAGCTGAAACGGGCTGATGCTGCA

CCAACTGTATCCATCTTCCCACCATCCAGTGAGCAGTTAACATCTGGAGGTGCCTCAGTCGTGTGC

TTCTTGAACAACTTCTACCCCAAAGACATCAATGTCAAGTGGAAGATTGATGGCAGTGAACGACAA

AATGGCGTCCTGAACAGTTGGACTGATCAGGACAGCAAAGACAGCACCTACAGCATGAGCAGCACC

CTCACGTTGACCAAGGACGAGTATGAACGACATAACAGCTATACCTGTGAGGCCACTCACAAGACA

TCAACTTCACCCATTGTCAAGAGCTTCAACAGGAATGAGTGTTGA
 (SNK0002AR antibody heavy chain cDNA sequence)
>SEQ ID NO: 67: SNK0002AR_H_CDNA 
ATGGGCAGGCTTACTTCTTCATTCCTGTTACTGATTGTCCCTGCATATGTCCTGTCCCAGGTTACT
CTGAAAGAGTCTGGCCCTGGGATATTGCAGCCCTCCCAGACCCTCAGTCTGACTTGTTCTTTCTCT
GGGTTTTCACTGAGCACTTTTGGTATGGGTGTAGGCTGGATTCGTCAGCCTTCAGGGAAGGGTCTG
GAGTGGCTGGCACACATTTGGTGGGATGATGATAAGTACTATAACCCAGCCCTGAAGAGTCGGCTC
ACAATCTCCAAGGATACCTCCAAAAACCAGGTATTCCTCAAGATCGCCAATGTGGACACTGCAGAT
ACTGCCACATACTACTGTGCTCGAATAGCTGGGTTTGACTACTGGGGCCAAGGCACCACTCTCACA
GTCTCCTCAGAGTCTGCGAGAAATCCCACCATCTACCCACTGACACTCCCACGAGCTCTGTCAAGT
GACCCAGTGATAATCGGCTGCCTGATTCACGATTACTTCCCTTCCGGCACGATGAATGTGACCTGG
GGAAAGAGTGGGAAGGATATAACCACCGTAAACTTCCCACCTGCCCTGGCCTCTGGGGGAGGGTAC
ACCATGAGCAGCCAGTTGACCCTGCCAGCTGTCGAGTGCCCAGAAGGAGAATCCGTGAAATGTTCC
GTGCAACATGACTCTAACGCCGTCCAAGAATTGGATGTGAAGTGCTCTGGTCCTCCTCCTCCTTGT
CCTCCTTGTCCTCCTTCCTGCCATCCCAGCCTGTCACTGCAGCGGCCAGCTCTTGAGGACCTGCTC
CTGGGTTCAGATGCCAGCCTCACATGTACTCTGAATGGCCTGAGAAATCCTGAGGGAGCTGTCTTC
ACCTGGGAGCCCTCCACTGGGAAGGATGCAGTGCAGAAGAAAGCTGTGCAGAATTCCTGCGGCTGC
TACAGTGTGTCCAGCGTCCTGCCTGGCTGTGCTGAGCGCTGGAACAGTGGCGCATCATTCAAGTGC
ACAGTTACCCATCCTGAGTCTGACACCTTAACTGGCACAATTGCCAAAATCACAGTGAACACCTTC
CCACCCCAGGTCCACCTGCTACCGCCGCCGTCGGAGGAGCTGGCCCTGAATGAGCTCGTGTCCCTG
ACATGCCTGGTGCGAGCTTTCAACCCTAAAGAAGTGCTGGTGCGATGGCTGCATGGAAATGAGGAG
CTGTCCCCAGAAAGCTACCTAGTGTTTGAGCCCCTAAAGGAGCCAGGCGAGGGAGCCACCACCTAC
CTGGTGACAAGCGTGTTGCGTGTATCAGCTGAACTCTGGAAACAGGGTGACCAGTACTCCTGCATG
GTGGGCCACGAGGCCTTGCCCATGAACTTCACCCAGAAGACCATCGACCGTCTGTCGGGTAAACCC
ACCAATGTCAGCGTGTCTGTGATCATGTCAGAGGGAGATGGCATCTGCTACTGA
 (SNK0002AR antibody light chain cDNA sequence)
>SEQ ID NO: 68: SNK0002AR_L_CDNA 
ATGCATTTTCAAGTGCAGATTTTCAGCTTCCTGCTAATCAGTGCCTCAGTCATAATGTCCAGAGGA
CAAATTGTTCTCACCCAGTCTCCAGCAATCATGTCTGCATCTCCAGGGGAGAAGGTCACCATAACC
TGCAGTGCCAGCTCAAGTGTAAGTTACATGCACTGGTTCCAGCAGAAGCCAGGCACTTCTCCCAAA
CTCTGGATTTATAGCACATCCAACCTGGCTTCTGGAGTCCCTGCTCGCTTCAGTGGCAGTGGATCT
GGGACCTCTTACTCTCTCACAATCAGCCGAATGGAGGCTGAAGATGCTGCCACTTATTACTGCCAG
CAAAGGAGTAGTTACCCGTACACGTTCGGAGGGGGGACCAAGCTGGAAATAAAACGGGCTGATGCT
GCACCAACTGTATCCATCTTCCCACCATCCAGTGAGCAGTTAACATCTGGAGGTGCCTCAGTCGTG
TGCTTCTTGAACAACTTCTACCCCAAAGACATCAATGTCAAGTGGAAGATTGATGGCAGTGAACGA
CAAAATGGCGTCCTGAACAGTTGGACTGATCAGGACAGCAAAGACAGCACCTACAGCATGAGCAGC
ACCCTCACGTTGACCAAGGACGAGTATGAACGACATAACAGCTATACCTGTGAGGCCACTCACAAG
ACATCAACTTCACCCATTGTCAAGAGCTTCAACAGGAATGAGTGTTGA
 (SNK0003A antibody heavy chain cDNA sequence)
>SEQ ID NO: 69: SNK0003A_H_CDNA 
ATGGGATTCAGCAGGATCTTTCTCTTCCTCCTGTCAGTAACTACAGGTGTCCACTCCCAGGCTTAT

CTACAGCAGTCTGGGGCTGAGCTGGTGAGGCCTGGGGCCTCAGTGAGGATGTCCTGCAAGGCTTCT

GACTACACATTTACCAGTTACAATATCCACTGGGTAAAGCAGACACCTAGACAGGGCCTGGAATGG

ATTGGAGCTATTTATTCAGGAAATGGTGCTACTTCCCACAATCAGAAGTTCAAGGGCAGGGCCACA

CTGACTGTAGACAAATCCTCCAGCACAGCCTACATGCAACTCAGCAGCCTGACATCTGAAGACTCT

GCGGTCTATTTCTGTACAAGAGTGGGACTCCGGTCCCCTTTTGACTTCTGGGGCCAAGGCACCACT

CTCACAGTCTCCTCAGAGTCTGCGAGAAATCCCACCATCTACCCACTGACACTCCCACGAGCTCTG

TCAAGTGACCCAGTGATAATCGGCTGCCTGATTCACGATTACTTCCCTTCCGGCACGATGAATGTG

ACCTGGGGAAAGAGTGGGAAGGATATAACCACCGTAAACTTCCCACCTGCCCTGGCCTCTGGGGGA

GGGTACACCATGAGCAGCCAGTTGACCCTGCCAGCTGTCGAGTGCCCAGAAGGAGAATCCGTGAAA

TGTTCCGTGCAACATGACTCTAACGCCGTCCAAGAATTGGATGTGAAGTGCTCTGGTCCTCCTCCT

CCTTGTCCTCCTTGTCCTCCTTCCTGCCATCCCAGCCTGTCACTGCAGCGGCCAGCTCTTGAGGAC

CTGCTCCTGGGTTCAGATGCCAGCCTCACATGTACTCTGAATGGCCTGAGAAATCCTGAGGGAGCT

GTCTTCACCTGGGAGCCCTCCACTGGGAAGGATGCAGTGCAGAAGAAAGCTGTGCAGAATTCCTGC
 
GGCTGCTACAGTGTGTCCAGCGTCCTGCCTGGCTGTGCTGAGCGCTGGAACAGTGGCGCATCATTC
AAGTGCACAGTTACCCATCCTGAGTCTGACACCTTAACTGGCACAATTGCCAAAATCACAGTGAAC
ACCTTCCCACCCCAGGTCCACCTGCTACCGCCGCCGTCGGAGGAGCTGGCCCTGAATGAGCTCGTG
TCCCTGACATGCCTGGTGCGAGCTTTCAACCCTAAAGAAGTGCTGGTGCGATGGCTGCATGGAAAT
GAGGAGCTGTCCCCAGAAAGCTACCTAGTGTTTGAGCCCCTAAAGGAGCCAGGCGAGGGAGCCACC
ACCTACCTGGTGACAAGCGTGTTGCGTGTATCAGCTGAACTCTGGAAACAGGGTGACCAGTACTCC
TGCATGGTGGGCCACGAGGCCTTGCCCATGAACTTCACCCAGAAGACCATCGACCGTCTGTCGGGT
AAACCCACCAATGTCAGCGTGTCTGTGATCATGTCAGAGGGAGATGGCATCTGCTACTGA
 (SNK0003A antibody light chain cDNA sequence)
>SEQ ID NO: 70: SNK0003A_L_CDNA 
ATGGAGTCACAGACTCAGGTCTTTGTATACATGTTGCTGTGGTTGTCTGGTGTTGATGGAGACATT

GTGATGACCCAGTCTCTAAAATTCATGTCCACATCGGGAGGAGACAGGGTCAGCGTCACCTGCAAG

GCCAGTCAGAGTGTGGGTACAAGTGTAGCCTGGTATCAGCAGAAACCAGGACAATCTCCTAAACCA

CTGATTTATTCGGCATCTTATCGGTACAGTGGAGTCCCTGATCGCTTCACAGGCAGTGGATCTGGG

ACAGATTTCAGTCTCACCATCAGCAATGTGCAGTCTGAAGACTTGGCAGAATATTTCTGTCAGCAA

TATAACAACTATCCGTACACGTTCGGAGGGGGGACCAAGCTGGAGATAAAACGGGCTGATGCTGCA

CCAACTGTATCCATCTTCCCACCATCCAGTGAGCAGTTAACATCTGGAGGTGCCTCAGTCGTGTGC

TTCTTGAACAACTTCTACCCCAAAGACATCAATGTCAAGTGGAAGATTGATGGCAGTGAACGACAA

AATGGCGTCCTGAACAGTTGGACTGATCAGGACAGCAAAGACAGCACCTACAGCATGAGCAGCACC

CTCACGTTGACCAAGGACGAGTATGAACGACATAACAGCTATACCTGTGAGGCCACTCACAAGACA

TCAACTTCACCCATTGTCAAGAGCTTCAACAGGAATGAGTGTTGA
 (Mouse W27G2 hybridoma-producing antibody heavy chain cDNA sequence)
>SEQ ID NO: 71:W27G_MOUSE_IGA_H_CDNA 
ATGAAATGCAGCTGGATCATCTTCTTCCTGATGGCAGTGGTTACAGGGGTCAATTCAGAGGTTCAG
CTGCAGCAGTCTGGGTCAGAGCTTGTGAAGTCTGGGGCCTCAGTCAAGTTGTCCTGCACAGTTTCT
GGGTTCAACTTTACAGACTACTATATACACTGGGTGAGGCAGAGGACTGAACAGGGCCTGGAATGG
ATTGGAAGGATTGATCCTGAGAATGATGAAACTACATATGCCCCGAAATTCCAGGGCAAGGCCACT
ATGACAGCAGACACATCTTCCAACACAGCCTACCTGCAGCTCACCAGCCTGACATCTGAAGACACT
GCCGTCTATTACTGTGCTAGATCTACGGTCCTTGACTACTGGGGCCACGGCACCACTCTCACAGTC
 
TCCTCAGAGTCTGCGAGAAATCCCACCATCTACCCACTGACACTCCCACGAGCTCTGTCAAGTGAC

CCAGTGATAATCGGCTGCCTGATTCACGATTACTTCCCTTCCGGCACGATGAATGTGACCTGGGGA

AAGAGTGGGAAGGATATAACCACCGTAAACTTCCCACCTGCCCTGGCCTCTGGGGGAGGGTACACC

ATGAGCAGCCAGTTGACCCTGCCAGCTGTCGAGTGCCCAGAAGGAGAATCCGTGAAATGTTCCGTG

CAACATGACTCTAACGCCGTCCAAGAATTGGATGTGAAGTGCTCTGGTCCTCCTCCTCCTTGTCCT

CCTTGTCCTCCTTCCTGCCATCCCAGCCTGTCACTGCAGCGGCCAGCTCTTGAGGACCTGCTCCTG

GGTTCAGATGCCAGCCTCACATGTACTCTGAATGGCCTGAGAAATCCTGAGGGAGCTGTCTTCACC

TGGGAGCCCTCCACTGGGAAGGATGCAGTGCAGAAGAAAGCTGTGCAGAATTCCTGCGGCTGCTAC

AGTGTGTCCAGCGTCCTGCCTGGCTGTGCTGAGCGCTGGAACAGTGGCGCATCATTCAAGTGCACA

GTTACCCATCCTGAGTCTGACACCTTAACTGGCACAATTGCCAAAATCACAGTGAACACCTTCCCA

CCCCAGGTCCACCTGCTACCGCCGCCGTCGGAGGAGCTGGCCCTGAATGAGCTCGTGTCCCTGACA

TGCCTGGTGCGAGCTTTCAACCCTAAAGAAGTGCTGGTGCGATGGCTGCATGGAAATGAGGAGCTG

TCCCCAGAAAGCTACCTAGTGTTTGAGCCCCTAAAGGAGCCAGGCGAGGGAGCCACCACCTACCTG

GTGACAAGCGTGTTGCGTGTATCAGCTGAACTCTGGAAACAGGGTGACCAGTACTCCTGCATGGTG

GGCCACGAGGCCTTGCCCATGAACTTCACCCAGAAGACCATCGACCGTCTGTCGGGTAAACCCACC

AATGTCAGCGTGTCTGTGATCATGTCAGAGGGAGATGGCATCTGCTACTGA
 (Mouse W27G2 hybridoma-producing antibody light chain cDNA sequence)
>SEQ ID NO: 72:W27G_MOUSE_IGA_L_CDNA 
ATGAAGTTGCCTGTTAGGCTGTTGGTGCTGATGTTCTGGATTCCTGGATTCAGCAGTGATGTTTTG

ATGACCCAAACTCCACTCTCCCTGCCTGTCAGTCTTGGAGATCAAGCCTCCATCTCTTGTAGAGCT

AGTCAGAGCATTGTACACACTAATGGGAACACCTATTTAGAATGGTACCTGCAGAAACCAGGCCAG

TCTCCAAAGCTCCTGATCTACAAAGTTTCCAACCGATTTTCTGGGGTCCCAGACAGGTTCAGTGGC

AGTGGATCTGGGACAGATTTCATACTCAAGATCAGCAGAGTGGAGGCTGAGGATCTGGGAGTTTAT

TACTGCTTTCAAGGTTCACATGTTCCTCCGACGTTCGGTGGAGGCACCAAGCTGGAAGTCAAACGG

GCTGATGCTGCACCAACTGTATCCATCTTCCCACCATCCAGTGAGCAGTTAACATCTGGAGGTGCC

TCAGTCGTGTGCTTCTTGAACAACTTCTACCCCAAAGACATCAATGTCAAGTGGAAGATTGATGGC

AGTGAACGACAAAATGGCGTCCTGAACAGTTGGACTGATCAGGACAGCAAAGACAGCACCTACAGC

ATGAGCAGCACCCTCACGTTGACCAAGGACGAGTATGAACGACATAACAGCTATACCTGTGAGGCC

ACTCACAAGACATCAACTTCACCCATTGTCAAGAGCTTCAACAGGAATGAGTGTTAG
 (Mouse W27G2 IGG antibody heavy chain cDNA sequence)
>SEQ ID NO: 73:W27G2_MOUSE_IGG_H_CDNA 
ATGAAATGCAGCTGGATCATCTTCTTCCTGATGGCAGTGGTTACAGGGGTCAATTCAGAGGTTCAG
CTGCAGCAGTCTGGGTCAGAGCTTGTGAAGTCTGGGGCCTCAGTCAAGTTGTCCTGCACAGTTTCT
GGGTTCAACTTTACAGACTACTATATACACTGGGTGAGGCAGAGGACTGAACAGGGCCTGGAATGG
ATTGGAAGGATTGATCCTGAGAATGATGAAACTACATATGCCCCGAAATTCCAGGGCAAGGCCACT
ATGACAGCAGACACATCTTCCAACACAGCCTACCTGCAGCTCACCAGCCTGACATCTGAAGACACT
GCCGTCTATTACTGTGCTAGATCTACGGTCCTTGACTACTGGGGCCACGGCACCACTCTCACAGTC
TCCTCAAAAACGACACCCCCATCTGTCTATCCACTGGCCCCTGGATCTGCTGCCCAAACTAACTCC
ATGGTGACCCTGGGATGCCTGGTCAAGGGCTATTTCCCTGAGCCAGTGACAGTGACCTGGAACTCT
GGATCCCTGTCCAGCGGTGTGCACACCTTCCCAGCTGTCCTGCAGTCTGACCTCTACACTCTGAGC
AGCTCAGTGACTGTCCCCTCCAGCACCTGGCCCAGCGAGACCGTCACCTGCAACGTTGCCCACCCG
GCCAGCAGCACCAAGGTGGACAAGAAAATTGTGCCCAGGGATTGTGGTTGTAAGCCTTGCATATGT
ACAGTCCCAGAAGTATCATCTGTCTTCATCTTCCCCCCAAAGCCCAAGGATGTGCTCACCATTACT
CTGACTCCTAAGGTCACGTGTGTTGTGGTAGACATCAGCAAGGATGATCCCGAGGTCCAGTTCAGC
TGGTTTGTAGATGATGTGGAGGTGCACACAGCTCAGACGCAACCCCGGGAGGAGCAGTTCAACAGC
ACTTTCCGCTCAGTCAGTGAACTTCCCATCATGCACCAGGACTGGCTCAATGGCAAGGAGTTCAAA
TGCAGGGTCAACAGTGCAGCTTTCCCTGCCCCCATCGAGAAAACCATCTCCAAAACCAAAGGCAGA
CCGAAGGCTCCACAGGTGTACACCATTCCACCTCCCAAGGAGCAGATGGCCAAGGATAAAGTCAGT
CTGACCTGCATGATAACAGACTTCTTCCCTGAAGACATTACTGTGGAGTGGCAGTGGAATGGGCAG
CCAGCGGAGAACTACAAGAACACTCAGCCCATCATGGACACAGATGGCTCTTACTTCGTCTACAGC
AAGCTCAATGTGCAGAAGAGCAACTGGGAGGCAGGAAATACTTTCACCTGCTCTGTGTTACATGAG
GGCCTGCACAACCACCATACTGAGAAGAGCCTCTCCCACTCTCCTGGTAAAtaa
 (Human chimeric W27G2 IgG antibody heavy chain cDNA sequence)
>SEQ ID NO: 74: W27G2_HUMAN_CHIMERA_IGG_H_CDNA 
ATGAAATGCAGCTGGATCATCTTCTTCCTGATGGCAGTGGTTACAGGGGTCAATTCAGAGGTTCAG

CTGCAGCAGTCTGGGTCAGAGCTTGTGAAGTCTGGGGCCTCAGTCAAGTTGTCCTGCACAGTTTCT

GGGTTCAACTTTACAGACTACTATATACACTGGGTGAGGCAGAGGACTGAACAGGGCCTGGAATGG

ATTGGAAGGATTGATCCTGAGAATGATGAAACTACATATGCCCCGAAATTCCAGGGCAAGGCCACT

ATGACAGCAGACACATCTTCCAACACAGCCTACCTGCAGCTCACCAGCCTGACATCTGAAGACACT
 
GCCGTCTATTACTGTGCTAGATCTACGGTCCTTGACTACTGGGGCCACGGCACCACTCTCACAGTC
TCCTCAGCATCCCCGACTAGTTCCACCAAGGGCCCATCGGTCTTCCCCCTGGCACCCTCCTCCAAG
AGCACCTCTGGGGGCACAGCGGCCCTGGGCTGCCTGGTCAAGGACTACTTCCCCGAACCGGTGACG
GTGTCGTGGAACTCAGGCGCCCTGACCAGCGGCGTGCACACCTTCCCGGCTGTCCTACAGTCCTCA
GGACTCTACTCCCTCAGCAGCGTGGTGACCGTGCCCTCCAGCAGCTTGGGCACCCAGACCTACATC
TGCAACGTGAATCACAAGCCCAGCAACACCAAGGTGGACAAGAAAGTTGAGCCCAAATCTTGTGAC
AAAACTCACACATGCCCACCGTGCCCAGCACCTGAACTCCTGGGGGGACCGTCAGTCTTCCTCTTC
CCCCCAAAACCCAAGGACACCCTCATGATCTCCCGGACCCCTGAGGTCACATGCGTGGTGGTGGAC
GTGAGCCACGAAGACCCTGAGGTCAAGTTCAACTGGTACGTGGACGGCGTGGAGGTGCATAATGCC
AAGACAAAGCCGCGGGAGGAGCAGTACAACAGCACGTACCGTGTGGTCAGCGTCCTCACCGTCCTG
CACCAGGACTGGCTGAATGGCAAGGAGTACAAGTGCAAGGTCTCCAACAAAGCCCTCCCAGCCCCT
ATCGAGAAAACCATCTCCAAAGCCAAAGGGCAGCCCCGAGAACCACAGGTGTACACCCTGCCCCCA
TCCCGGGATGAGCTGACCAAGAACCAGGTCAGCCTGACCTGCCTGGTCAAAGGCTTCTATCCCAGC
GACATCGCCGTGGAGTGGGAGAGCAATGGGCAGCCGGAGAACAACTACAAGACCACGCCTCCCGTG
CTGGACTCCGACGGCTCCTTCTTCCTCTACAGCAAGCTCACCGTGGACAAGAGCAGGTGGCAGCAG
GGGAACGTCTTCTCATGCTCCGTGATGCATGAGGGTCTGCACAACCACTACACGCAGAAGAGCCTC
TCCCTGTCTCCGGGTAAATGA
 (Human chimeric W27G2 IgG antibody light chain cDNA sequence)
>SEQ ID NO: 75: W27G2_HUMAN_CHIMERA_IGG_L_CDNA 
ATGGACATGAAGTTGCCTGTTAGGCTGTTGGTGCTGATGTTCTGGATTCCTGGATTCAGCAGTGAT

GTTTTGATGACCCAAACTCCACTCTCCCTGCCTGTCAGTCTTGGAGATCAAGCCTCCATCTCTTGT

AGAGCTAGTCAGAGCATTGTACACACTAATGGGAACACCTATTTAGAATGGTACCTGCAGAAACCA

GGCCAGTCTCCAAAGCTCCTGATCTACAAAGTTTCCAACCGATTTTCTGGGGTCCCAGACAGGTTC

AGTGGCAGTGGATCTGGGACAGATTTCATACTCAAGATCAGCAGAGTGGAGGCTGAGGATCTGGGA

GTTTATTACTGCTTTCAAGGTTCACATGTTCCTCCGACGTTCGGTGGAGGCACCAAGCTGGAAGTC

AAACGGGCTGATGCTGCACCAACTGTATCCATCTTCCCACCATCCAGTGAGCAGTTAACATCTGGA

GGTGCCTCTGTTGTGTGCCTGCTGAATAACTTCTATCCTCGAGAGGCCAAAGTACAGTGGAAGGTG

GATAACGCCCTCCAATCGGGTAACTCCCAGGAGAGTGTCACAGAGCAGGACAGCAAGGACAGCACC

TACAGCCTCAGCAGCACCCTGACGCTGAGCAAAGCAGACTACGAGAAACACAAAGTCTACGCCTGC

GAAGTCACCCATCAGGGCCTGAGCTCGCCCGTCACAAAGAGCTTCAACAGGGGAGAGTGTTAG
 (Mouse W27G2 IGG antibody heavy chain amino acid sequence)
>SEQ ID NO: 76: W27G2_MOUSE_IGG_H 
MKCSWIIFFLMAVVTGVNSEVQLQQSGSELVKSGASVKLSCTVSGFNFTDYYIHWVRQRTEQGLEW
IGRIDPENDETTYAPKFQGKATMTADTSSNTAYLQLTSLTSEDTAVYYCARSTVLDYWGHGTTLTV
SSKTTPPSVYPLAPGSAAQTNSMVTLGCLVKGYFPEPVTVTWNSGSLSSGVHTFPAVLQSDLYTLS
SSVTVPSSTWPSETVTCNVAHPASSTKVDKKIVPRDCGCKPCICTVPEVSSVFIFPPKPKDVLTIT
LTPKVTCVVVDISKDDPEVQFSWFVDDVEVHTAQTQPREEQFNSTFRSVSELPIMHQDWLNGKEFK
CRVNSAAFPAPIEKTISKTKGRPKAPQVYTIPPPKEQMAKDKVSLTCMITDFFPEDITVEWQWNGQ
PAENYKNTQPIMDTDGSYFVYSKLNVQKSNWEAGNTFTCSVLHEGLHNHHTEKSLSHSPGK
 (Human chimeric W27G2 IgG antibody heavy chain amino acid sequence)
>SEQ ID NO: 77:W27G2_HUMAN_CHIMERA_IGG_H 
MKCSWIIFFLMAVVTGVNSEVQLQQSGSELVKSGASVKLSCTVSGFNFTDYYIHWVRQRTEQGLEW
IGRIDPENDETTYAPKFQGKATMTADTSSNTAYLQLTSLTSEDTAVYYCARSTVLDYWGHGTTLTV
SSASPTSSTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPELLGGPSVFLF
PPKPKDTLMISRTPEVTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVMHEGLHNHYTQKSL
SLSPGK
 (Human chimeric W27G2 IgG antibody light chain amino acid sequence)
>SEQ ID NO: 78:W27G2_HUMAN_CHIMERA_IGG_L

MDMKLPVRLLVLMFWIPGFSSDVLMTQTPLSLPVSLGDQASISCRASQSIVHTNGNTYLEWYLQKP
GQSPKLLIYKVSNRFSGVPDRFSGSGSGTDFILKISRVEAEDLGVYYCFQGSHVPPTFGGGTKLEV
KRADAAPTVSIFPPSSEQLTSGGASVVCLLNNFYPREAKVQWKVDNALQSGNSQESVTEQDSKDST
YSLSSTLTLSKADYEKHKVYACEVTHQGLSSPVTKSFNRGEC

### (Method for Producing Antibody Binding to C. difficile Bacterial Body)

A method for producing an antibody binding to a C. difficile bacterial body according to the present invention includes the following steps 1 to 3.

### (Step 1)

Step of preparing antibody-producing immortalized cells from B cells collected from intestinal mucosa lamina propria or spleen.

### (Step 2)

Step of culturing the antibody-producing immortalized cells prepared in the step 1 and determining cells that produce an antibody binding to a C. difficile bacterial body.

### (Step 3)

Step of recovering the antibodies from the cells that produce an antibody binding to a C. difficile bacterial body.

The method for producing an antibody binding to a C. difficile bacterial body according to the present invention may include, after the step 3, for example, a step of treating a heavy chain variable region and/or a light chain variable region as described below with, for example, a protease or the like, in order to form a configuration in which these regions are appropriately combined as necessary, a step of introducing a functional group that enables a chemical bond such as a disulfide bond, and a step of subsequently forming the chemical bond via the functional group, and the like.

The above steps 1 to 3 in the method for producing an antibody binding to a C. difficile bacterial body according to the present invention are described in detail below.

### (Regarding Step 1)

The step 1 in the method for producing an antibody binding to a C. difficile bacterial body according to the present invention is a step of preparing antibody-producing immortalized cells from B cells collected from intestinal mucosa lamina propria or spleen.

The intestinal mucosa lamina propria is not particularly limited, and may be, for example, one of layers constituting a mucous membrane present in esophagus, stomach, small intestine (including duodenum, jejunum, ileum, and the like), large intestine (including cecum, colon, rectum, and the like), and the like, and may be a layer located between a layer containing epithelial cells and muscularis mucosae. Among them, the intestinal mucosa lamina propria present in the small intestine, which includes lymphoid tissue, capillaries, lymphatic vessels, and the like, is preferable.

The cells of the spleen are not particularly limited, but cells including B cells can be used.

A method for collecting B cells from intestinal mucosa lamina propria or spleen is not particularly limited, but for example, the intestinal tract is collected, and the obtained intestinal tract is washed and then incised to expose a mucosal layer. Next, epithelial cells are then shaken in saline comprising an appropriate concentration of EDTA to release and remove the epithelial cells. Thereafter, a method for recovering B cells by a treatment with a digestive enzyme such as collagenase at an appropriate concentration can be exemplified. Other known methods may be employed, and commercially available B cells derived from intestinal mucosa lamina propria or spleen may be purchased and obtained.

The origin from intestinal mucosa lamina propria or spleen, that is, the origin of the B cells is not particularly limited, and examples thereof include a human, a mouse, a rat, a hamster, a rabbit, a goat, a sheep, a donkey, a pig, a cow, a horse, a chicken, a monkey, a chimpanzee, a camel, and a llama.

In the step of preparing the antibody-producing immortalized cells from B cells collected from intestinal mucosa lamina propria or spleen, any method known to those skilled in the art can be used. For example, as the antibody-producing immortalized cells, a hybridoma produced by fusing the B cells and the type of cells other than the B cells can be prepared, and immortalized B cells can be prepared by infecting B cells with an EB virus or the like, but the present invention are not limited thereto.

In the case of preparing the hybridoma by fusing B cells and the type of cells other than the B cells, the type of cells other than the B cells (in the present specification, the term "other cells" may be used) is not particularly limited as long as the cells are fused by being in contact with the B cell to form a hybridoma, and the hybridoma does not lose the function of producing an antibody exhibited by the B cells described above.

As such other cells, cells which are fused with the B cells to form a hybridoma so that the hybridoma can acquire an immortalizing function are preferable, and specific examples thereof include cancer cells, and in particular, cells derived from bone marrow species such as myeloma. Other preferred cells are myeloma, for example, mouse NS1 cells.

The origin from the other cells is not particularly limited, and examples thereof include a human, a mouse, a rat, a hamster, a rabbit, a goat, a sheep, a donkey, a pig, a cow, a horse, a chicken, a monkey, a chimpanzee, a camel, and a llama.

The fusion means that the B cells and other cells are integrally and inseparably united. Here, integrally inseparable excludes cell division that is a phenomenon when the fused cells proliferate. In this way, fused cells are included as an example of a hybridoma in the present specification.

Conditions at the time of mixing and fusing are not particularly limited, and conditions usually used in a method for fusing cells may be appropriately adopted. For example, it may be performed by appropriately modifying conditions used when B cells or other cells are cultured. Examples of such a method include a method of mixing B cells and the type of cells other than the B cells in an appropriate medium to contact with each other in the presence of polyethylene glycol or the like; a method of applying electrical stimulation after the mixing; and a method including, after a method using viruses such as Sendai virus, incubating the viruses under conditions of 37°C and 5% carbon dioxide.

A time required for fusion is also not particularly limited, and a time until fusion itself is completed may be appropriately set. A method for confirming that fusion is completed may be performed by appropriately modifying a known method used when normal cell fusion is performed. Examples of such a method include a method of observing a degree of progress of fusion under a microscope, a known kit may be appropriately selected, and cells may be fused according to the use conditions.

### (Regarding Step 2)

The step 2 in the method for producing an antibody binding to a C. difficile bacterial body according to the present invention is a step of culturing the antibody-producing immortalized cells prepared in the step 1 and determining cells that produce an antibody binding to a C. difficile bacterial body.

Preferably, the step 2 is performed by determining cells that produce an antibody binding to the entire C. difficile bacterial body immobilized on a carrier. Preferably, the immobilized C. difficile bacterial body is not subjected to a lysis treatment. For example, the C. difficile bacterial body is immobilized on a carrier such as an ELISA plate in a state of being suspended in a Na₂CO₃ buffer.

Before the step 2 of the present invention, the antibody-producing immortalized cells obtained in the step 1 may be subjected to a subcloning step such as a limiting dilution method, which is usually used when producing a monoclonal antibody.

Antibody-producing immortalized cells that produce an antibody binding to a C. difficile bacterial body can be determined by means such as ELISA, EIA, RIA, ELISA, or FIA, or by means such as FACS.

In addition, after the determination of the antibody-producing immortalized cells binding to the C. difficile bacterial body determined by the method described above, the method may further include a step of determining antibody-producing immortalized cells that produce an antibody binding to another intestinal bacterium.

### (Regarding Step 3)

The step 3 in the method for producing an antibody binding to a C. difficile bacterial body according to the present invention is a step of recovering the antibodies from the cells that produce an antibody binding to a C. difficile bacterial body determined in the step 2.

A specific recovery method in the step 3 is not particularly limited, and examples thereof include a method of recovering a supernatant of a culture solution of cells that produce an IgA antibody and a method of recovering a lysate of the cells. Cells can be lysed with the lysate of the cells using an appropriate combination of known mechanical means such as ultrasonic disruption and French press and/or known chemical treatments using a surfactant, a cell wall-digesting enzyme, and a cell membrane-digesting enzyme, and thereafter, a liquid phase fraction after being subjected to a solid-liquid separation step can be recovered to produce an antibody binding to a C. difficile bacterial body of the present invention.

Before recovering the antibody molecule from the antibody-producing immortalized cells and after culturing the antibody-producing immortalized cells for a certain period using an appropriate medium, a method of recovering the supernatant or a method of recovering the supernatant from a lysate of the cells may be provided. In addition, the antibody-producing immortalized cells may be intraperitoneally administered to an animal individual such as an immunodeficient mouse, and the antibody may be recovered from ascites of such an individual.

Note that the antibody binding to a C. difficile bacterial body recovered by the method described above may be appropriately subjected to a known purification step. Specific purification means is not particularly limited, and for example, known protein purification means such as purification means by precipitation using acetone, ammonium sulfate, or the like, and purification means using column chromatography such as affinity, anion exchange, cation exchange, size exclusion, or reverse phase may be combined and employed.

### (Method for Producing Recombinant Antibody)

In another aspect, in the case of the antibody binding to a C. difficile bacterial body of the present invention, a method of introducing a nucleic acid encoding an antibody recognized to bind to a C. difficile bacterial body into cells capable of producing monoclonal antibodies, culturing the cells, recovering monoclonal antibodies from a cell extract or culture supernatant thereof, and as necessary, purifying the recovered monoclonal antibodies is exemplified.

In order to confirm that the antibody binding to a C. difficile bacterial body, means well known to those skilled in the art can be used, and for example, an antibody molecule or a cell producing the antibody can be confirmed by identifying using means by ELISA, EIA, RIA, ELISA, FIA, or the like, means by FACS, or the like.

The cell capable of producing a monoclonal antibody is usually not particularly limited as long as it is a cell capable of producing various proteins that exhibit functions by forming a higher order structure derived from mammals and the like, and for example, known cells such as cells derived from mammals such as COS cells, HEK cells (HEK293, HEK 293T, and the like), HELA cells, and CHO cells, and cells derived from insects such as Sf9 may be appropriately selected. In one aspect, the monoclonal antibody can also be produced using plant cells such as yeast cells, filamentous fungi cells, soybean, and Arabidopsis thaliana.

Specific methods for introducing a nucleic acid, culture conditions for cells into which the nucleic acid is introduced, recovery methods, and purification methods are not particularly limited depending on the type of cells to be used, and known methods are appropriately modified and combined, such that an antibody binding to a C. difficile bacterial body according to the present invention can be produced.

In one aspect, a monoclonal antibody can also be produced as an antibody-comprising composition suitable for enteral administration by producing a monoclonal antibody in these cells, and then drying or lyophilizing the monoclonal antibody secreted into a culture solution from these cells together with the culture solution, or disrupting, drying, or lyophilizing the monoclonal antibodies accumulated in these cells together with the cells (Virdi et al., Nat. Biotechnol., 2019, Vol. 37, pp. 527-530). In one aspect, the cell that produces the antibody-comprising composition suitable for enteral administration is preferably a plant cell such as a yeast cell, a filamentous fungus cell, soybean, or Arabidopsis thaliana, and the monoclonal antibody to be produced is preferably in a form in which a VHH fragment is fused to IgAFc.

In a case where the antibody binding to a C. difficile bacterial body according to the present invention is a chimeric antibody, regarding the nucleic acid described above, nucleic acids comprising a base sequence encoding a heavy chain variable region and a light chain variable region and a base sequence encoding a heavy chain constant region and a light chain constant region derived from a different species may be prepared, the prepared nucleic acid comprising a base sequence encoding a heavy chain variable region and the prepared nucleic acid comprising a base sequence encoding a heavy chain constant region may bind to each other, or the prepared base sequence encoding a light chain variable region and the prepared nucleic acid comprising a base sequence encoding a light chain constant region may bind to each other, and these binding nucleic acids may be introduced into the cells that produce an antibody as described above.

Furthermore, also in the production method in a case where the antibody binding to a C. difficile bacterial body according to the present invention is an antibody comprising an amino acid sequence in which heavy chains and/or light chains CDR1 to 3 are derived from a mouse, and comprising an amino acid sequence in which other regions are derived from a human (this may be called a humanized antibody), similarly to the chimeric antibody described above, nucleic acids comprising a base sequence encoding heavy chains and/or light chains CDR1 to 3 and a base sequence encoding other regions may be prepared, the nucleic acids may rebind to each other to form a heavy chain and a light chain, and the binding nucleic acids may be introduced into the cells that may produce a monoclonal IgA antibody as described above. In order to maintain the binding ability of the antibody, it may be required to replace some bases with other bases.

### Examples

Hereinafter, the present invention will be described in more detail with reference to Example, but these Examples are merely illustrative and do not limit the present disclosure.

### (Methods and Materials)

### 1. Mouse

8- to 25-week-old C57BL/6 or BALB/c mice were used. These mice were raised in SPF, and the raised mice were transplanted with human intestinal bacterial flora or infected with specific pathogenic bacteria.

### u

### (1-1) Separation of Small Intestinal and Large Intestinal Mucous Lamina Propria Cells

The mice were euthanized and then subjected to laparotomy to remove the entire small intestine. After the connective tissue and Peyer's patches were removed from the removed small intestine, the small intestine was longitudinally incised, and contents of the small intestine were washed with PBS. Next, the washed small intestine was cut into a length of about 1 cm and added to a 100 ml beaker filled with 50 ml of PBS containing 1 mM EDTA. After shaking at 37°C for 20 minutes, small intestine fragments were collected in a strainer and PBS containing EDTA was discarded. The small intestine fragments were added to a 50 ml tube, 20 ml of PBS was added thereto, the mixture was vigorously shaken for 10 seconds, the small intestine fragments were collected again in a strainer, and the PBS was discarded. This operation was repeated twice, and only small intestine epithelial cells were removed from the small intestine tissue. Next, 50 ml of a digestive enzyme solution (adjusted with 100 ml RPMI 1640, 5 ml of FCS (final concentration 5%), 50 µl of 2-mercaptoethanol (final concentration 55 µM), 0.15 g of collagenase, and 1 ml of dispase (50 U/ml) (final concentration 0.5 U/ml)) warmed to 37°C was added to a 100 ml beaker, and the small intestine tissue was further finely cut and shaken at 37°C for 30 minutes. Thereafter, the 100 ml beaker was allowed to stand, and the small intestine tissue was immersed, and then the supernatant was transferred to a new 50 ml tube. The digestion step using the digestive enzyme solution was repeated once for 20 minutes. The digestive fluid was centrifuged at 1,500 rpm and room temperature for 5 minutes and the supernatant was discarded. Mucosal lamina propria cells obtained as precipitates were washed once with RPMI 1640 containing 2% FCS, suspended in 2 ml of RPMI 1640 containing 2% FCS, and then stored on ice. The same operation was performed on the supernatant subjected to the reaction in the second digestion step, the first cell suspension and the second cell suspension were combined, and then the combined suspension was passed through a filter to remove tissue pieces, thereby obtaining small intestine (large intestine) mucosa lamina propria cells.

### (1-2) Preparation and Cloning of Antibody-Producing Hybridomas

Hybridomas were prepared by fusing NS1 cells, which are mouse myeloma cells, with small intestine (large intestine) mucosal lamina propria cells or spleen cells. Cell fusion was performed according to ClonaCell-HY Hybridoma Cloning Kit (STEMCELL Technologies) according to the reagent and procedure of the kit. The resulting hybridomas were grown in a methylcellulose-containing medium according to the ClonaCell-HY Hybridoma Cloning Kit (STEMCELL Technologies), clones were picked up with the naked eye, and each clone was further grown in a 96-well plate. Thereafter, a frozen cell stock was prepared from the cloned hybridomas, and at the same time, a culture supernatant was obtained. For the antibody contained in the supernatant, the isotype of the antibody was confirmed by a normal sandwich ELISA method, and then the antibody titer of the antibody contained in the supernatant was measured and separated as each isotype antibody-producing hybridoma. For the antibodies used in the ELISA, 2 µg/ml of Anti-goat-mouse IgA (Southern Biotech), Anti-goat-mouse IgG (Southern Biotech), or Anti-goat-mouse IgM (Southern Biotech) was used for coating the plate, and 0.5 µg/ml of Alkaline Phosphatase (ALP)-conjugated anti-goat mouse IgA (Southern Biotech), Alkaline Phosphatase (ALP)-conjugated anti-goat mouse IgG (Southern Biotech), or Alkaline Phosphatase (ALP)-conjugated anti-goat mouse IgM (Southern Biotech) was used for detection. Mouse IgAκ (Immunology Consultants Laboratory), Purified mouse IgG1κ Isotype Control (BD Pharmingen), and PE-CF594 mouse IgMκ Isotype Control (BD Horizon) were used as control antibodies. OD₄₀₅ nm was measured using TriStar² LB942 (BERTHOLD TECHNOLOGIES). 500 or more hybridoma clones were obtained in total by the above operation.

### (1-3) Analysis of Binding Force of Hybridoma IgA Antibody to Each Intestinal Bacterium

To screen for antibodies binding to each intestinal bacterium, ELISA for each bacterium was performed. In particular, a screening method performed on Clostridium difficile (C. difficile) is described. C. difficile was subjected to anaerobic culture (Brain Heart Infusion media, 80% N₂, 10% H₂, 10% CO₂) at 37°C, and collected by centrifugation. Bacteria were suspended in a 0.05 M Na₂CO₃ buffer and coated onto an ELISA plate. After blocking with PBS containing 1% BSA, each antibody culture supernatant in a 96-well plate was added, and a reaction was allowed to proceed at room temperature for about 1 hour. Thereafter, the plate was washed with PBS added with 0.05% Tween 20, the secondary detection antibody (described above) corresponding to each antibody isotype was added and reacted, and then a color development reaction was performed using Alkali Phosphatase tablet (Sigma). For sufficient reaction, the ELISA plate after color development was reacted at 4 degrees overnight, and OD₄₀₅ nm was measured using TriStar² LB942 (BERTHOLD TECHNOLOGIES). A clone having OD₄₀₅ nm of 2.0 or more was defined as an antibody binding to C. difficile.

The selected clones were expanded and cultured (about 100 mL culture), and antibodies were purified from the culture solution using a Protein L column (Cytiva) in the case of IgM and IgA antibodies, and using a Protein A column (Cytiva) in the case of IgG antibodies. Elution of the antibodies was performed using a 10 mM citrate buffer (pH 2.5), the eluate was immediately neutralized with a 1 M citrate buffer (pH 9.0), and then, the neutralized eluate was concentrated with Amicon (100 kDa), dialyzed against a dialysis membrane (100 kDa pore size), and replaced with PBS. After dialysis, the antibody solution was recovered in a clean bench, and then the antibodies were sterilized using a syringe filter (0.22 um) and stored at 4°C. A concentration of the antibodies was measured by a sandwich ELISA method in the same manner as described above.

RNA of the selected clone was extracted by ELISA, and the full length of the antibody gene was cloned and used to prepare a recombinant antibody.

### (1-4) Cloning of Full Length of Antibody Gene of Hybridoma-Derived Antibody

RNA was extracted from the cells of each clone using IsogenII (Nippon Gene Co., Ltd.). cDNA was synthesized using the extracted RNA as a template, and RT-PCR was performed using 7 types of primers (MH1-7) for a V_{H} region of the antibody and Cα, Cα, or Cγ region-specific primers.

For the light chain, PCR was performed with a Vκ primer and a Cκ primer. The base sequences of the primers are shown in Table 1. The amplified V_{H} region PCR product or Vκ region PCR product was directly sequenced, and the variable region gene sequence of each clone was obtained. This was searched with Ig blast, further upstream sequences (including signal sequences) of specific V_{H} or Vκ genes were obtained, PCR primers were prepared based on the most upstream sequences, and PCR was performed together with the reverse primer at the 3' end of each C region secretory sequence, thereby obtaining full-length gene base sequences of an H chain and an L chain. Based on this, pcDNA 3.1 (+) vector (Invitrogen) was cloned into the H chain, L chain, and J chain (search a base sequence from database and obtain full-length gene sequences by PCR using cDNA of hybridoma).

**Table 1. Base sequence of primer for amplification of antibody gene variable region of hybridoma-derived antibody**

| Primer name | | Base sequence (5'-3') |
|---|---|---|
| MH1 | SARGTNMAGCTGSAGTC | |
| MH2 | SARGTNMAGCTGSAGSAGTCWGG | |
| MH3 | CAGGTTACTCTGAAAGWTSTG | |
| MH4 | GAGGTCCARCTGCAACARTC | |
| MH5 | CAGGTCCAACTVCAGCARCC | |
| MH6 | GAGGTGAASSTGGTGGAATC | |
| MH7 | GATGTGAACTTGGAAGTGTC | |
| IgAR | GATGGTGGGATTTCTCGCAGAC | |
| degVκ | GGCTGCAGSTTCAGTGGCAGTGGRTCWGGRAC | |
| CacR | AACGTGAGGGTGCTGCTCATG | |

In the base sequences in the table, S represents G or C, R represents A or G, N represents A, C, G, or T, W represents A or T, and V represents G, C, or A.

The above expression vectors were transfected into Expi CHOS cells (Thermo Fischer Scientific) according to the kit protocol so that the amount ratio was H:L:J = 2:2:1. On days 10 to 12 after transfection (Thermo Fischer Scientific, ExpiCHO Expression System), the culture supernatant was recovered, and the antibody was purified and concentrated on a Protein L column in the same manner as described above, replaced with PBS by dialysis, sterilized with a filter, and then used for antibody titer measurement or activity test.

### Example 2: Bacterial Growth Inhibition Test

### (Materials and Methods)

Under the same conditions as in Example 1, C. difficile was anaerobically cultured at 37°C overnight. The culture solution was centrifuged, and bacteria were collected and then washed twice with the culture solution. After the bacterial solution was diluted 10 times with a culture solution, SYTO24 (Invitrogen) and Counting beads contained in Cell Viability Kit (Becton Dickinson) were added, and the number of SYTO24 positive viable bacteria was calculated by flow cytometry from comparison with the Counting beads. The culture solution was diluted to 10,000 cells/5 µl of bacteria. 5 µl of the bacterial diluent was added to an Eppendorf tube, 25 µl (antibody concentration: 1 mg/ml) of PBS (anaerobized) or purified test antibodies (anaerobized) was further added, and static culture was performed at 37°C for 1 hour. Thereafter, 30 µl of the culture solution was added, and static anaerobic culture was performed at 37°C for 20 hours (final concentration of antibody: 0.42 mg/ml).

After culturing for 20 hours, SYTO24 and Counting beads were added in the same manner as described above, and the number of viable bacteria was measured by flow cytometry. An antibody in which the number of viable bacteria decreased to 30% or less as compared with the PBS sample (without addition of the antibody) was regarded as having a growth inhibitory effect, and used for the next mouse infection experiment.

7 types of antibodies SNK0001MR, SNK0002MR, SNK0001AR, SMK0002AR, RS_H007_L004, RS_H000_L001, and SNK0003A were used for the growth inhibition test. Antibodies SNK0001MR and SNK0002R are recombinant IgM antibodies prepared by transfecting CHO cells with expression vectors (H chain and L chain) obtained based on antibody gene sequence information extracted from IgM-producing hybridomas (SNK0001M and SNK0002M). SNK0003A is an IgA antibody derived from an IgA-producing hybridoma. SNK0001AR and SNK0002AR are recombinant multimeric IgA antibodies prepared by preparing an expression vector in which a gene sequence of an antibody gene variable region derived from SNK0001M and SNK0002M hybridomas and an IgA antibody constant region gene sequence bound, further constructing a J-chain expression vector, and transfecting CHO cells with 3 types of expression vectors (H chain, L chain, and J chain). RS_H007_L004 and RS_H00_L001 are recombinant multimeric IgA antibodies obtained by synthesizing a gene based on the gene sequence of the W27 antibody produced by the W27-producing hybridoma obtained from intestinal mucosa lamina propria cells reported by the inventors in WO 2014/142084 A and the like, and applying a recombination technique. All recombinant antibodies were purified by Protein L column by the method described above. These antibodies were confirmed to bind to C. difficile by the same test as in Example 1.

### (Results)

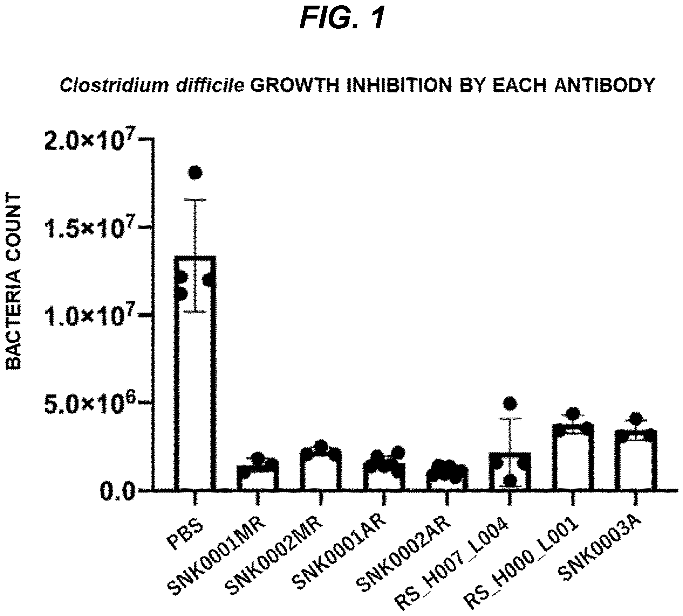

The results are illustrated in Fig. 1. All the tested antibodies showed a remarkable ability to inhibit a growth of C. difficile as compared to the negative control (PBS).

### Example 3: C. difficile Mouse Infection Experiment

### (Materials and Methods)

For C. difficile strain (VPI10483), a spore fluid was prepared, divided into small portions, and stored at -80 degrees. The number of viable bacteria obtained by thawing the spore liquid, culturing the thawed spore liquid, and then seeding the thawed spore liquid on C. difficile selective medium plate (TCCFA plate) was determined in advance.

The preparation method of spores is as follows.

C. difficile was plated on SMC medium and anaerobically cultured at 37°C for 7 days. 3 ml of ice-cold sterile water was added to the plate, and colonies were scraped off with a cell scraper and transferred to a 50 ml tube. Further, the plate was washed with 3 ml of ice-cold sterile water, and the washing liquid was also transferred to the same tube. After centrifugation at 8,000 g and 4°C for 10 minutes, the supernatant was discarded, and the bacterial bodies were suspended in 20 ml of ice-cold sterile water.

After centrifugation again at 8,000 g and 4°C for 10 minutes, the supernatant was discarded, and the bacterial bodies were suspended in 10 ml of ice-cold sterile water. After the suspension, 10 ml of ethanol was added, stirring was performed by vortex, and the resulting product was allowed to stand at room temperature for 1 hour. Centrifugation was performed at 8,000 g and 4°C for 10 minutes, the supernatant was discarded, and the bacterial bodies were suspended in 20 ml of ice-cold sterile water. This operation was repeated twice, and the supernatant was discarded and then suspended in 5 ml of ice-cold sterile water. Each 100 µl was dispensed into a 1.5 ml tube and stored at -80°C. The SMC medium composition is shown below.

### SMC Medium Composition

The reagents were dissolved in 200 ml of sterile distilled water and sterilized in an autoclave. When the medium was cooled to about 60°C, 600 µl of 10% (w/v) L-cysteine was added, and the medium was dispensed in an appropriate amount onto a 10 cm plate.

| Reagent name | Usage |
|---|---|
| Bacto Peptone | 18 g |
| Bacto Proteose Pe ptone | 1 g |
| Ammonium sulfate | 200 mg |
| Tris | 300 mg |
| Agar | 3 g |

In the C. difficile selective medium plate, the reagents in Table 1 were dissolved in 400 ml of sterile distilled water and sterilized in an autoclave. The medium was naturally cooled to 60°C, 2 ml of Cycloserine (Sigma-Aldrich) (50 mg/ml) and 2 ml of Cefoxitin (Sigma-Aldrich) (1.6 mg/ml) were added thereto, and the medium was dispensed in an appropriate amount onto a 10 cm plate to prepare spores.

**(Table 1) C. difficile selective medium composition**

| Reagent name | Usage |
|---|---|
| Bacto Proteose Peptone | 20 g |
| Disodium hydrogen phosphate dodecahydrate | 2.68 g |
| Potassium dihydrogen phosphate | 0.4 g |
| Sodium chloride | 0.8 g |
| Magnesium sulfate | 0.048 g |
| D- (-) -Fructose | 2.4 g |
| Taurocholic acid sodium salt hydrate | 0.4 g |
| Agar | 6.64 g |
| Neutral Red Solution(0.3 3%) | 1 ml |

C57BL/6 mice (8-week-old) were purchased from CLEA Japan, Inc., and acclimatized in a sterile isolator for an infection experiment for 1 week, and then 3 types of mixed antibiotics (Gentamicin: 500 mg/kg, Kanamycin: 150 mg/kg, Metronidazole: 50 mg/kg) were orally administered for 4 days using a sonde. After 4 days, the body weight of each mouse was measured, individuals in which a weight loss occurred by antibiotic administration were excluded, and the remaining mice were randomly grouped. On the day after the completion of antibiotic administration, each mouse was orally infected with C. difficile spores (1 × 10³ cfu) using a sonde. After 6 hours, 100 µg of the test antibody was orally administered by a sonde. Thereafter, 100 µg of the antibody was administered once a day for a total of 7 days. After the 8^{th} day, progress was observed without antibody administration. During this time, body weight measurement, property observation of feces, and collection of feces of the mice were performed. Feces collected on days 4 and 10 after infection were suspended by adding PBS in an amount depending on the body weight, and then serial dilution was further performed with PBS. These diluted bacterial solutions were seeded on a C. difficile selective medium plate, and anaerobic culture was performed to determine a viable cell count in 1 g of feces.

### (Results)

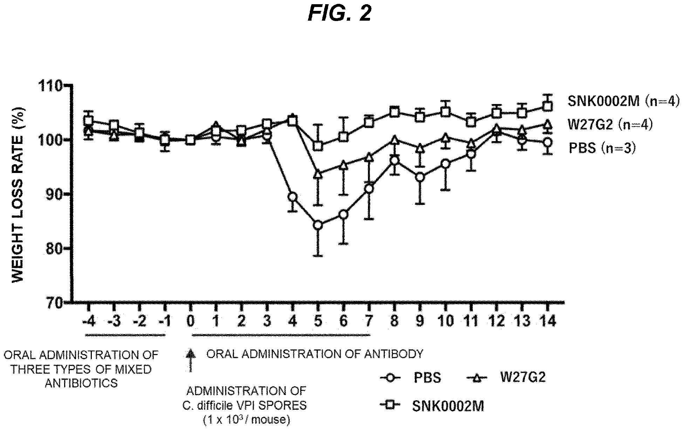

In the mouse group to which the W27G2 or SNK0002M antibody was administered, weight loss after administration of the C. difficile spore fluid was suppressed as compared with the negative control (PBS administration group) (Fig. 2).

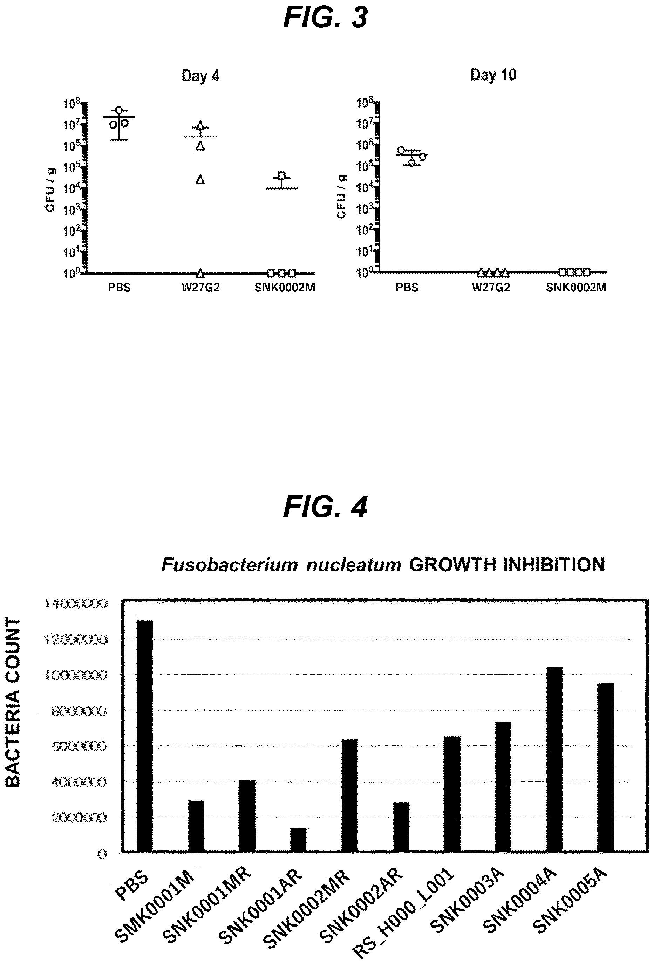

In addition, with respect to C. difficile viable bacteria in feces, it was confirmed that infection to each mouse was established from observation of a viable cell count sufficient for feces on the 4^{th} day. Regarding feces on the 10^{th} day, the viable cell count was drastically reduced in the mouse group to which the W27G2 antibody or the SNK0002M antibody was administered, and thus it was confirmed that the growth of bacteria was significantly inhibited even in a living body by these antibodies (Fig. 3).

### Example 4: Effect on Bacteria Other Than C. difficile

For the antibody binding to C. difficile, the binding characteristics to other bacteria and the effect on the bacteria were examined by the same method as the ELISA used in Example 1. As an example, the results of examining the binding characteristics and effects on Fusobacterium nucleatum considered to be a causative bacterium of colorectal cancer, are presented. After anaerobic culture of the bacteria, the number of bacteria was adjusted to 1,000 cells/tube using flow cytometry and reacted with each test antibody to verify the growth inhibitory effect by the antibody. The final concentration and culture time of the added antibody were similar to those in Example 2.

After the reaction between the antibody and the bacteria, the culture solution was added, and the viable cell count was measured after further culturing for 20 hours.

### (Results)

Antibodies SNK0001M, SNK0001MR, SNK0001AR, SNK0002MR, SNK0002AR, RS_H000_L001, and SNK0003A showed a remarkable growth inhibitory effect against Fusobacterium nucleatum (Fig. 4). On the other hand, SNK0004A and SNK0005A did not show the growth inhibitory effect (negative control).

### Example 5: Identification of C. difficile Antigen Molecules of W27 Antibodies

It was confirmed that the W27 antibody bound to serine hydroxymethyl transferase (SHMT) of E. coli (WO 2014/142084 A and the like), and it was confirmed that the W27 antibody bound to E. coli SHMT and a C. difficile antigen molecule of the W27 antibody was identified by the following method. The W27G2 antibody has the same heavy chain variable region and light chain variable region as the W27 antibody.

### (Materials and Methods)

E. coli was statically cultured in 25 ml of LB medium for 16 hours. Bacteria were collected by centrifugation at 2,150 g for 5 minutes. After removing the supernatant, the medium was suspended in 1 ml of a lysis buffer (sterile 1 × PBS, 10% NP-40, ×100 Protease Inhibitor Cocktail (Nacalai)), ultrasonically crushed, and then allowed to stand on ice for 30 minutes. After dispensing 200 µl of the bacterial lysate into a new tube, 50 µl of 2-ME and 200 µl of 10% SDS were added and denatured (95°C, 10 min). To dilute 2-ME and SDS, 800 µl of a diluent (sterile 1 × PBS, 0.1% NP-40, ×100 Protease Inhibitor Cocktail) was added. In order to separate the protein solution into soluble and insoluble fractions, centrifugation was performed at 15,000 rpm for 5 minutes. The soluble fractions were used to perform 2D-PAGE to identify target protein spots.

For C. difficile, a bacterial lysate was prepared in the same manner, and 2D-PAGE was performed using the soluble fractions.

The method of 2D-PAGE is described below. First, in order to prepare a sample of 2D-PAGE, cooled acetone was added in an amount of 2 times the amount of the immunoprecipitation product, the mixture was allowed to stand at -20°C for 20 minutes, the supernatant was removed by centrifugation (16,630 g, 10 min), and then the mixture was dried. The sample was dissolved in a sample buffer for the one-dimensional electrophoresis (60 mM Tris-HCl (pH 8.8), 5 M Urea, 1 M Thiourea, 5 mM EDTA, 1% CHAPS, 1% NP-40), 1/10 of 1 M iodoacetamide was added thereto, and the mixture was allowed to stand at room temperature for 10 minutes, thereby preparing a one-dimensional sample for 2D-PAGE. For the two-dimensional electrophoresis, two SDS-PAGE gels were used for performing Western blot and silver stain. After the one-dimensional electrophoresis using Immobiline^{™} DryStrip (GE Healthcare) with a pH of 3 to 8, the two-dimensional SDS-PAGE was performed, and then Western blot and silver stain were performed on each gel. In order to easily identify the spot recognized by the W27 antibody, the membrane after blotting was stained with Pierce^{™} Reversible Protein Stain, and the positions of all proteins on the membrane were confirmed. This stain was erased with a stain eraser, and then the W27 antibody was reacted to confirm a spot by Western blot. Silver stain was performed using Sil-Best Stain One (Nacalai) and following the protocol. By comparing the results of these three types (Western blot, Pierce^{™} Reversible Protein Stain, and silver stain by W27), a spot specifically recognized by the W27 antibody was cut out and subjected to in-gel enzyme digestion.

A method of in-gel enzyme digestion is described below. 400 µl of MilliQ water was added to the cut gel piece, the mixture was shaken for 10 minutes, and then the supernatant was removed. This operation was repeated twice, 200 µl of 100% acetonitrile was then added, and the mixture was shaken for 10 minutes. After removing the supernatant, drying under reduced pressure was performed for 20 minutes, 20 µl of a protease solution (50 mM ammonium hydrogen carbonate, 10 µl/ml Trypsin) was added, and the gel piece was swollen on ice for 30 minutes to absorb trypsin. The surplus protease solution was removed, 100 µl of a reaction solution (50 mM ammonium hydrogen carbonate) was added, and enzyme digestion was performed at 37°C overnight. 50 µl of the extract (5% formic acid, 50% acetonitrile) was further added to the reaction solution, and the mixture was shaken for 30 minutes. 200 µl of 0.1% formic acid was added to the recovered solution, and drying under reduced pressure was performed until the sample amount reached about half. The sample was transferred to a spin filter (UltraFree 0.1 µm PVDF) and centrifuged (2,460 g, 5 min, 4°C). The solution dropped on the lower layer was transferred to a vial and analyzed with LCMS-IT-TOF (Shimadzu). Mascot search was used to identify the amino acid sequence.

### (Results)

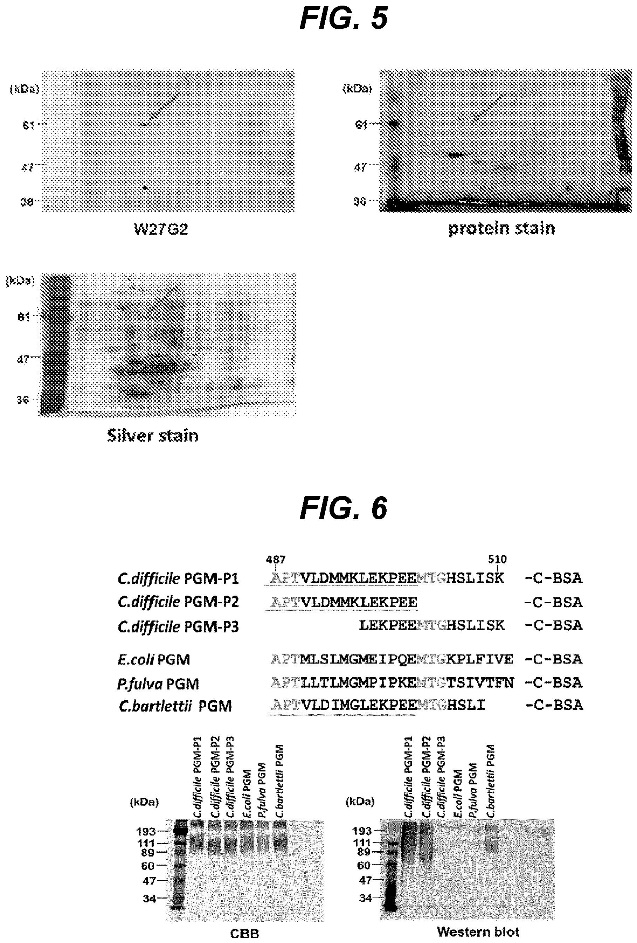

E. coli SHMT and 2,3-bisphosphoglycerate-independent phosphoglycerate mutase (iPGM) of C. difficile were identified as antigen molecules of the W27 antibody by the above method. As an example, two-dimensional electrophoresis data when an antigen molecule of C. difficile is identified is illustrated in Fig. 5.

### Example 6: Determination of Epitope of Antigen Molecule of W27 Antibody

It was identified that the W27 antibody was an epitope at the N-terminal 25 to 30 amino acids of E. coli SHMT (published paper). An attempt was made to prepare a truncation protein also for C. difficile iPGM, but the efficiency of protein expression was significantly reduced, and thus, the reaction of the W27 antibody was not confirmed by Western blot analysis.

Therefore, the fact that the W27 antibody did not recognize E. coli iPGM was confirmed by Western blot, and a plasmid expressing a chimeric protein of C. difficile iPGM and E. coli iPGM recognized by the W27 antibody was prepared using In-Fusion (registered trademark) HD Cloning Kit (TaKaRa). Primers were created from the iPGM gene sequence information of each of bacteria on the database (table). The chimeric protein was overexpressed with DH5α, and the recognition of the W27 antibody was confirmed by Western blot to narrow down the site of the epitope sequence.

**Table 2: Primer base sequences used for In-Fusion cloning**

| ***C. difficile* PGM** | |
|---|---|
| | Base sequence (5'-3') |
| *C.dif* F primer | GTG GTG GAA TTC ACC ATG ATG AAA AAA CCA GTT GCT |
| *C.dif* R primer | GAA GGG CCC TCT AGA TTT TGA AAT AAG TGA ATG ACC |
| *C.dif* 50F primer | GAT GTT GGT CTA CCT GAT GGT CAA ATG GGA AAT TCA |
| *C.dif* 50R primer | AAG ACC ACT AGC ATT TAT CAA TGT GTT TGG ATA CTC |
| *C.dif* 320F primer | AAA ACT CAA CTT AGA GCT GCT GAA ACT GAA AAG TAT |
| *C.dif* 320R primer | TCC ATT TTT AGC TAG ATA CTC ACC TAG AGT GTT GGC |
| *C.dif* 390F primer | GTA TTA AAC TTT GCA AAT CCT GAT ATG GTA GGT CAT |
| *C.dif* 390R primer | TAT AAA GTC AAA CTT ATC TTC TCC TAG TTT ATC TAA |
| *C.dif* 411F primer | ATG GAA GCG GCG GTT AAG GCT GTT GAA ACT GTA GAT |
| *C.dif* 443F primer | GCT GAC CAC GGT AAC GCT GAA TAT ATG TTA GAC CCA |
| *C.dif* 464F primer | ACC AAC CTG CCA GTT CCA TTT ATT GTA GTT GGT CAA |
| *C.dif* 487F primer | AAA CTT TCT GAC ATC GCA CCG ACT GTT TTA GAT ATG |
| *E.coli* F primer | GTG GTG GAA TTC ACC ATG TTG GTT TCT AAA AAA CCT |
| *E.coli* R primer | GAA GGG CCC TCT AGA TTC CAC GAT GAA CAG CGG CTT |
| *E.coli* 52F primer | AAT GCT AGT GGT CTT GAA GTC GGT CTG CCT GAC CGT |
| *E.coli* 52R primer | CAG ACC GGA AGC GTC GAT CAG AGG TAG ACC AAC ATC |
| *E.coli* 325F primer | CTA GCT AAA AAT GGA AAA ACT CAG TTG CGT ATT TCC |
| *E.coli* 325R primer | GTC GTT TTT CGC CAT CCA CTC TCT AAG TTG AGT TTT |
| *E.coli* 395F primer | AAG TTT GAC TTT ATA ATC TGT AAC TAT CCG AAC GGC |
| *E.coli* 395R primer | AAT GGT GTC GTA TTT GCC GCT TGC AAA GTT TAA TAC |

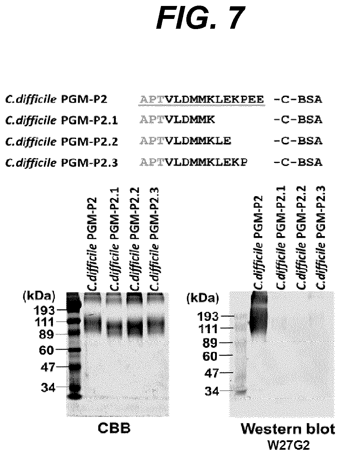

As a result of the analysis, in C. difficile iPGM, the amino acids at positions 490 to 510 at the C-terminus were important as epitopes. Peptides containing each epitope candidate (BSA-conjugate) were outsourced to SIGMA to further confirm epitopes. Since the molecular weight of the peptide alone was small, BSA was conjugated to each peptide as confirmed by Western blot. 4 × SDS buffer was added to the peptide, and Western blot with the W27 antibody was performed to confirm which synthetic peptide was reacted. The results are illustrated in Figs. 6 and 7.

As a result of the above test, the epitope sequence of E. coli SHMT was found to be RQEEHIELIASEN, and the epitope sequence of C. difficile iPGM was found to be APTVLDMMKLEKPEEMTGHSLISK.

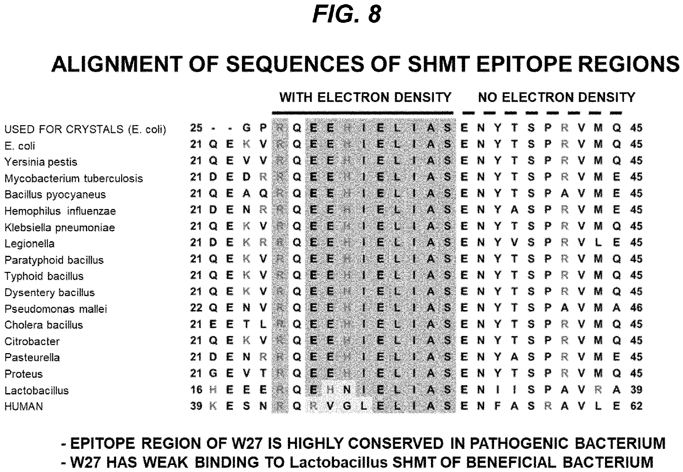

As a result of database analysis of bacteria sharing an epitope of E. coli SHMT recognized by the W27 antibody, bacteria having an EEHI sequence were mostly bacteria belonging to Proteabacteria, and contained many pathogenic bacteria (Fig. 8). The amino acid sequences at the same portion of lactobacillus and human SHMT were different, and it was considered that the W27 antibody recognized this difference. (Okai et al., Nature microbiology 1,16103,2016)

### Example 7: Crystal Structure Analysis of W27 Antibody and Antigen Molecule

### (7-1) Preparation and Purification of IgG W27 Fab

To examine the antigen recognition of the W27 antibody, a W27 recombinant IgG antibody expressed by CHO (Chinese hamster ovary) cells was prepared. This is because it is necessary to secure a large amount of antigen binding sites (Fab) for crystal structure analysis. The crude purified IgG W27 was adjusted to 1 mg/ml with 10 mM Tris-HCl (pH 8.0), and then digested with 0.05 mg/ml papain (Nacalai Tesque) at 20°C for 24 hours to digest the Fc region and the Fab region. Thereafter, purification was performed by cation exchange column chromatography using HiTrap SP HP column (Cytiva). As the buffer, 10 mM Bis-Tris buffer (pH 6.0) (buffer A), 10 mM Bis-Tris buffer (pH 6.0), and 300 mM NaCl (buffer B) were used, and Fab and Fc were separated and purified by linear gradient elution in which a concentration of the buffer B was gradually increased. Thereafter, the Fab rich fraction was further purified by gel filtration chromatography using a HiLoad 26/600 Superdex 2000 pg gel filtration column (Cytiva). As a buffer for gel filtration, 5 mM BisTris (pH 6.5) and 100 mM NaCl (gel filtration buffer) were used. After purification by gel filtration chromatography, impurities of a trace amount of Fc were adsorbed and removed by a column filled with rProtein A Sepharose Fast Flow resin (Cytiva), and Fab present in the flow-through fraction was recovered. The purified Fab was concentrated to 48.2 mg/ml with Amicon Ultra 10,000 MWCO (Millipore) under the conditions of a gel filtration buffer, dispensed into an Eppendorf tube by 20 µl, instantaneously frozen with liquid nitrogen, and then stored at -80°C until use. The yield of Fab was 17.4 mg from 50 mg of IgG W27.

### (7-2) Expression and Purification of E. coli SHMT (25-45) (For Crystal Structure Analysis)

The cDNA region encoding E. coli-derived SHMT (25-45) was amplified using PrimeSTAR Max DNA Polymerase (TaKaRa), and then the plasmid pGEX6P-3 (Cytiva) was incorporated into the Sma I and NotI multiple cloning sites of the modified plasmid pGEXM (Kim SY, et al. (2021) Sci Rep 11 (1):2120) using In-Fusion HD Cloning Kit (Clontech) to construct an expression plasmid. The prepared expression plasmid was transformed into E. coli Rosetta2 strain (Merck). Note that SHMT (25-45) was expressed as a Gultathione-S-transferase (GST) fusion protein (hereinafter, referred to as GST-SHMT (25-45)). In addition, since a cleavage sequence LEVLFQGP by human rhinovirus 3C protease (hereinafter, HRV3C protease) (the cleavage site is between Q and G) is inserted between GST and the target protein, the expressed GST fusion protein can separate GST and the target protein from each other by HRV3C protease. The amino acid sequence of the final purified E. coli SHMT (25-45) is GPRQEEHIELIASENYTSPRVMQ.

Culture of E. coli was started at 37°C using LB medium containing 50 ug/ml of ampicillin, 25 ug/ml of chloramphenicol, 0.5% (W/V) of glycerin, 0.05% (W/V) of glucose, 0.2% (W/V) of lactose, and 1 mM magnesium sulfate. When the turbidity of the medium reached 0.6 (measured at a wavelength of 600 nm), the medium was cooled, and then 1 M isopropyl-β-D-thiogalactopyranoside (hereinafter, IPTG) was added so that the final concentration was 0.1 mM to induce expression of a target protein. After addition of IPTG, culture was further performed at 18°C for 24 hours. The bacterial bodies in the culture solution were collected by centrifugation at 4,000 rpm (Beckman J2-M1 JA10 rotor) at 4°C for 20 minutes. The collected bacterial bodies were stored at -30°C until used for purification.

Purification of SHMT was performed as follows. The bacterial bodies were suspended in 10 mM Tris-HCl (pH 8.0) and 300 mM NaCl, ultrasonically disrupted at 4°C, and then centrifuged at 4°C and 20,000 rpm for 40 minutes. Then, the insoluble fraction was removed, and the soluble fraction of the supernatant was used for the next purification. Purification of GST-SHMT (25-45) from the soluble fraction was performed using an affinity column using Glutathione Sepharose 4B resin (hereinafter, GS4B) (Cytiva). The soluble fraction after disruption was applied to an affinity column filled with GS4B to adsorb GST-SHMT (25-45), and then GS4B was sufficiently washed with 10 mM Tris-HCl (pH 8.0), 300 mM NaCl (hereinafter, washing buffer). Thereafter, GST-SHMT (25-45) was eluted from the resin with 10 mM Tris-HCl (pH 8.0), 300 mM NaCl, and 20 mM Glutathione. The eluate was digested using HRV 3C protease (Merck) at 4°C for 18 hours to cleave into GST and SHMT (25-45), and GST was removed. In SHMT (25-45), an extra GP sequence including a part of the recognition sequence of HRV3C protease was added at the N-terminal. SHMT (25-45) after GST cleavage was subjected to gel filtration purification using HiLoad 26/600 Superdex 75 pg gel filtration column (Cytiva) under the conditions of 5 mM BisTris (pH 6.5) and 100 mM NaCl. The purified SHMT (25-45) was concentrated to 4.72 mg/ml with a centrifugal device with 1 K available from PALL Corporation under the conditions of 5 mM BisTris (pH 6.5) and 100 mM NaCl (gel filtration buffer), dispensed into an Eppendorf tube by 20 µl, instantaneously frozen with liquid nitrogen, and then stored at - 80°C until use. As for the yield, 5.9 mg of SHMT (25-45) used for crystallization was obtained from 2 L of the cultured bacterial bodies.

### (7-3) Expression and Purification of C. difficile iPGM (486-509) (For Crystal Structure Analysis)

Cloning of cDNA of Clostridium difficile-derived iPGM (486-509) (C. difficile iPGM (486-509)) into a pGEX vector was performed by almost the same method as SHMT. At this time, since tyrosine or tryptophan was not present in C. difficile iPGM (486-509) and absorption at a wavelength of 280 nm was not observed in the purification process, a tyrosine 1 residue was added at the N-terminal and cloning was performed. In iPGM (486-509), an extra GPY sequence including a part of the recognition sequence of HRV3C protease was added at the N-terminal. The amino acid sequence of the final purified C. difficile iPGM (486-509) is GPYAPTVLDMMKLEKPEEMTG HSLISK. The procedures related to transformation, culture, and purification were performed in the same manner as in SHMT (25-45), and 48.52 mg of iPGM (486-509) used for crystallization was obtained from 4 L of the cultured bacterial bodies.

### (7-4) Crystallization Screening of RS_H000_L000GR Fab-E. coli SHMT (25-45) Complex

A mixed solution was prepared using the purified RS_H000_L000GR Fab and E. coli SHMT (25-45) at a molar ratio of 1:5 (0.2 mM:1.0 mM), and crystallization screening was performed.

Screening of crystallization conditions was performed using JCSG core suite I-IV, PACT suite (Qiagen), which is a commercially available crystallization screening kit, and a crystallization robot mosquito (TTP Labtech). A crystallization plate (VIOLAMO) was filled with a precipitating agent for equilibration, droplets (drops) obtained by mixing the precipitating agent and the protein solution at a volume ratio of 1:1 (0.2 µl:0.2 µl) were prepared, and the crystallization conditions were searched by a sitting drop vapor diffusion method. As the protein solution used in the crystallization screening, a solution obtained by removing an insoluble fraction by centrifugation in advance was used. The temperature conditions were 4°C and 20°C.

### (7-5) Optimization of Crystallization Conditions of RS_H000_L000GR Fab-E. coli SHMT (25-45) Complex

A precipitating agent whose pH and PEG concentration were changed based on the conditions of the crystals obtained by the crystallization screening was prepared, and the precipitation was performed by a sitting drop vapor diffusion method.

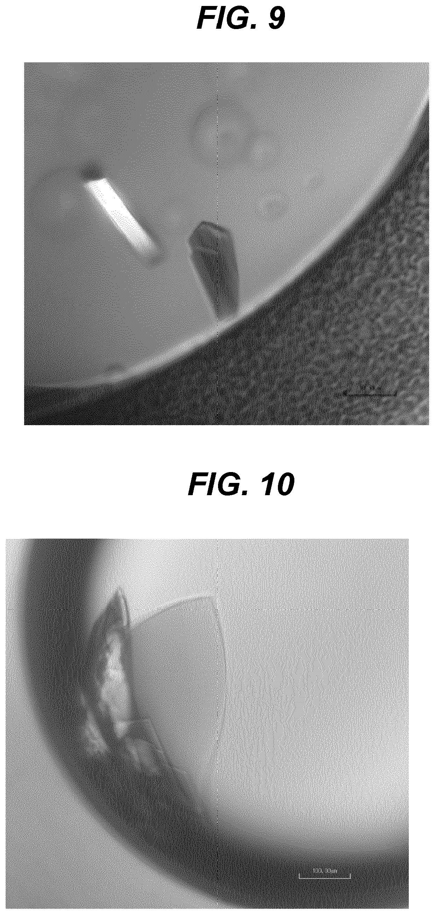

Crystallization conditions were 0.2 M Magnesium Acetate, 0.1 M Sodium Cacodylate, pH 6.3, and 22% PEG 10000 as a reservoir solution. The protein solution obtained by mixing 0.2 mM RS_H000_L000GR Fab and 0.6 mM E. coli SHMT (25-45) and the reservoir were mixed at 0.2 µl:0.2 ul, the temperature condition was 20°C, and crystallization by the sitting drop vapor diffusion method was observed after 7 days. The scale bar indicates 100 um (Fig. 9).

### (7-6) X-Ray Diffraction Experiment and Three-Dimensional Structure Determination

of RS_H000_L000GR Fab-E. coli SHMT (25-45) Complex

The RS_H000_L000GR Fab-E. coli SHMT (25-45) complex crystals obtained in the optimization of the crystallization conditions described above were frozen in liquid nitrogen using 0.2 M Magnesium Acetate, 0.1 M Sodium Cacodylate pH 6.3, 22.0% PEG 10000, 20.0% Glycerol as an anti-freezing agent (cryoprotectant).

Thereafter, measurement was performed at BL44XU of a large synchrotron radiation facility SPring-8 in Hyogo-ken. The obtained diffraction data was processed by an XDS program, and then a phase was determined by a molecular substitution method using structural information of RS_H000_L000GR Fab.

The space group was P2₁2₁2₁, the lattice constants were a = 60.4 Å, b = 140.7 Å, c = 180.7 Å, α = 90°, β = 90°, and γ = 90°, and three molecules of RS_H000_L000GR Fab-E. coli SHMT (25-45) were present in the asymmetric unit. The structure refinement was performed using the programs refmac 5, phenix refine, and coot in combination, and R_{work}/R_{free} finished the refinement at 22.8% and 25.7%, respectively. The structural diagram was created using the program PyMOL.

### (7-7) Optimization of Crystallization Conditions of RS_H000_L000GR Fab-C. difficile iPGM (486-509) Complex

Crystallization of IgG W27 Fab C. difficile iPGM (486-509) was performed by a sitting drop vapor diffusion method using the precipitating agent prepared by changing the pH and the concentration of PEG 10,000 based on the structurally analyzed IgG W27 Fab-E. coli SHMT (25-45) crystallization conditions (0.2 M Magnesium Acetate, 0.1 M Sodium Cacodylate pH 6.3, 22% PEG 10000).

Crystallization conditions were 0.2 M Magnesium Acetate, 0.1 M Sodium Cacodylate, pH 6.5, and 23% PEG 10000 as a reservoir solution. The protein solution obtained by mixing 0.25 mM RS_H000_L000GR Fab and 1.0 mM C. difficile iPGM (486-509) and the reservoir were mixed at a mixing ratio of 0.2 µl:0.2 ul, the temperature condition was 20°C, the scale bar was 100 µm, and crystallization by the sitting drop vapor diffusion method was observed after 10 days (Fig. 10).

### (7-8) X-Ray Diffraction Experiment and Three-Dimensional Structure Determination of RS_H000_L000GR Fab-C. difficile iPGM (486-509) Complex

The RS_H000_L000GR Fab-C. difficile iPGM (486-509) complex crystals obtained in the optimization of the crystallization conditions described above were frozen in liquid nitrogen using 0.2 M Magnesium Acetate, 0.1 M Sodium Cacodylate pH 6.5, 23.0% PEG 10000, 20.0% Glycerol as an anti-freezing agent (cryoprotectant). Thereafter, measurement was performed at BL41XU of a large synchrotron radiation facility SPring-8 in Hyogo-ken. The obtained diffraction data was processed by an XDS program, and then a phase was determined by a molecular substitution method using structural information of IgG W27 Fab. The space group was P2₁2₁2₁, the lattice constants were a = 60.423 Å, b = 140.185 Å, c = 185.956 Å, α = 90°, β = 90°, and γ = 90°, and three molecules of RS_H000_L000GR Fab-C. difficile iPGM (486-509) were present in the asymmetric unit. The structure refinement was performed using the programs refmac 5, phenix refine, and coot in combination, and R_{work}/R_{free} finished the refinement at 22.2% and 25.2%, respectively. The structural diagram was created using the program PyMOL.

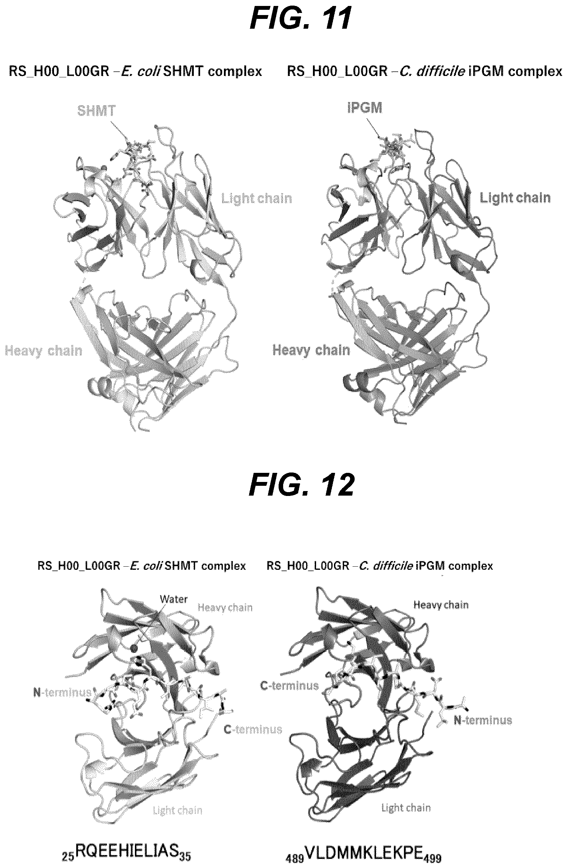

As a result of a series of experiments described above, the details of the interaction between the epitope peptide and the RS_H000_L000GR antibody were clarified (Figs. 11 and 12).

Specifically, epitope peptides of SHMT and iPGM bound in opposite directions to each other, and the amino acid sequence of the epitope showed the following similarity.
***E. coli* SHMT (N→C): RQ**E**E**H**-**I**E**LI**AS-**
***C. difficile* iPGM(C→N)**: **--**E**P**K**E**L**K**MM**DLV**

Based on these pieces of information, not only the type of the amino acid residue but also the effect of the amino acid side chain on the interaction can be determined. Based on these pieces of information obtained from the structural analysis, amino acid mutations that did not affect antigen binding were selected to prepare a series of RS mutant antibodies, and as described in detail below, it was revealed that all of them retained the same activity as that of the W27G2 antibody. Therefore, using the structural analysis result of the present invention, it has become possible to produce various RS mutant recombinant antibodies retaining the same activity as that of the W27G2 antibody.

### Example 8: Preparation of Genetically Modified Recombinant Antibody

For preparation of RS mutations, PCR was performed using an H-chain expression vector and an L-chain vector as templates and primers for mutagenesis containing base sequences corresponding to respective mutations, thereby amplifying the full length of the vector. Thereafter, the template vector DNA was treated with DpnI, and then the DNA was purified by isopropanol precipitation to transform into DH5α competent cells. The plasmid was extracted from the obtained clone, the clone into which the target mutation was introduced was selected, and the gene was introduced into ExpiCHO to obtain a mutant recombinant antibody.

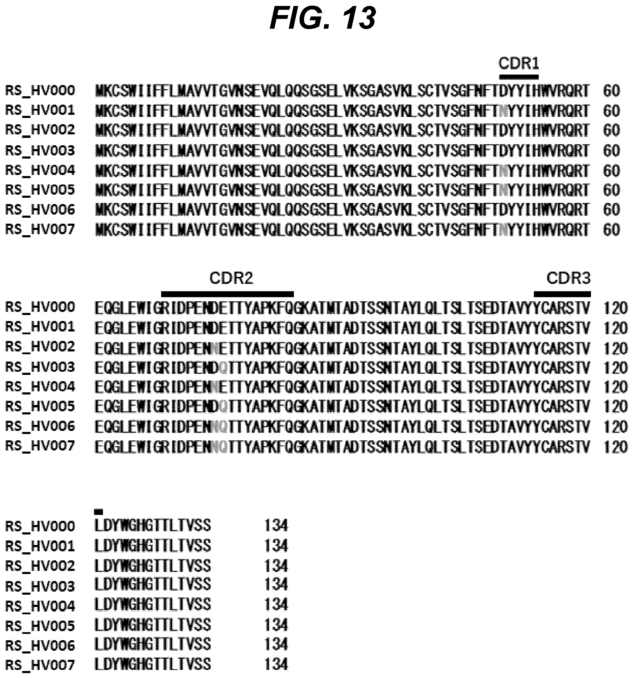

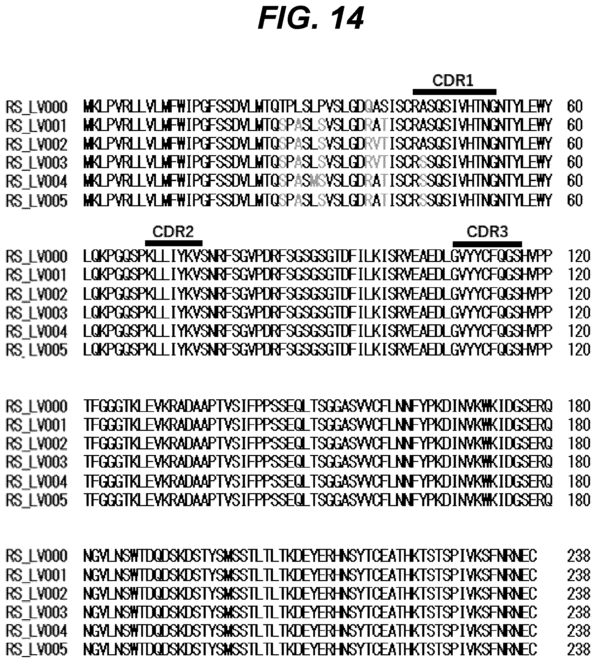

The amino acid sequences of the heavy chain variable region and the light chain variable region of a part of the produced mutant recombinant antibody are shown in Figs. 13 and 14.

By non-reduced SDS-PAGE and Coomassie blue stain of each RS mutation recombinant purified antibody, it was confirmed that dimers were mainly produced (Fig. 15).

### Example 9: Antigen Recognition Activity of Recombinant RS Modified Antibody

The binding force of the recombinant RS modified antibody to E. coli serine hydroxymethyltransferase (SHMT), which is an antigen molecule of the W27G2 antibody, was measured by ELISA.

### (Materials and Methods)

Wild-type SHMT and SHMT mutant of E. coli were overexpressed in E. coli as GST fusion proteins and purified. These bacteria were suspended in 0.05 M Na₂CO₃ buffer so as to be 2 µg/ml and immobilized to an ELISA plate, a dilution series of each variant purified antibody was prepared and added, and the binding force was detected by the same method as ELISA for measuring the binding force to the bacteria.

### (Results)

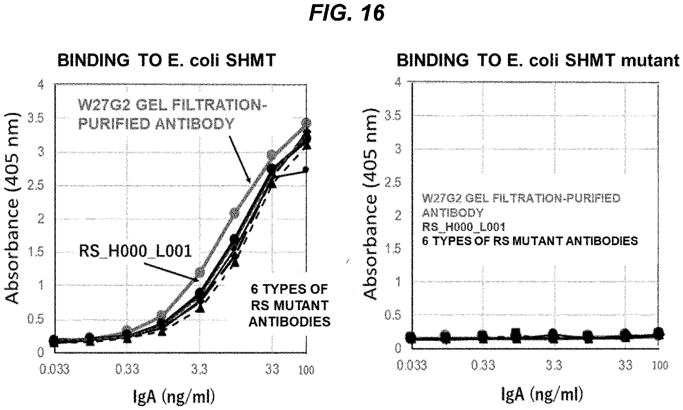

As a representative example, 6 types of mutant purified antibodies (RS_H00_L001, RS_H007_L001, RS_H007_L002, RS_H00_L005, RS_H007_L005, and RS_H007_L004) were tested, and it was confirmed that these antibodies bound to E. coli wild-type SHMT to almost the same extent as the hybridoma-derived W27G2 antibody (gel filtration purification), and did not bind to SHMT mutant (Fig. 16) .

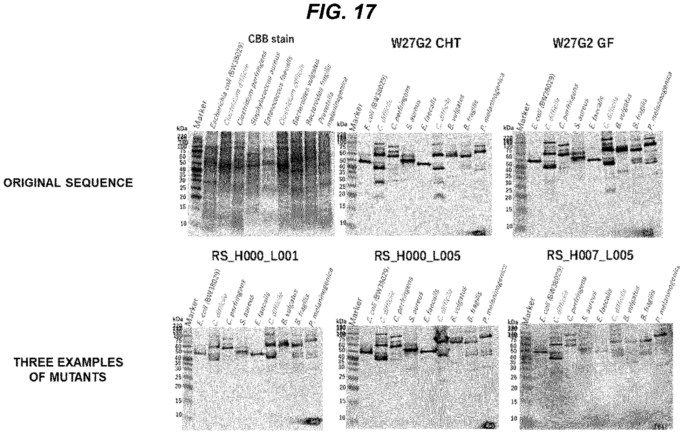

It was found that the W27G2 antibody was an antibody that recognizes different antigen molecules of a plurality of bacteria. Total proteins were extracted from the plurality of bacteria (including pathogenic bacteria) and proteins were transferred to nitrocellulose membranes after reduced SDS-PAGE, as shown by the Western analysis illustrated in Fig. 17. W27G2CHT (obtained by crudely purifying a W27G2 hybridoma culture supernatant with a hydroxyapatite column) and W27G2GF (obtained by further purifying W27G2CHT into a multimer fraction with a gel filtration column) were used as primary antibodies, and each of 3 types of recombinant purified antibodies (RS_H000_L001, RS_H000_L005, and RS_H007_L005) was reacted at a concentration of 2 ug/ml. Goat anti-mouse IgA (Southern Biotech) as a secondary antibody and IR800-conjugated anti-goat IgG (LI-COR) as a tertiary antibody were reacted, and a signal was detected with an Odyssey scanner. The gel after SDS-PAGE electrophoresis was stained using Coomassie brilliant blue (Nacalai) for the measurement of the protein amount of the sample loaded into each well. Since the hybridoma-derived W27G2 antibody and the recombinant purified antibody recognized antigen molecules of the same pattern, it was confirmed that there was no change in antigen recognition after introduction of mutation.

### Example 10: Confirmation of E. coli Growth Inhibitory Effect of Recombinant Gene-Modified Antibody

An E. coli BW38029 strain was subjected to stationary anaerobic culture at 37°C for 16 hours in LB medium. Bacteria were collected by centrifugation at 8,000 g for 5 minutes. After washing with sterile anaerobic LB, the viable cell count was measured with a flow cytometer.

Based on the above measurement results, bacteria were diluted with LB medium so as to be 300 cells/5 µl, 25 µl of each antibody or PBS was added, 30 µl of M9 minimum medium was further added, and after 20 hours, a bacterial count was measured by the above method using a flow cytometer. The final concentration of antibodies was 0.42 mg/ml.

### (Results)

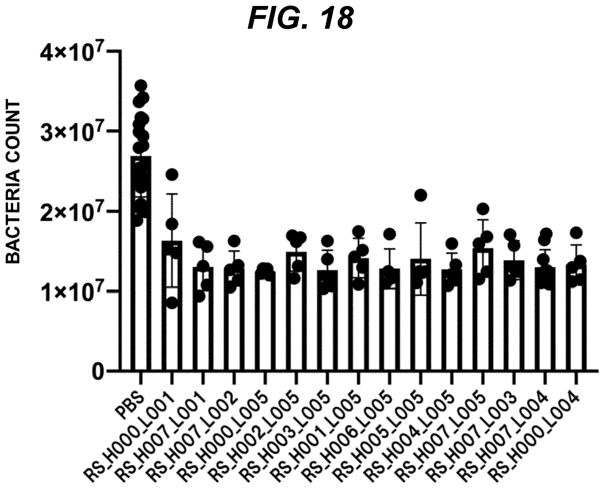

All of the various mutant recombinant antibodies inhibited the growth of E. coli as compared with the negative control (PBS added) sample (Fig. 18).

From all the experiments described above, it was revealed that the RS variant recombinant antibody prepared based on the three-dimensional structure analysis result retained the same properties as the original hybridoma W27G2 antibody.

### Example 11: Effect of Recombinant IgA Antibody on C. difficile Infected Mice

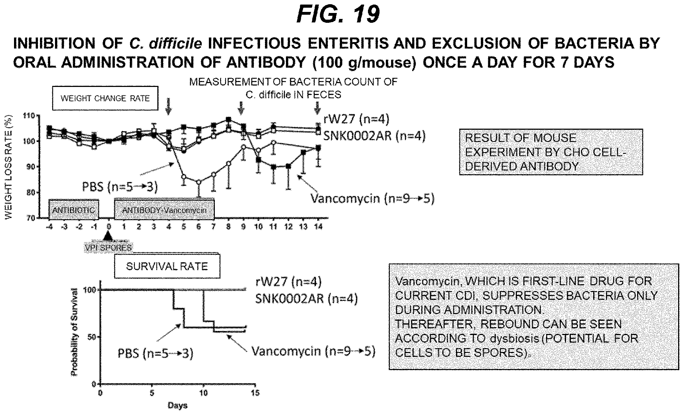

In Example 3, the effect of the antibody derived from the hybridoma on the C. difficile infected mouse was examined, and the same effect confirmation experiment on the C. difficile infected mouse was also performed for the recombinant IgA antibody. As recombinant IgA antibodies, antibody RS_H000_L001 (shown as rW 27 in Fig. 19) and antibody SNK0002AR were used. When recombinant IgA antibody, antibody RS_H000_L001 (rW 27), and SNK0002AR were administered to C. difficile infected mice, respectively, both antibodies significantly suppressed the weight loss of C. difficile infected mice (Fig. 19). Interestingly, mice to which Vancomycin was administered instead of the antibody showed no weight loss during the administration, but after the end of the administration, a significant weight loss was observed, and the survival rate also decreased (Fig. 19). As one of the causes of the decrease, it was considered that, due to the administration of Vancomycin in which gram-positive bacteria were sensitive, the number of gram-negative bacteria selectively increased in the intestinal tract to cause a state of dysbiosis, and C. difficile remaining after the discontinuation of the administration of Vancomycin was grown and showed the above disease state.

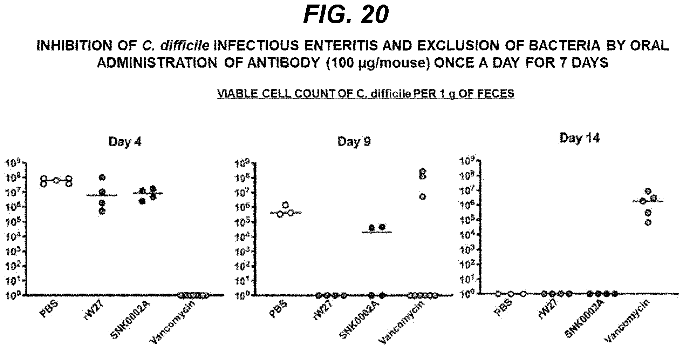

In order to confirm the growth state of C. difficile bacteria, a suspension diluent of feces at each time point after administration was seeded on a C. difficile selective medium, anaerobic culture was performed, and colonies were measured. As a result, as illustrated in Fig. 20, as expected, C. difficile increased on Day 14 after the discontinuation of administration in the mice to which Vancomycin was administered. On the other hand, in the mice to which each of the two types of IgA antibodies was administered, C. difficile was not detected in the feces on Day 14, and it was confirmed that C. difficile was successfully eliminated.

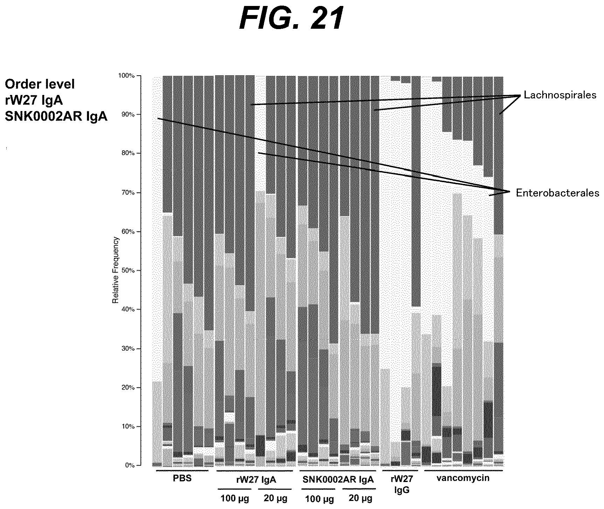

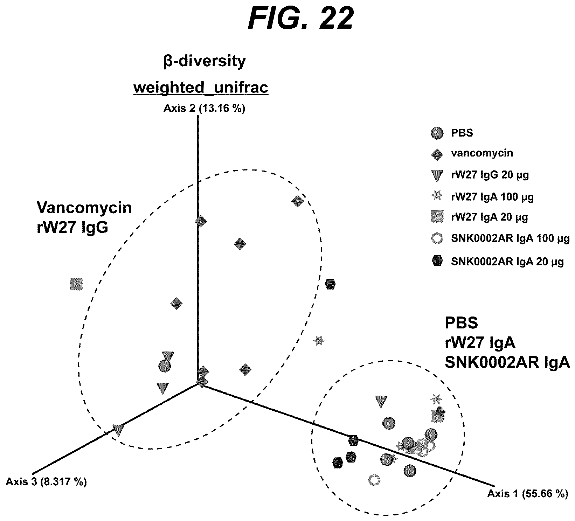

Next, whether dysbiosis actually occurred by administration of Vancomycin was examined by 16S rRNA analysis. Feces of the mice that died on the way were collected at the time of death. The surviving mice were analyzed by collecting feces at Day 14. In addition, an rW27 IgG antibody was also prepared and administered to mice to compare bacterial flora. The results thereof are illustrated in Figs. 21 and 22. Fig. 21 illustrates an analysis result of a relative abundance ratio of order levels. In the present analysis, data obtained as a result of administering the recombinant IgG antibody rW27 IgG antibody (recombinant IgG antibody linking sequences of variable regions of heavy chain H000 and light chain L001 to sequences of mouse IgG1 and mouse Igk (kappa)) as a control was also added to the analysis.

The largest change is that the rW27 IgG antibody and the Vancomycin administration group have considerably increased Enterobacterales. As described above, it is considered that gram-negative bacteria are increased by administration of Vancomycin. On the other hand, in the group in which no antibody was administered or each of the two types of recombinant IgA antibodies was administered, there was no large change in the bacterial flora, and the bacterial flora in which Lachnospirales, which is the original dominant bacterial species, was dominant was maintained. Fig. 22 illustrates the result of this change in β-diversity. It is found that the bacterial flora is clearly changed in the Vancomycin and rW27 IgG groups as compared with PBS, recombinant IgA antibody RS_H000_L001 (rW27), and SNK0002AR groups. Although it is obvious that Vancomycin, which is a first-line drug for human C. difficile enteritis, is effective in alleviating symptoms, it is considered that Vancomycin also causes dysbiosis in this way, and thus, enteritis is repeated. Combination therapy of IgA antibody and Vancomycin for C. difficile enteritis is expected to be radical therapy.

### Industrial Applicability

The present invention provides an antibody binding to C. difficile to inhibit a growth thereof, such that the antibody can be used in a treatment of a disease associated with C. difficile, and can also be used in a test to detect C. difficile in a biological sample and the like.

## Claims

1. A composition for inhibiting a growth of Clostridium difficile in a living body, the composition comprising a monoclonal antibody binding to a Clostridium difficile bacterial body or an antigen-binding fragment thereof.

2. The composition according to claim 1, wherein the composition is used for treating an inflammatory bowel disease.

3. The composition according to claim 1 or 2, wherein the monoclonal antibody binding to a Clostridium difficile bacterial body further binds to a Fusobacterium nucleatum bacterial body.

4. The composition according to any one of claims 1 to 3, wherein the monoclonal antibody binding to a Clostridium difficile bacterial body is:
a) an antibody comprising:
a heavy chain variable region comprising an amino acid sequence of a heavy chain CDR1, an amino acid sequence of a heavy chain CDR2, and an amino acid sequence of a heavy chain CDR3, the heavy chain variable region comprising an amino acid sequence set forth in SEQ ID NO: 7; and
a light chain variable region comprising an amino acid sequence of a light chain CDR1, an amino acid sequence of a light chain CDR2, and an amino acid sequence of a light chain CDR3, the light chain variable region comprising an amino acid sequence set forth in SEQ ID NO: 8,
b) an antibody comprising:
a heavy chain variable region comprising an amino acid sequence of a heavy chain CDR1, an amino acid sequence of a heavy chain CDR2, and an amino acid sequence of a heavy chain CDR3, the heavy chain variable region comprising an amino acid sequence set forth in SEQ ID NO: 17; and
a light chain variable region comprising an amino acid sequence of a light chain CDR1, an amino acid sequence of a light chain CDR2, and an amino acid sequence of a light chain CDR3, the light chain variable region comprising an amino acid sequence set forth in SEQ ID NO: 18,
c) an antibody comprising:
a heavy chain variable region comprising an amino acid sequence of a heavy chain CDR1, an amino acid sequence of a heavy chain CDR2, and an amino acid sequence of a heavy chain CDR3, the heavy chain variable region comprising an amino acid sequence set forth in SEQ ID NO: 27; and
a light chain variable region comprising an amino acid sequence of a light chain CDR1, an amino acid sequence of a light chain CDR2, and an amino acid sequence of a light chain CDR3, the light chain variable region comprising an amino acid sequence set forth in SEQ ID NO: 28,
d) an antibody comprising:
a heavy chain variable region comprising an amino acid sequence of a heavy chain CDR1, an amino acid sequence of a heavy chain CDR2, and an amino acid sequence of a heavy chain CDR3, the heavy chain variable region comprising an amino acid sequence set forth in SEQ ID NO: 37; and
a light chain variable region comprising an amino acid sequence of a light chain CDR1, an amino acid sequence of a light chain CDR2, and an amino acid sequence of a light chain CDR3, the light chain variable region comprising an amino acid sequence set forth in SEQ ID NO: 38, or
e) an antibody comprising at least one amino acid mutation in at least one region selected from heavy chains CDR1 to 3, light chains CDR1 to 3, and a light chain FR1, and
binding to the amino acid sequence RQEEHIELIAS in E. coli SHMT protein and the amino acid sequence VLDMMKLEKPE in C. difficile iPGM protein,
as a reference antibody comprising a heavy chain variable region comprising an amino acid sequence set forth in SEQ ID NO: 37, and
a light chain variable region comprising an amino acid sequence set forth in SEQ ID NO: 38.

5. The composition according to any one of claims 1 to 3, wherein the monoclonal antibody binding to a Clostridium difficile bacterial body is an antibody comprising a heavy chain variable region comprising:
a heavy chain CDR1 comprising an amino acid sequence set forth in SEQ ID NO: 1;
a heavy chain CDR2 comprising an amino acid sequence set forth in SEQ ID NO: 2; and
a heavy chain CDR3 comprising an amino acid sequence set forth in SEQ ID NO: 3, and
a light chain variable region comprising:
a light chain CDR1 comprising an amino acid sequence set forth in SEQ ID NO: 4;
a light chain CDR2 comprising an amino acid sequence set forth in SEQ ID NO: 5; and
a light chain CDR3 comprising an amino acid sequence set forth in SEQ ID NO: 6.

6. The composition according to any one of claims 1 to 3, wherein the monoclonal antibody binding to a Clostridium difficile bacterial body is an antibody comprising a heavy chain variable region comprising:
a heavy chain CDR1 comprising an amino acid sequence set forth in SEQ ID NO: 11;
a heavy chain CDR2 comprising an amino acid sequence set forth in SEQ ID NO: 12; and
a heavy chain CDR3 comprising an amino acid sequence set forth in SEQ ID NO: 13, and
a light chain variable region comprising:
a light chain CDR1 comprising an amino acid sequence set forth in SEQ ID NO: 14;
a light chain CDR2 comprising an amino acid sequence set forth in SEQ ID NO: 15; and
a light chain CDR3 comprising an amino acid sequence set forth in SEQ ID NO: 16.

7. The composition according to any one of claims 1 to 3, wherein the monoclonal antibody binding to a Clostridium difficile bacterial body is an antibody comprising a heavy chain variable region comprising:
a heavy chain CDR1 comprising an amino acid sequence set forth in SEQ ID NO: 21;
a heavy chain CDR2 comprising an amino acid sequence set forth in SEQ ID NO: 22; and
a heavy chain CDR3 comprising an amino acid sequence set forth in SEQ ID NO: 23, and
a light chain variable region comprising:
a light chain CDR1 comprising an amino acid sequence set forth in SEQ ID NO: 24;
a light chain CDR2 comprising an amino acid sequence set forth in SEQ ID NO: 25; and
a light chain CDR3 comprising an amino acid sequence set forth in SEQ ID NO: 26.

8. The composition according to any one of claims 1 to 3, wherein the monoclonal antibody binding to a Clostridium difficile bacterial body is an antibody comprising a heavy chain variable region comprising:
a heavy chain CDR1 comprising an amino acid sequence set forth in SEQ ID NO: 31;
a heavy chain CDR2 comprising an amino acid sequence set forth in SEQ ID NO: 32; and
a heavy chain CDR3 comprising an amino acid sequence set forth in SEQ ID NO: 33, and
a light chain variable region comprising:
a light chain CDR1 comprising an amino acid sequence set forth in SEQ ID NO: 34;
a light chain CDR2 comprising an amino acid sequence set forth in SEQ ID NO: 35; and
a light chain CDR3 comprising an amino acid sequence set forth in SEQ ID NO: 36.

9. The composition according to any one of claims 1 to 3, wherein the monoclonal antibody binding to a Clostridium difficile bacterial body is, as a reference antibody comprising a heavy chain variable region comprising an amino acid sequence set forth in SEQ ID NO: 37, and
a light chain variable region comprising an amino acid sequence set forth in SEQ ID NO: 38,
an antibody comprising at least one amino acid mutation in at least one region selected from heavy chains CDR1 to 3, light chains CDR1 to 3, and a light chain FR1, and
binding to the amino acid sequence RQEEHIELIAS in E. coli SHMT protein and the amino acid sequence VLDMMKLEKPE in C. difficile iPGM protein.

10. The composition according to any one of claims 1 to 3, wherein the monoclonal antibody binding to a Clostridium difficile bacterial body is an antibody comprising:
a heavy chain variable region comprising:
a heavy chain CDR1 comprising an amino acid sequence of X₁YYIH;
a heavy chain CDR2 comprising an amino acid sequence of RIDPENX₂X₃TTYAPKFQ; and
a heavy chain CDR3 comprising an amino acid sequence of YCARSTVL; and
a light chain variable region comprising:
a light chain CDR1 comprising an amino acid sequence of RX₄SQSIVHTNG;
a light chain CDR2 comprising an amino acid sequence of KLLIYKV; and
a light chain CDR3 comprising an amino acid sequence of GVYYFQGS,
in which a light chain FR1 contains an amino acid sequence of TPLSLPVSLGDQA or an amino acid sequence of SPASX₅SVSLGDRX₆,
X₁, X₂, and X₃ are each independently a neutral polar amino acid or an acidic polar amino acid, X₄ is a non-polar amino acid or a neutral polar amino acid, and X₅ and X₆ are each independently a non-polar amino acid.

11. The composition according to any one of claims 1 to 10, wherein the composition is provided as a lyophilizing agent.
